# EUROPEAN PATENT SPECIFICATION

(11) **EP 4 441 056 B1**
(45) Date of publication and mention of the grant of the patent: **11.11.2026**
(21) Application number: 22826084.0
(22) Date of filing: 30.11.2022
(51) Int. Cl.: C07D 498/10, A61P 35/00, A61K 31/423

(54) **ANNULATED 2-AMINO-3-CYANO THIOPHENES AND DERIVATIVES FOR THE TREATMENT OF CANCER**
ANELLIERTE 2-AMINO-3-CYANOTHIOPHENE UND DERIVATE ZUR BEHANDLUNG VON KREBS
2-AMINO-3-CYANO THIOPHÈNES ANNELÉS ET LEURS DÉRIVÉS POUR LE TRAITEMENT DU CANCER

(30) Priority: 01.12.2021 US 202163284757 P
(43) Date of publication of application: 09.10.2024
(73) Proprietor: Boehringer Ingelheim International GmbH, 55216 Ingelheim am Rhein (DE); Vanderbilt University, Nashville, TN 37240 (US)
(72) Inventor: BROEKER, Joachim, 55216 Ingelheim am Rhein (DE); ABBOTT, Jason, Nashville, Tennessee 37240 (US); CUI, Jianwen, Nashville, Tennessee 37240 (US); FESIK, Stephen W., Nashville, Tennessee 37240 (US); FUCHS, Julian, 55216 Ingelheim am Rhein (DE); HERDEIS, Lorenz, 55216 Ingelheim am Rhein (DE); HODGES, Tim, Nashville, Tennessee 37240 (US); LITTLE, Andrew, Nashville, Tennessee 37240 (US); MANTOULIDIS, Andreas, 55216 Ingelheim am Rhein (DE); PHAN, Jason, Nashville, Tennessee 37240 (US); RAMHARTER, Juergen, 55216 Ingelheim am Rhein (DE); SARKAR, Dhruba, Nashville, Tennessee 37240 (US); SMETHURST, Christian Alan Paul, (Deceased) (DE); SUN, Qi, Nashville, Tennessee 37240 (US); TREU, Matthias, 55216 Ingelheim am Rhein (DE); WATERSON, Alex, Nashville, Tennessee 37240 (US); WILDING, Birgit, 55216 Ingelheim am Rhein (DE); WUNBERG, Tobias, 55216 Ingelheim am Rhein (DE)
(74) Representative: Boehringer Global Patents
(86) International application number: PCT/EP2022/083930
(87) International publication number: WO 2023/099608

(56) References cited:
- WO-A1-2015/054572
- WO-A1-2020/028706
- WO-A1-2020/177629
- WO-A1-2020/236940
- WO-A1-2021/139748
- WO-A1-2021/150613
- WO-A1-2021/245051
- WO-A1-2021/245055

## Description

### Field of the invention

The present disclosure relates to annulated 2-amino-3-cyano thiophenes and derivatives of formula (I) 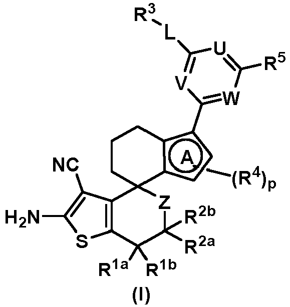 wherein **R^{1a}**, **R^{1b}**, **R^{2a}**, **R^{2b}**, Z, **R³** to **R⁵**, A, p, L, U, V and W have the meanings given in the claims and specification, their use as inhibitors of KRAS, pharmaceutical compositions and preparations containing such compounds and their use as medicaments/medical uses, especially as agents for treatment and/or prevention of oncological diseases, e.g. cancer.

### Background of the invention

V-Ki-ras2 Kirsten rat sarcoma viral oncogene homolog (KRAS) is a small GTPase of the Ras family of proteins that exists in cells in either GTP-bound or GDP-bound states (McCormick et al., J. Mol. Med. (Berl)., 2016, 94(3):253-8; Nimnual et al., Sci. STKE., 2002, 2002(145):pe36). Binding of GTPase activating proteins (GAPs) such as NF1 increases the GTPase activity of Ras family proteins. The binding of guanine nucleotide exchange factors (GEFs) such as SOS1 (Son of Sevenless 1) promotes release of GDP from Ras family proteins, enabling GTP binding (Chardin et al., Science, 1993, 260(5112):1338-43). When in the GTP-bound state, Ras family proteins are active and engage effector proteins including C-RAF and phosphoinositide 3-kinase (Pl3K) to promote the RAF/mitogen or extracellular signal-regulated kinases (MEK/ERK) pathway, Pl3K/AKT/mammalian target of rapamycin (mTOR) pathway and RalGDS (Ral guanine nucleotide dissociation stimulator) pathway (McCormick et al., J. Mol. Med. (Berl)., 2016, 94(3):253-8; Rodriguez-Viciana et al., Cancer Cell. 2005, 7(3):205-6). These pathways affect diverse cellular processes such as proliferation, survival, metabolism, motility, angiogenesis, immunity and growth (Young et al., Adv. Cancer Res., 2009, 102:1-17; Rodriguez-Viciana et al., Cancer Cell. 2005, 7(3):205-6).

Cancer-associated mutations in Ras family proteins suppress their intrinsic and GAP-induced GTPase activity leading to an increased population of GTP-bound/active mutant Ras family proteins (McCormick et al., Expert Opin. Ther. Targets., 2015, 19(4):451-4; Hunter et al., Mol. Cancer Res., 2015, 13(9):1325-35). This in turn leads to persistent activation of effector pathways (e.g. RAF/MEK/ERK, PI3K/AKT/mTOR, RalGDS pathways) downstream of mutant Ras family proteins. KRAS mutations (e.g. amino acids G12, G13, Q61, A146) are found in a variety of human cancers including lung cancer, colorectal cancer and pancreatic cancer (Cox et al., Nat. Rev. Drug Discov., 2014, 13(11):828-51). Alterations *(e.g.* mutation, overexpression, gene amplification) in Ras family proteins/Ras genes have also been described as a resistance mechanism against cancer drugs such as the EGFR antibodies cetuximab *a*nd panitumumab (Leto et al., J. Mol. Med. (Berl). 2014 Jul;92(7):709-22) and the EGFR tyrosine kinase inhibitor osimertinib/AZD9291 (Ortiz-Cuaran et al., Clin. Cancer Res., 2016, 22(19):4837-47; Eberlein et al., Cancer Res., 2015, 7 5(12):2489-500).

In a subset of tumor indications such as gastric cancer, gastroesophageal junction cancer and esophageal cancer prominent amplification of the wildtype (WT) KRAS proto-oncogene acts as a driver alteration and renders tumor models bearing this genotype addicted to KRAS in vitro and in vivo (Wong et al. Nat Med., 2018, 24(7):968-977). In contrast, non-amplified KRAS WT cell lines are KRAS independent, unless they carry secondary alterations in genes indirectly causing activation of KRAS (Meyers et al., Nat Genet., 2017, 49:1779-1784). Based on these data, a therapeutic window is expected for a KRAS targeting agent with a KRAS WT targeting activity.

Genetic alterations affecting e.g. codon 12 of KRAS substitute the glycine residue naturally occurring at this position for different amino acids such as aspartic acid (the G12D mutation or KRAS G12D), cysteine (the G12C mutation or KRAS G12C), valine (the G12V mutation or KRAS G12V) among others. Similarly, mutations within codons 13, 61 and 146 of KRAS are commonly found in the KRAS gene. Altogether KRAS mutations are detectable in 35 % of lung, 45 % of colorectal-, and up to 90 % of pancreatic cancers (Herdeis et al., Curr Opin Struct Biol., 2021, 71:136-147).

WO 2021/139748, WO 2021/150613, WO 2020/236940, WO 2020/028706, and WO 2020/17762 disclose spiro-based KRAS inhibitors and their use in therapy, the difference essentially lies in the spiro core structure (different cyclic moieties involved).

In summary, binders/inhibitors of wildtype or mutated KRAS (e.g., G12D, G12V and G12C) are expected to deliver anti-cancer efficacy.

Thus, there is the need to develop new compounds efficacious in the treatment of cancers mediated by KRAS, especially KRAS mutated in position 12 or 13 and/or in wild-type amplified KRAS mediated cancer, which also possess desirable pharmacological properties, including but not limited to: metabolic stability, plasma protein binding, solubility and permeability.

### Summary of the invention

The invention relates to a compound of the formula (**Ia**) or a salt thereof 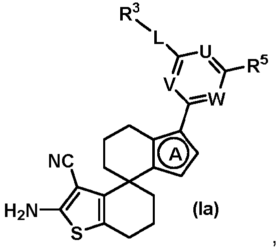 wherein
L is selected from -O-, -S- and -N(R⁷)-,
**R⁷** is hydrogen or C₁₋₆alkyl;
**R³** is selected from the group consisting of C₁₋₆alkyl, C₁₋₆alkoxy, 5-10 membered heteroaryl and 3-11 membered heterocyclyl, wherein the C₁₋₆alkyl, 5-10 membered heteroaryl, C₁₋₆alkoxy and 3-11 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with -N(C₁₋₄alkyl)₂;
**W** is nitrogen (-N=);
**V** is nitrogen (-N=);
**U** is -C(**R¹¹**)=;
   **R¹¹** is selected from hydrogen, halogen and C₁₋₄alkoxy;
ring A is a ring selected from the group consisting 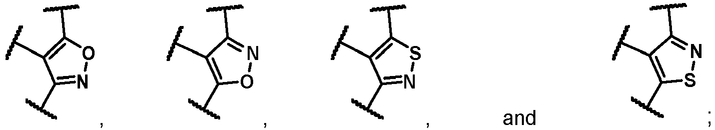
**R⁵** is selected from the group consisting of

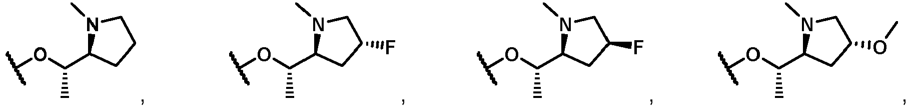

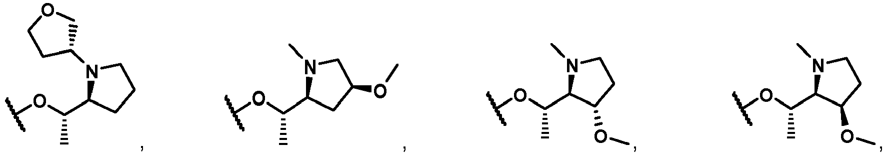

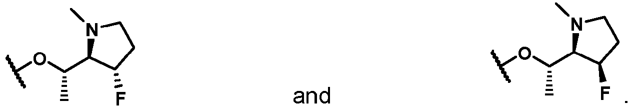

Another aspect of the invention relates to a compound of the formula (Ib) or a salt thereof 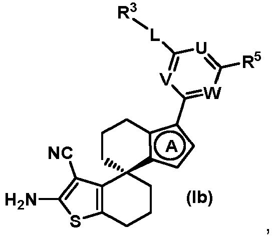 wherein
**A, V, U, W, L, R³** and **R⁵** are defined as in claim 1.

Another aspect of the invention relates to compound of formular (**Ia**) and (**Ib**) or salt, wherein **R³** is selected from the group consisting of C₁₋₆alkyl, C₁₋₆alkoxy, 5-6 membered heteroaryl and 4-5 membered heterocyclyl, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

Another aspect of the invention relates to compound of formular (**Ia**) and (**Ib**) or salt, wherein **R³** is selected from the group consisting of C₁₋₆alkyl, C₁₋₆alkoxy, 5-6 membered heteroaryl and 4-5 membered heterocyclyl, each of which independently contains one or two nitrogen or one oxygen heteroatom, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

Another aspect of the invention relates to compound of formular (**Ia**) and (Ib) or salt, wherein
**R³** is -C₁₋₄alkyl substituted with a 4-7 membered heterocyclyl or a C₃₋₅cycloalkyl, wherein the 4-7 membered heterocyclyl is optionally further substituted with -N(C₁₋₄alkyl)₂.

Another aspect of the invention relates to compound of formular (**Ia**) and (**Ib**) or salt, wherein
**R³** is selected from the group consisting of C₁₋₆alkyl, -CH(CH₃)CH₂-O-CH₃, -(CH₂)₂-O-CH₃, -(CH₂)₂-OH and -(CH₂)₂-N-(CH₃)₂,
   or
**R³** is a ring selected from the group consisting of 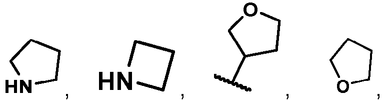 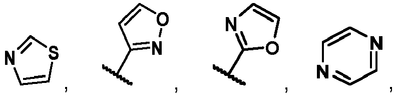 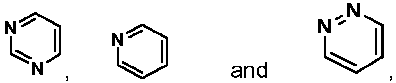 wherein each of these rings is optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.

Another aspect of the invention relates to compound of formular (**Ia**) and (**Ib**) or salt, wherein
**R³** is selected from the group consisting of C₁₋₆alkyl, -CH(CH₃)CH₂-O-CH₃, -(CH₂)₂-O-CH₃, -(CH₂)₂-OH, -(CH₂)₂-N-(CH₃)₂,

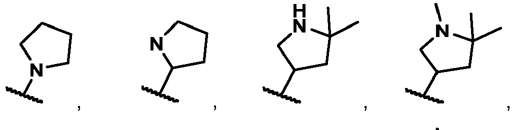

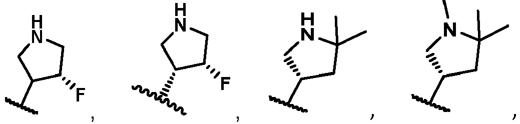

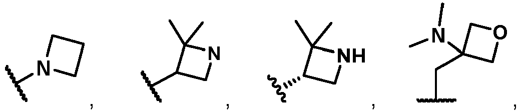

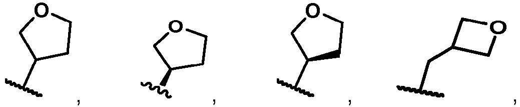

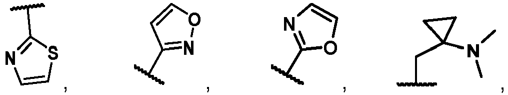

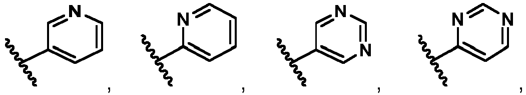

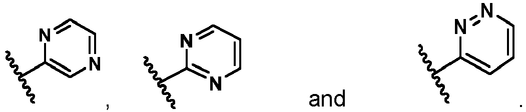

Another aspect of the invention relates to a compound or its pharmaceutically acceptable salt selected from the group consisting of: 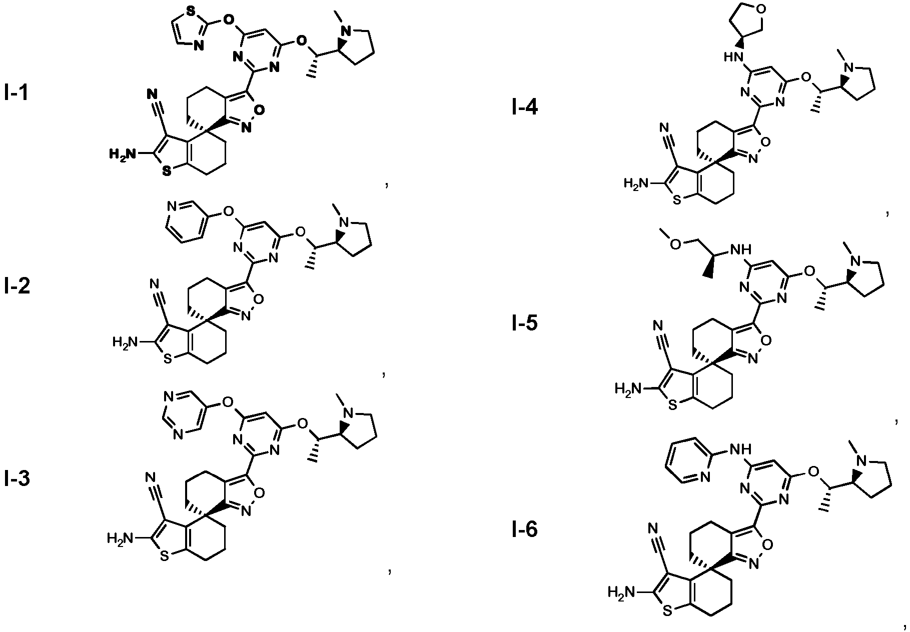 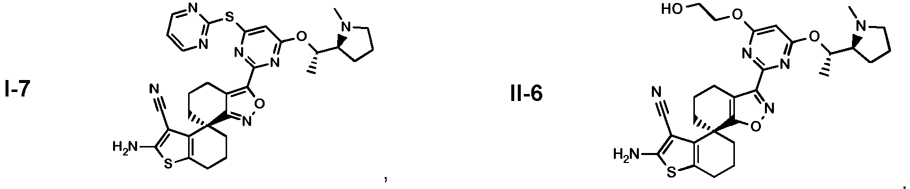 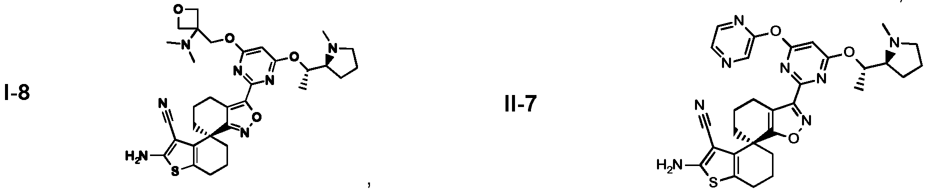 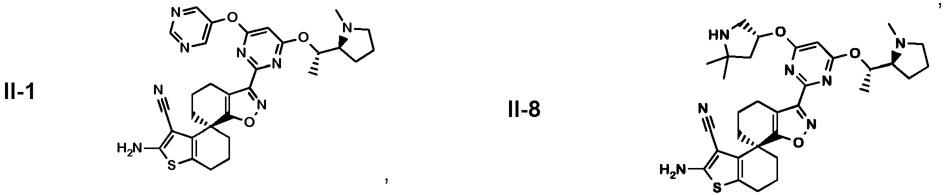 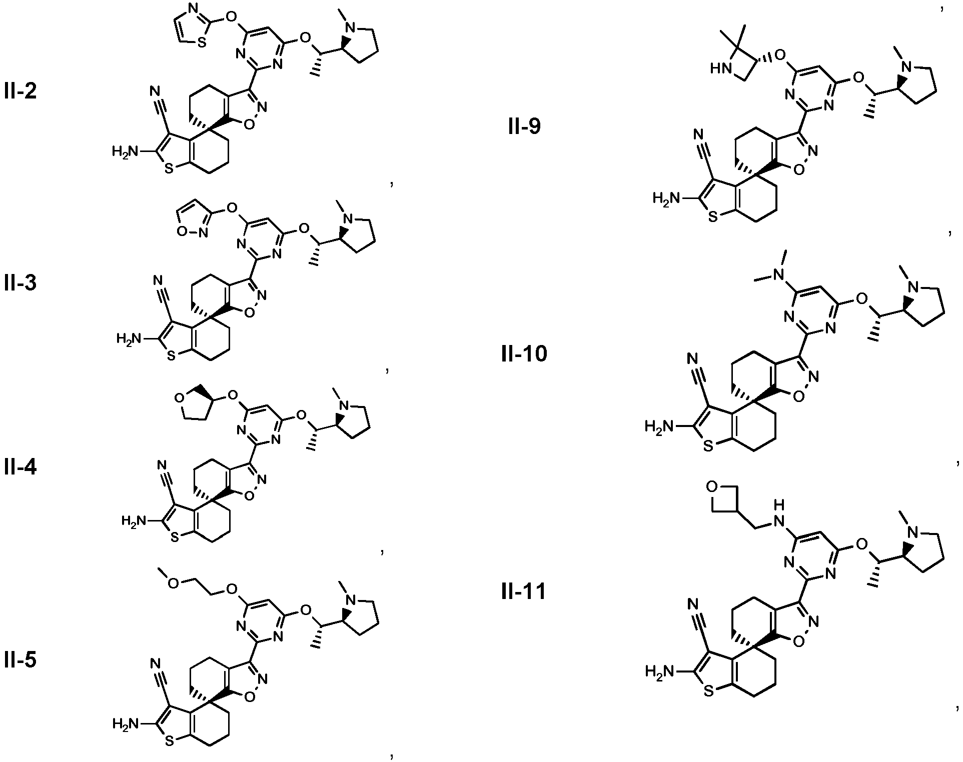 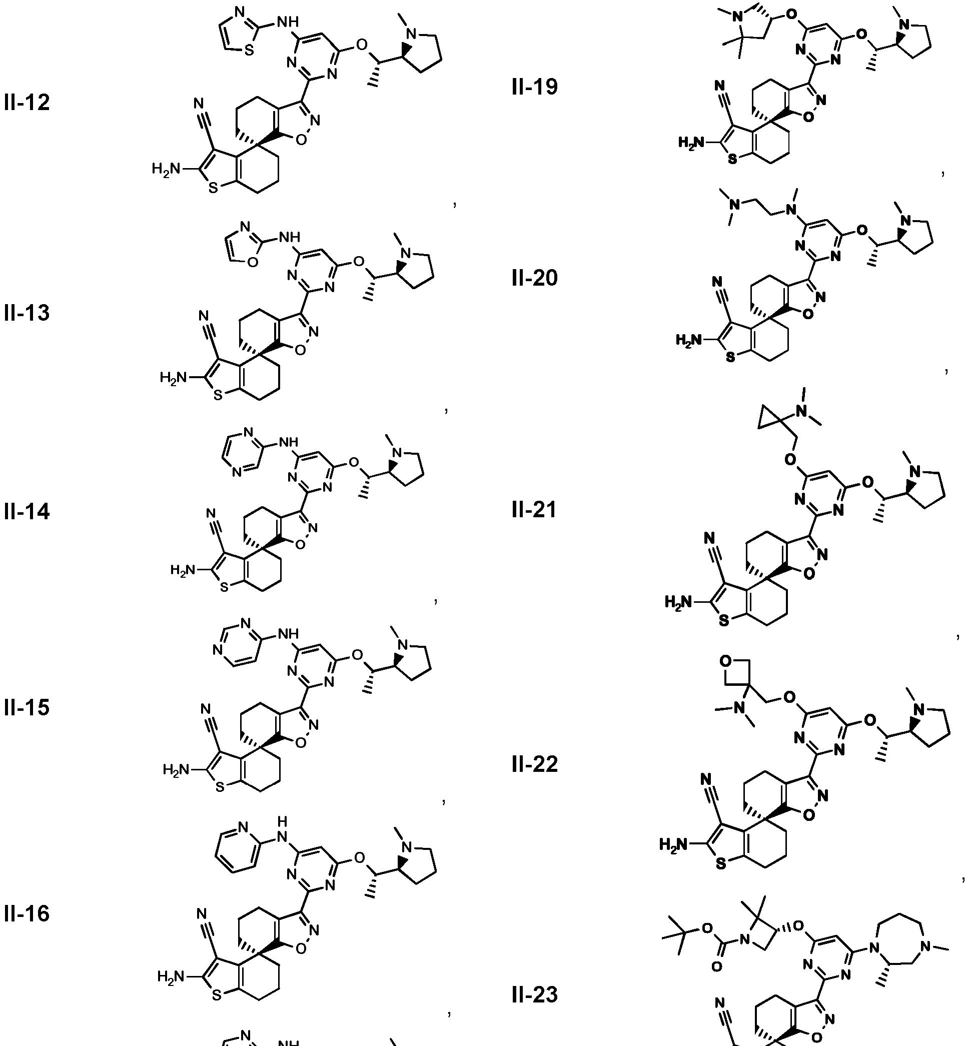 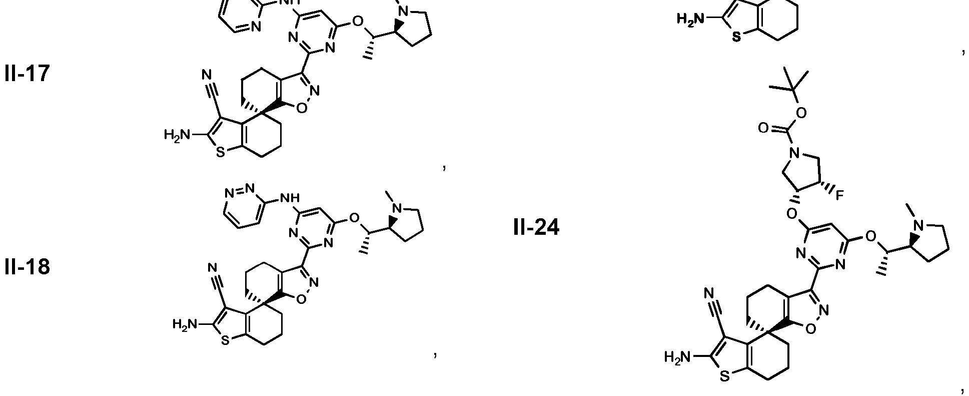 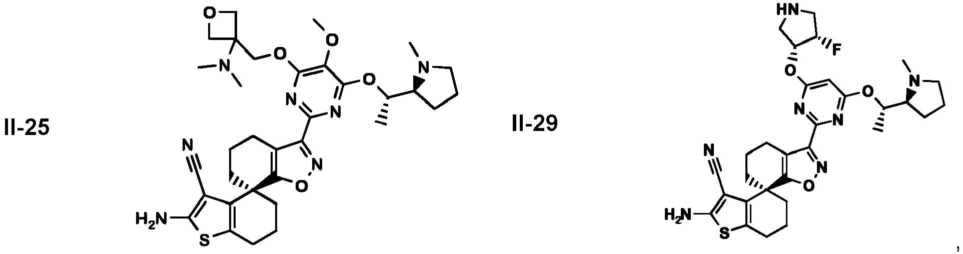 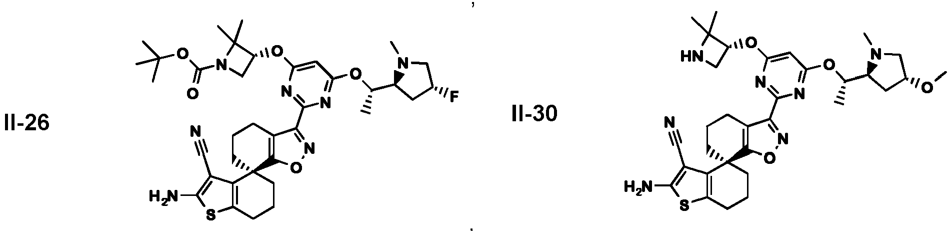 and

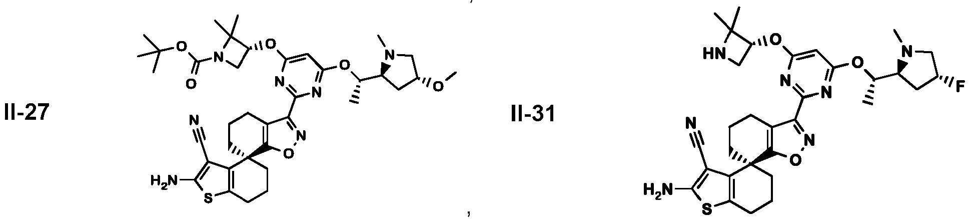

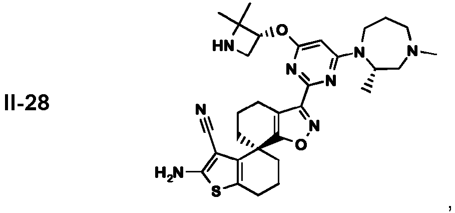

Another aspect of the invention relates to compound of formular (**Ia**) and (**Ib**) or a pharmaceutically acceptable salt thereof - for use in the treatment and/or prevention of cancer.

Another aspect of the invention relates to compound of formular (**Ia**) and (**Ib**) or a pharmaceutically acceptable salt thereof - for use in combination with one or more other pharmacologically active substance(s).

Another aspect of the invention relates to a composition comprising a compound of formular (**Ia**) and (**Ib**) or a pharmaceutically acceptable salt thereof - and one or more pharmaceutically acceptable excipient(s).

### Detailed description of the invention

Some aspects of the following disclosure go beyond the subject-matter of the claimed invention, which is defined in the appended claims. These aspects are given for illustration purposes.

It has now been found that, surprisingly, compounds of formula **(I)** 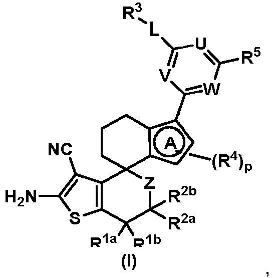 wherein
**R^{1a}**, **R^{1b}**, **R^{2a}**, **R^{2b}**, **Z, R³** to **R⁵**, **A, p, L, U, V** and **W** have the meanings given hereinafter act as inhibitors of KRAS and are involved in controlling cell proliferation. Thus, the compounds according to the invention may be used for example for the treatment of diseases characterized by excessive or abnormal cell proliferation.

Surprisingly, the compounds described herein have been found to possess anti-tumour activity, being useful in inhibiting the uncontrolled cellular proliferation which arises from malignant diseases. It is believed that this anti-tumor activity is, inter alia, derived from inhibition of KRAS mutated in position 12 or 13, preferably G12D, G12V or G12S mutant KRAS, or inhibition of WT KRAS, especially KRAS WT amplified. Advantageously, the compounds can be selective for certain KRAS mutants, preferably KRAS G12D, or can be effective against a panel of KRAS mutants including KRAS wildtype amplified.

In addition, the compounds of the invention advantageously possess desirable pharmacological properties, including but not limited to: metabolic stability, plasma protein binding, solubility and permeability.

Thus, in a first aspect, the disclosure provides a compound of formula **(I)** 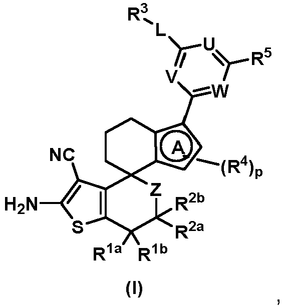 wherein
**R^{1a}** and **R^{1b}** are both independently selected from the group consisting of hydrogen, C₁₋₄alkyl, C₁₋₄haloalkyl, C₁₋₄alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocyclyl;
**R^{2a}** and **R^{2b}** are both independently selected from the group consisting of hydrogen, C₁₋₄alkyl, C₁₋₄haloalkyl, C₁₋₄alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocyclyl;
and/or, optionally, one of **R^{1a}** or **R^{1b}** and one of **R^{2a}** or **R^{2b}** together with the carbon atoms they are attached form a cyclopropane ring;
**Z** is -(CR^{6a}R^{6b})ₙ-;
each **R^{6a}** and **R^{6b}** is independently selected from the group consisting of hydrogen, C₁₋₄alkyl, C₁₋₄haloalkyl, C₁₋₄alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocyclyl;
or **R^{6a}** and **R^{6b}** together with the carbon atom they are attached form a cyclopropane ring;
n is selected from the group consisting of 0, 1 and 2;
L is selected from -O-, -S- and -N(**R⁷**)-, wherein **R⁷** is hydrogen or C₁₋₆alkyl;
**R³** is selected from the group consisting of C₁₋₆alkyl, C₁₋₆alkoxy, 5-10 membered heteroaryl and 3-11 membered heterocyclyl, wherein the C₁₋₆alkyl, 5-10 membered heteroaryl, C₁₋₆alkoxy and 3-11 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂;
**W** is nitrogen (-N=) or -CH=;
**V** is nitrogen (-N=) or -CH=;
**U** is nitrogen (-N=) or -C(**R¹¹**)=;
   **R¹¹** is selected from hydrogen, halogen and C₁₋₄alkoxy;
ring A is a ring selected from the group consisting of pyrrole, furan, thiophene, imidazole, pyrazole, oxazole, isoxazole, thiazole, isothiazole and triazole;
each **R⁴**, if present, is independently selected from the group consisting of C₁₋₆alkyl, C₁₋₆haloalkyl, C₁₋₆alkoxy, C₁₋₆haloalkoxy, cyano-C₁₋₆alkyl, halogen, -OH, -NH₂, -NH(C₁₋₄alkyl), -N(C₁₋₄alkyl)₂, -CN, C₃₋₅cycloalkyl and 3-5 membered heterocyclyl;
**p** is selected from the group consisting of 0, 1, 2 and 3;
**R⁵** is a 3-11 membered heterocyclyl optionally substituted with one or more identical or different C₁₋₆alkyl, C₁₋₆alkoxy or a 5-6 membered heterocyclyl, wherein the C₁₋₆alkyl is optionally substituted with cyclopropyl;
or **R⁵** is -O-C₁₋₆alkyl substituted with a 3-11 membered heterocyclyl, wherein the 3-11 membered heterocyclyl is optionally substituted with one or more, identical or different **R¹²**,
   each **R¹²** is selected from the group consisting of C₁₋₆alkyl, C₁₋₆alkoxy, halogen and 3-11 membered heterocyclyl;
or a salt thereof.

In another aspect, the disclosure provides the compound of the formula (I), or a salt thereof, wherein **R^{1a}** and **R^{1b}** are both independently selected from the group consisting of hydrogen and C₁₋₄alkyl.

In another aspect, the disclosure provides the compound of the formula (I), or a salt thereof, wherein **R^{2a}** and **R^{2b}** are both independently selected from the group consisting of hydrogen and halogen.

In another aspect, the disclosure provides the compound of the formula (I), or a salt thereof, wherein **R^{1a}** and **R^{1b}** are both independently selected from the group consisting of hydrogen and methyl.

In another aspect, the disclosure provides the compound of the formula (I), or a salt thereof, wherein **R^{2a}** and **R^{2b}** are both independently selected from the group consisting of hydrogen and fluorine.

In another aspect, the disclosure provides the compound of the formula (I), or a salt thereof, wherein **R^{1a}**, **R^{1b}**, **R^{2a}** and **R^{2b}** are hydrogen.

In another aspect, the disclosure provides the compound of the formula (I), or a salt thereof, wherein n is 0.

In another aspect, the disclosure provides the compound of the formula **(I),** or a salt thereof, wherein n is 1;
each **R^{6a}** and **R^{6b}** is independently selected from the group consisting of hydrogen, C₁₋₄alkyl, C₁₋₄haloalkyl, C₁₋₄alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocyclyl.

In another aspect, the disclosure provides the compound of the formula **(I),** or a salt thereof, wherein **Z** is -CH₂-.

In another aspect, the disclosure provides the compound of the formula **(I),** or a salt thereof, wherein **n** is 2;
each **R^{6a}** and **R^{6b}** is independently selected from the group consisting of hydrogen, C₁₋₄alkyl, C₁₋₄haloalkyl, C₁₋₄alkoxy, C₁₋₄haloalkoxy, halogen, -NH₂, -NH(C₁₋₄alkyl), -N(C₁₋₄alkyl)₂, C₃₋₅cycloalkyl and 3-5 membered heterocyclyl.

In another aspect, the disclosure provides the compound of the formula (I), or a salt thereof, wherein p is 0.

In another aspect, the disclosure provides a compound of the formula (I*) or a salt thereof 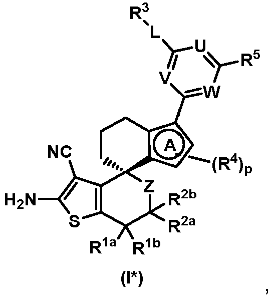 wherein
**R^{1a}**, **R^{1b}**, **R^{2a}**, **R^{2b}**, **R³**, **R⁴**, **R⁵**, **Z, L, U, V, W,** ring **A** and p are as defined herein above or below.

In another aspect the present invention relates to a compound of the formula **(Ia)** or a salt thereof 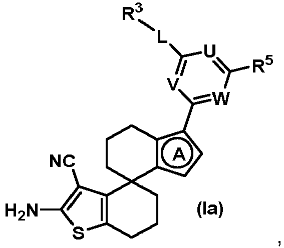 wherein
**A, V, U, W, L, R³** and R**⁵** are as defined herein.

In another aspect, the invention relates to a compound of formula (Ib) or a salt thereof 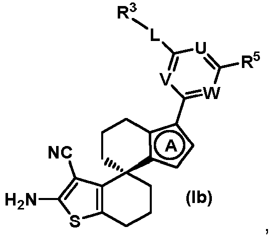 wherein
**A, V, U, W, L, R³** and **R⁵** are as defined herein.

In another aspect, the disclosure provides the compound of the invention, or a salt thereof, wherein ring **A** is a ring selected from the group consisting of pyrrole, furan, thiophene, imidazole, pyrazole, isoxazole, isothiazole and triazole.

In another aspect, the disclosure provides the compound of the invention, or a salt thereof, wherein ring A is selected from the group consisting of 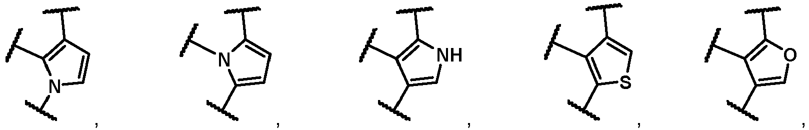 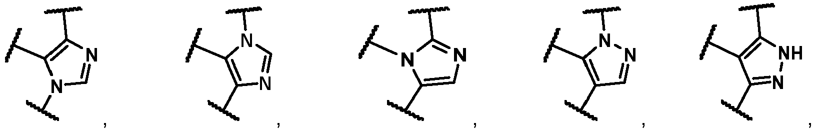 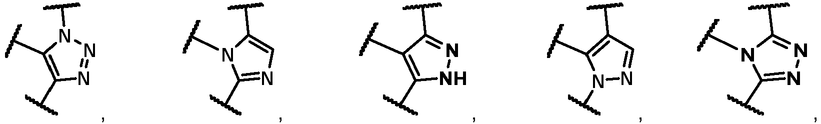 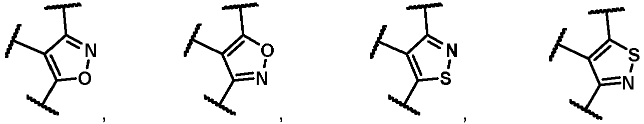 and

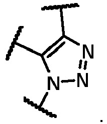

In another aspect, the invention relates to the compound of the invention, or a salt thereof, wherein ring A is isoxazole or isothiazole.

In another aspect, the invention relates to the compound of the invention, or a salt thereof, wherein ring A is selected from

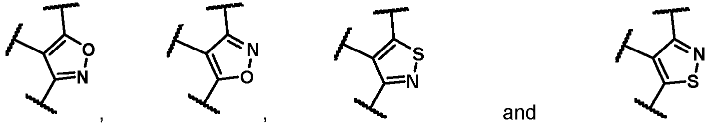

In another aspect the invention relates to a compound of formula (Ic), or a salt thereof 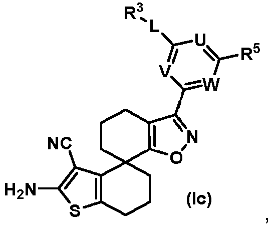 wherein
**V, U, W, L, R³** and R**⁵** are as defined herein.

In another aspect the invention relates to a compound of formula **(Id),** or a salt thereof, 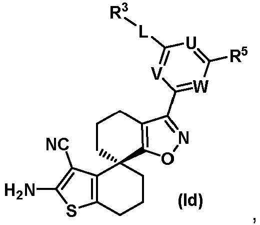 wherein
**V, U, W, L, R³** and R**⁵** are as defined herein.

In another aspect the invention relates to a compound of formula **(Ie)**, or a salt thereof 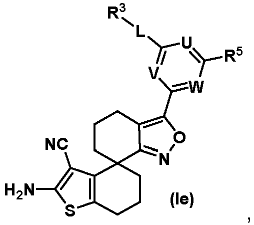 wherein
**V, U, W, L, R³** and R**⁵** are as defined herein.

In another aspect the invention relates to a compound of formula **(If),** or a salt thereof 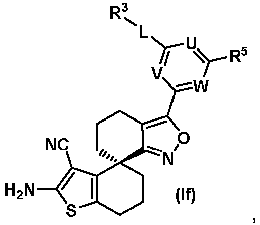 wherein
**V, U, W, L, R³** and **R⁵** are as defined herein.

In another aspect, the disclosure provides the compound of the formula **(I), (I*)**, **(Ia)**, **(Ib), (Ic), (Id), (Ie)** or **(If),** or a salt thereof, wherein at least one of **W, V** and **U** is nitrogen.

In another aspect, the invention relates to the compound of the formula **(I), (I*), (Ia), (Ib), (Ic), (Id), (Ie)** or **(If),** or a salt thereof, wherein
**W** is nitrogen (-N=);
**V** is nitrogen (-N=);
**U is** =C(**R¹¹**)-;
   **R¹¹** is selected from hydrogen, halogen and C₁₋₄alkoxy.

In another aspect, the disclosure provides the compound of the formula **(I), (I*)**, **(Ia)**, **(Ib), (Ic), (Id), (Ie)** or **(If),** or a salt thereof, wherein
**W** is -CH=;
**V** is nitrogen (-N=);
**U is** =C(**R¹¹**)-;
   **R¹¹** is selected from hydrogen, halogen and C₁₋₄alkoxy.

In another aspect, the disclosure provides the compound of the formula **(I), (I*)**, **(Ia)**, **(Ib), (Ic), (Id), (Ie)** or **(If),** or a salt thereof, wherein
**V** is -CH=;
**W** is nitrogen (-N=);
**U** is =C(**R¹¹**)-;
   **R¹¹** is selected from hydrogen, halogen and C₁₋₄alkoxy.

In another aspect, the disclosure relates to the compound of the formula **(I), (I*)**, **(Ia)**, **(Ib), (Ic), (Id), (Ie)** or **(If),** or a salt thereof, wherein
**R¹¹** is selected from hydrogen, fluorine, chlorine and -O-CH₃.

In another aspect, the disclosure provides the compound of the formula **(I), (I*)**, **(Ia)**, **(Ib), (Ic), (Id), (Ie)** or **(If),** or a salt thereof, wherein
**V** is nitrogen (-N=);
**W** is -CH=;
**U** is nitrogen (-N=).

In another aspect, the disclosure provides the compound of the formula **(I), (I*)**, **(Ia)**, **(Ib), (Ic), (Id), (Ie)** or **(If),** or a salt thereof, wherein
**W** is nitrogen (-N=);
**V** is -CH=;
**U** is nitrogen (-N=).

In another aspect, the disclosure provides the compound of the formula (I), **(I*)**, **(Ia)**, **(Ib), (Ic), (Id), (Ie)** or **(If),** or a salt thereof, wherein
**W** is -CH=;
**V** is -CH=;
**U** is nitrogen (-N=).

In another aspect, the disclosure provides the compound of the formula **(I), (I*)**, **(Ia)**, **(Ib), (Ic), (Id)**, **(Ie)** or **(If)** or a salt thereof, wherein
**W** is nitrogen (-N=);
**V** is nitrogen (-N=);
**U** is nitrogen (-N=).

In another aspect, the disclosure provides the compound of the formula **(I), (I*), (Ia), (Ib), (Ic), (Id), (Ie)** or **(If),** or a salt thereof, wherein
**R⁵** is a 6-11 membered heterocyclyl optionally substituted with one or more identical or different C₁₋₆alkyl, C₁₋₆alkoxy or a 5-6 membered heterocyclyl, wherein the C₁₋₆alkyl is optionally substituted with cyclopropyl.

In another aspect, the disclosure provides the compound of the formula **(I), (I*), (Ia), (Ib), (Ic), (Id), (Ie)** or **(If),** or a salt thereof, wherein
**R⁵** is a 7 membered heterocyclyl, optionally substituted with one or more identical or different C₁₋₄alkyl.

In another aspect, the disclosure provides the compound of the formula **(I), (I*), (Ia), (Ib), (Ic), (Id), (Ie)** or **(If),** or a salt thereof, wherein
**R⁵** is -O-C₁₋₆alkyl substituted with a 5-8 membered heterocyclyl, wherein the 5-8 membered heterocyclyl is optionally substituted with one or more, identical or different **R¹²**,
each **R¹²** is selected from the group consisting of C₁₋₆alkyl, C₁₋₆alkoxy, halogen and 5 membered heterocyclyl.

In another aspect, the disclosure provides the compound of the formula **(I), (I*)**, **(Ia)**, **(Ib), (Ic), (Id), (Ie)** or **(If),** or a salt thereof, wherein
**R⁵** is selected from the group consisting of 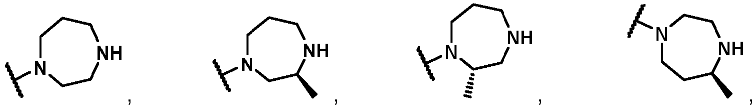 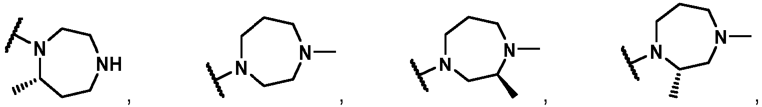 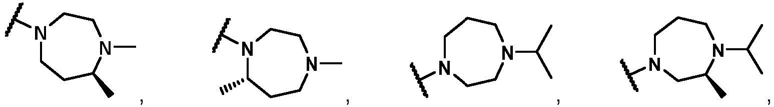 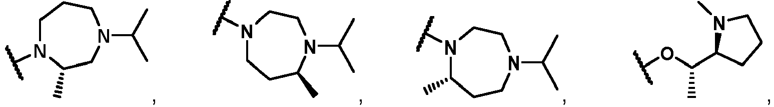 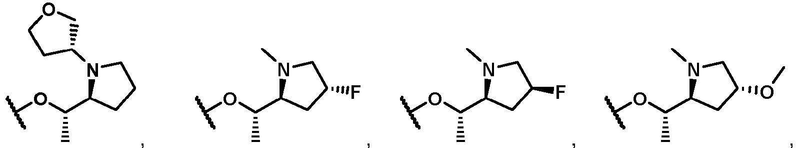 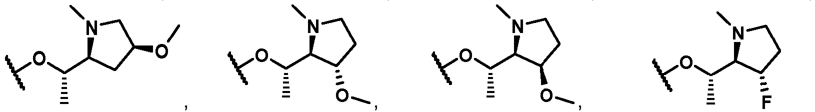 and

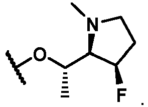

In another aspect, the disclosure provides the compound of the formula **(I), (I*)**, **(Ia)**, **(Ib), (Ic), (Id), (Ie)** or **(If),** or a salt thereof, wherein
**R⁵** is selected from the group consisting of 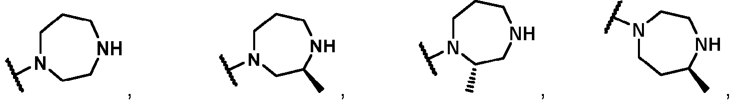 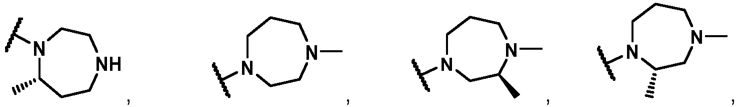 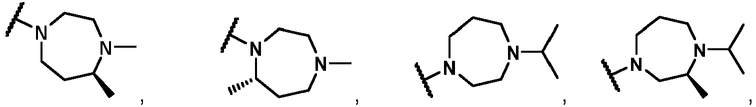 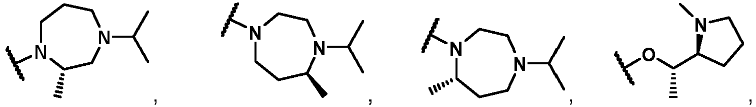 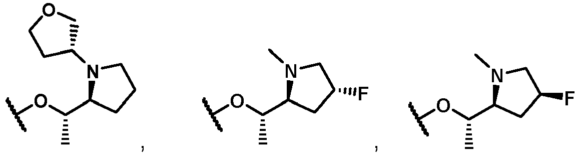 and

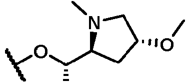

In another aspect, the disclosure provides the compound of the formula **(I), (I*)**, **(Ia)**, **(Ib), (Ic), (Id), (Ie)** or **(If),** or a salt thereof, wherein
**R⁵** is selected from the group consisting of

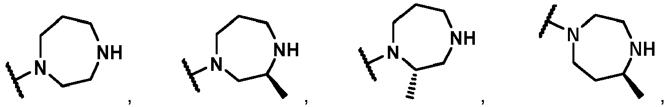

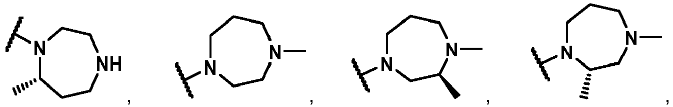

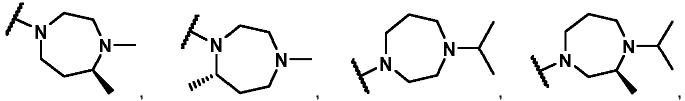

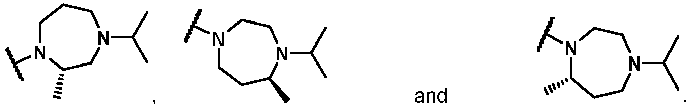

In another aspect, the disclosure relates to the compound of the formula **(I), (I*)**, **(Ia)**, **(Ib), (Ic), (Id), (Ie)** or **(If),** or a salt thereof, wherein
**R⁵** is selected from the group consisting of

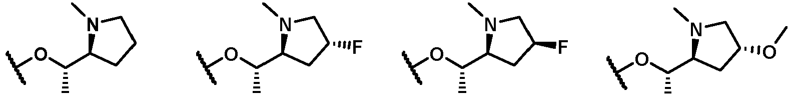

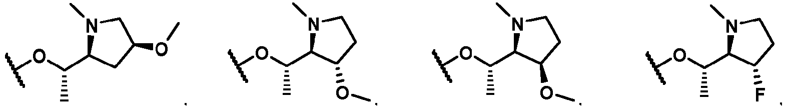

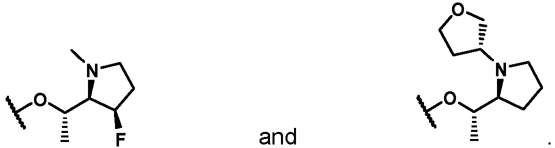

In another aspect, the disclosure relates to the compound of the formula **(I), (I*)**, **(Ia)**, **(Ib), (Ic), (Id), (Ie)** or **(If),** or a salt thereof, wherein
**R⁵** is selected from the group consisting of 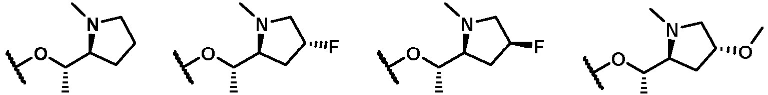 and

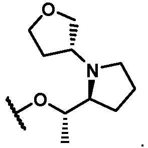

In another aspect, the disclosure relates to the compound of the formula **(I), (I*)**, **(Ia)**, **(Ib), (Ic), (Id)**, **(Ie)** or **(If),** or a salt thereof, wherein **R⁵** is selected from the group consisting of

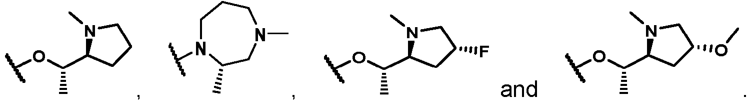

In another aspect, the disclosure relates to the compound of the formula **(I), (I*)**, **(Ia)**, **(Ib), (Ic), (Id)**, **(Ie)** or **(If)** or a salt thereof, wherein
**R³** is selected from the group consisting of C₁₋₆alkyl, C₁₋₆alkoxy, 5-10 membered heteroaryl and 3-11 membered heterocyclyl, wherein the C₁₋₆alkyl, 5-10 membered heteroaryl, C₁₋₆alkoxy and 3-11 membered heterocyclyl are all optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl;

In another aspect, the disclosure relates to the compound of the formula **(I), (I*), (Ia), (Ib), (Ic), (Id)**, **(Ie)** or **(If)** or a salt thereof, wherein
**R³** is selected from the group consisting of C₁₋₆alkyl, C₁₋₆alkoxy, 5-6 membered heteroaryl and 4-5 membered heterocyclyl wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

In another aspect, the disclosure relates to the compound of the formula **(I), (I*), (Ia), (Ib), (Ic), (Id)**, **(Ie)** or **(If)** or a salt thereof, wherein
**R³** is selected from the group consisting of C₁₋₆alkyl, C₁₋₆alkoxy, 5-6 membered heteroaryl and 4-5 membered heterocyclyl wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.

In another aspect, the disclosure relates to the compound of the formula **(I), (Ia)**, **(I*)**, **(Ib), (Ic), (Id)**, **(Ie)** or **(If)** or a salt thereof, wherein
**R³** is selected from the group consisting of C₁₋₆alkyl, C₁₋₆alkoxy, 5-6 membered heteroaryl and 4-5 membered heterocyclyl, each of which independently contains one or two nitrogen or one oxygen heteroatom, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

In another aspect, the disclosure relates to the compound of the formula **(I), (Ia)**, **(I*)**, **(Ib)**, **(Ic), (Id)**, **(Ie)** or **(If)** or a salt thereof, wherein
**R³** is selected from the group consisting of C₁₋₆alkyl, C₁₋₆alkoxy, 5-6 membered heteroaryl and 4-5 membered heterocyclyl, each of which independently contains one or two nitrogen or one oxygen heteroatom, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.

In another aspect, the disclosure relates to the compound of the formula **(I), (Ia)**, **(I*)**, **(Ib)**, **(Ic), (Id), (Ie)** or **(If),** or a salt thereof, wherein
**R³** is C₁₋₄alkyl substituted with a 4-7 membered heterocyclyl or a C₃₋₅cycloalkyl, wherein the 4-7 membered heterocyclyl is optionally further substituted with -N(C₁₋₄alkyl)₂.

In another aspect, the disclosure relates to the compound of the formula **(I), (I*)**, **(Ia)**, **(Ib), (Ic), (Id), (Ie)** or **(If),** or a salt thereof, wherein
**R³** is selected from the group consisting of C₁₋₆alkyl, -CH(CH₃)CH₂-O-CH₃, -(CH₂)₂-O-CH₃, -(CH₂)₂-OH and -(CH₂)₂-N-(CH₃)₂,
   or
**R³** is a ring selected from the group consisting of 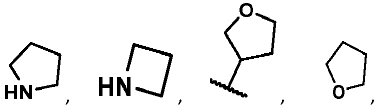 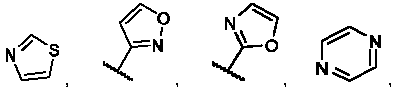 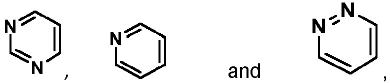 wherein each of these rings is optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.

In another aspect, the disclosure relates to the compound of the formula **(I), (I*), (Ia), (Ib), (Ic), (Id), (Ie)** or **(If),** or a salt thereof, wherein
**R³** is selected from the group consisting of C₁₋₆alkyl, -CH(CH₃)CH₂-O-CH₃, -(CH₂)₂-O-CH₃, -(CH₂)₂-OH, -(CH₂)₂-N-(CH₃)₂,

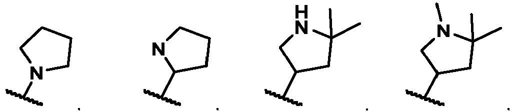

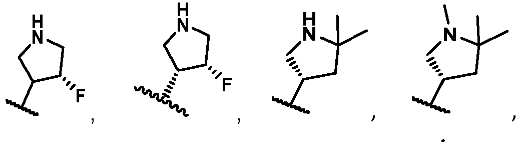

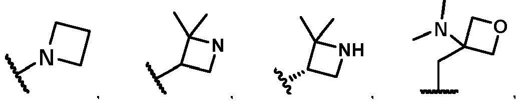

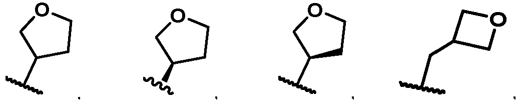

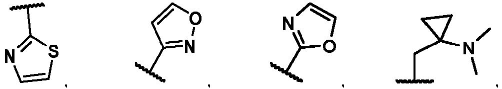

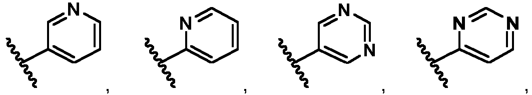

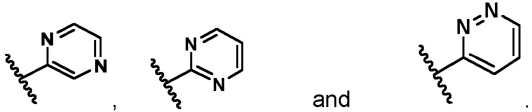

In another aspect, the invention relates to the compound of the formula **(Ic), (Id), (Ie)** or **(If),** or a salt thereof, wherein
-L- is -O-;
**R³** is a 4-5 membered heterocyclyl which contains one or two nitrogen heteroatom(s), wherein the 4-5 membered heterocyclyl is optionally substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.
**W** is nitrogen (-N=);
**V** is nitrogen (-N=);
**U** is -CH=;
**R⁵** is -O-C₁₋₆alkyl substituted with a 5-8 membered heterocyclyl, wherein the 5-8 membered heterocyclyl is optionally substituted with one or more, identical or different **R¹²,**
   each **R¹²** is selected from the group consisting of C₁₋₆alkyl, C₁₋₆alkoxy, halogen and 5 membered heterocyclyl.

Preferred embodiments of compounds of formula **(I)** according to the invention are example compounds **I-1** to **I-7** and **II-1** to **II-31** and any subset thereof.

It is to be understood that any two or more aspects and/or preferred embodiments of formula **(I)** - or subformulas thereof - may be combined in any way leading to a chemically stable structure to obtain further aspects and/or preferred embodiments of formula **(I)** - or subformulas thereof.

The present disclosure further relates to hydrates, solvates, polymorphs, metabolites, derivatives, stereoisomers and prodrugs of a compound of formula **(I), (I*), (Ia), (Ib), (Ic), (Id)**, **(Ie)** or **(If)** (including all embodiments thereof).

The present disclosure further relates to a hydrate of a compound of formula **(I), (I*), (Ia), (Ib), (Ic), (Id), (Ie)** or **(If)** (including all embodiments thereof).

The present disclosure further relates to a solvate of a compound of formula **(I), (I*), (Ia), (Ib), (Ic), (Id), (Ie)** or **(If)** (including all embodiments thereof).

Compounds of formula **(I), (I*), (Ia)**, **(Ib), (Ic), (Id), (Ie)** or **(If)** (including all embodiments thereof) which e.g. bear ester groups are potential prodrugs the ester being cleaved under physiological conditions and are also part of the disclosure.

The present disclosure further relates to a pharmaceutically acceptable salt of a compound of formula **(I), (I*), (Ia)**, **(Ib), (Ic), (Id)**, **(Ie)** or **(If)** (including all embodiments thereof).

The present disclosure further relates to a pharmaceutically acceptable salt of a compound of formula **(I), (I*), (Ia)**, **(Ib), (Ic), (Id), (Ie)** or **(If)** (including all embodiments thereof) with anorganic or organic acids or bases.

### Pharmaceutical compositions

A further object of the disclosure is a pharmaceutical composition comprising a compound of formula **(I), (I*), (Ia)**, **(Ib), (Ic), (Id)**, **(Ie)** or **(If)** - or a pharmaceutically acceptable salt thereof - and one or more pharmaceutically acceptable excipient(s).

In one aspect, said pharmaceutical composition optionally comprises one or more other pharmacologically active substance(s). Said one or more other pharmacologically active substance(s) may be the pharmacologically active substances or combination partners as herein defined.

Suitable pharmaceutical compositions for administering the compounds of formula **(I), (I*), (Ia)**, **(Ib), (Ic), (Id)**, **(Ie)** or **(If)** according to the invention will be apparent to those with ordinary skill in the art and include for example tablets, pills, capsules, suppositories, lozenges, troches, solutions, suspensions - particularly solutions, suspensions or other mixtures for injection (s.c., i.v., i.m.) and infusion (injectables) - elixirs, syrups, sachets, emulsions, inhalatives or dispersible powders. The content of the compounds **(I), (I*), (Ia)**, **(Ib), (Ic), (Id), (Ie)** or **(If)** should be in the range from 0.1 to 90 wt.-%, preferably 0.5 to 50 wt.-% of the composition as a whole, *i.e.* in amounts which are sufficient to achieve the dosage range specified below. The doses specified may, if necessary, be given several times a day.

Suitable tablets may be obtained, for example, by mixing the compounds of formula **(I), (I*), (Ia)**, **(Ib), (Ic), (Id)**, **(Ie)** or **(If)** with known pharmaceutically acceptable excipients, for example inert diluents, carriers, disintegrants, adjuvants, surfactants, binders and/or lubricants. The tablets may also comprise several layers.

Coated tablets may be prepared accordingly by coating cores produced analogously to the tablets with excipients normally used for tablet coatings, for example collidone or shellac, gum arabic, talc, titanium dioxide or sugar. To achieve delayed release or prevent incompatibilities the core may also consist of a number of layers. Similarly, the tablet coating may consist of a number of layers to achieve delayed release, possibly using the excipients mentioned above for the tablets.

Syrups or elixirs containing one or more **(I), (I*), (Ia)**, **(Ib), (Ic), (Id)**, **(Ie)** or **(If)** or combinations with one or more other pharmaceutically active substance(s) may additionally contain excipients like a sweetener such as saccharine, cyclamate, glycerol or sugar and a flavour enhancer, e.g. a flavouring such as vanillin or orange extract. They may also contain excipients like suspension adjuvants or thickeners such as sodium carboxymethyl cellulose, wetting agents such as, for example, condensation products of fatty alcohols with ethylene oxide, or preservatives such as p-hydroxybenzoates.

Solutions for injection and infusion are prepared in the usual way, *e.g.* with the addition of excipients like isotonic agents, preservatives such as p-hydroxybenzoates, or stabilisers such as alkali metal salts of ethylenediamine tetra acetic acid, optionally using emulsifiers and/or dispersants, whilst if water is used as the diluent, for example, organic solvents may optionally be used as solvating agents or dissolving aids, and transferred into injection vials or ampoules or infusion bottles.

Capsules containing one or more compounds of formula **(I), (I*), (Ia)**, **(Ib), (Ic), (Id), (Ie)** or **(If)** or combinations with one or more other pharmaceutically active substance(s) may for example be prepared by mixing the compounds/active substance(s) with inert excipients such as lactose or sorbitol and packing them into gelatine capsules.

Suitable suppositories may be made for example by mixing with excipients provided for this purpose such as neutral fats or polyethylene glycol or the derivatives thereof.

Excipients which may be used include, for example, water, pharmaceutically acceptable organic solvents such as paraffins (*e.g.* petroleum fractions), vegetable oils (*e.g.* groundnut or sesame oil), mono- or polyfunctional alcohols (*e.g.* ethanol or glycerol), carriers such as *e.g.* natural mineral powders (*e.g.* kaolins, clays, talc, chalk), synthetic mineral powders (*e.g.* highly dispersed silicic acid and silicates), sugars (*e.g.* cane sugar, lactose and glucose), emulsifiers (*e.g*. lignin, spent sulphite liquors, methylcellulose, starch and polyvinylpyrrolidone) and lubricants (*e.g*. magnesium stearate, talc, stearic acid and sodium lauryl sulfate).

The pharmaceutical compositions are administered by the usual methods, preferably by oral or transdermal route, most preferably by oral route. For oral administration the tablets may of course contain, apart from the above-mentioned excipients, additional excipients such as sodium citrate, calcium carbonate and dicalcium phosphate together with various excipients such as starch, preferably potato starch, gelatine and the like. Moreover, lubricants such as magnesium stearate, sodium lauryl sulfate and talc may be used at the same time for the tabletting process. In the case of aqueous suspensions, the active substances may be combined with various flavour enhancers or colourings in addition to the excipients mentioned above.

For parenteral use, solutions of the active substances with suitable liquid excipients may be used.

The dosage range of the compounds of formula **(I), (I*), (Ia), (Ib), (Ic), (Id), (Ie) or (If)** applicable per day is usually from 1 mg to 2000 mg, preferably from 250 to 1250 mg.

However, it may sometimes be necessary to depart from the amounts specified, depending on the body weight, age, the route of administration, severity of the disease, the individual response to the drug, the nature of its formulation and the time or interval over which the drug is administered (continuous or intermittent treatment with one or multiple doses per day). Thus, in some cases it may be sufficient to use less than the minimum dose given above, whereas in other cases the upper limit may have to be exceeded. When administering large amounts, it may be advisable to divide them up into a number of smaller doses spread over the day.

Thus, in a further aspect the disclosure relates to a pharmaceutical composition comprising at least one (preferably one) compound of formula **(I), (I*), (Ia), (Ib), (Ic), (Id), (Ie)** or **(If)** - or a pharmaceutically acceptable salt thereof - and one or more pharmaceutically acceptable excipient(s).

The compounds of formula **(I), (I*), (Ia), (Ib), (Ic), (Id), (Ie)** or **(If)** - or the pharmaceutically acceptable salts thereof - and the pharmaceutical compositions comprising such compound and salts may also be co-administered with other pharmacologically active substances, *e.g*. with other anti-neoplastic compounds (*e.g.* chemotherapy), *i.e.* used in combination (see combination treatment further below).

The elements of such combinations may be administered (whether dependently or independently) by methods customary to the skilled person and as they are used in monotherapy, *e.g.* by oral, enterical, parenteral (*e.g*., intramuscular, intraperitoneal, intravenous, transdermal or subcutaneous injection, or implant), nasal, vaginal, rectal, or topical routes of administration and may be formulated, alone or together, in suitable dosage unit formulations containing conventional non-toxic pharmaceutically acceptable excipients appropriate for each route of administration.

The combinations may be administered at therapeutically effective single or divided daily doses. The active components of the combinations may be administered in such doses which are therapeutically effective in monotherapy, or in such doses which are lower than the doses used in monotherapy, but when combined result in a desired (joint) therapeutically effective amount.

However, when the combined use of the two or more active substances or principles leads to a synergistic effect, it may also be possible to reduce the amount of one, more or all of the substances or principles to be administered, while still achieving the desired therapeutic action. This may for example be useful for avoiding, limiting or reducing any unwanted side-effects that are associated with the use of one or more of the substances or principles when they are used in their usual amounts, while still obtaining the desired pharmacological or therapeutic effect.

Thus, in a further aspect the disclosure also relates to a pharmaceutical composition comprising a compound of formula **(I), (I*), (Ia), (Ib), (Ic), (Id), (Ie)** or **(If)** - or a pharmaceutically acceptable salt thereof - and one or more (preferably one or two, most preferably one) other pharmacologically active substance(s).

In a further aspect the disclosure also relates to a pharmaceutical preparation comprising a compound of formula **(I), (I*)**, **(Ia)**, **(Ib), (Ic), (Id), (Ie)** or **(If)** - or a pharmaceutically acceptable salt thereof - and one or more (preferably one or two, most preferably one) other pharmacologically active substance(s).

Pharmaceutical compositions to be co-administered or used in combination can also be provided in the form of a kit.

Thus, in a further aspect the disclosure also relates to a kit comprising
- a first pharmaceutical composition or dosage form comprising a compound of formula **(I), (I*)**, **(Ia)**, **(Ib), (Ic), (Id)**, **(Ie)** or **(If)** and, optionally, one or more pharmaceutically acceptable excipient(s), and
- a second pharmaceutical composition or dosage form comprising another pharmacologically active substance and, optionally, one or more pharmaceutically acceptable excipient(s).

In one aspect such kit comprises a third pharmaceutical composition or dosage form comprising still another pharmacologically active substance and, optionally, one or more pharmaceutically acceptable excipient(s).

### Medical Uses - Methods of Treatment

### Indications - patient populations

The present invention is directed to compounds inhibiting KRAS, preferably KRAS mutated at residue 12, such as KRAS G12C, KRAS G12D, KRAS G12V, KRAS G12A and KRAS G12R inhibitors, preferably inhibitors of KRAS G12C and/or KRAS G12D, or inhibitors selective for KRAS G12D, as well as compounds inhibiting KRAS wildtype, preferably amplified, KRAS mutated at residue 13, such as KRAS G13D, or KRAS mutated at residue 61, such as KRAS Q61H. In particular, compounds of formula **(I), (I*), (Ia)**, **(Ib), (Ic), (Id), (Ie)** or **(If)** (including all embodiments thereof) are potentially useful in the treatment and/or prevention of diseases and/or conditions mediated by KRAS, preferably by KRAS mutated at residue 12, e.g. KRAS G12C, KRAS G12D, KRAS G12V, more preferably G12D, or by an amplification of KRAS wildtype, or by KRAS mutated at residue 13, e.g. KRAS G13D, or by KRAS mutated at residue 61, such as KRAS Q61H.

Thus, in a further aspect the disclosure relates to a compound of formula **(I), (I*), (Ia)**, **(Ib)**, **(Ic), (Id)**, **(Ie)** or **(If)** - or a pharmaceutically acceptable salt thereof - for use as a medicament.

In a further aspect the disclosure relates to a compound of formula **(I), (I*), (Ia)**, **(Ib), (Ic), (Id)**, **(Ie)** or **(If)** - or a pharmaceutically acceptable salt thereof - for use in a method of treatment of the human or animal body.

In a further aspect the disclosure relates to a compound of formula **(I), (I*), (Ia)**, **(Ib), (Ic), (Id)**, **(Ie)** or **(If)** - or a pharmaceutically acceptable salt thereof - for use in the treatment and/or prevention of a disease and/or condition mediated by KRAS, preferably by KRAS mutated at residue 12, e.g. KRAS G12C, KRAS G12D, KRAS G12V, more preferably G12D, or by an amplification of KRAS wildtype, or by KRAS mutated at residue 13, e.g. KRAS G13D.

In a further aspect the disclosure relates to a compound of formula **(I), (I*), (Ia)**, **(Ib), (Ic), (Id)**, **(Ie) or (If)** - or a pharmaceutically acceptable salt thereof - for use in the treatment and/or prevention of cancer.

In a further aspect the disclosure relates to a compound of formula **(I), (I*), (Ia)**, **(Ib), (Ic), (Id)**, **(Ie) or (If)** - or a pharmaceutically acceptable salt thereof - for use in a method of treatment and/or prevention of cancer in the human or animal body.

Preferably, the cancer as defined herein (above or below) comprises a KRAS mutation. In particular, KRAS mutations include e.g. mutations of the *KRAS* gene and of the KRAS protein, such as overexpressed KRAS, amplified *KRAS* or KRAS, KRAS mutated at residue 12, KRAS mutated at residue 13, KRAS mutated at residue 61, KRAS mutated at residue 146, in particular KRAS G12A, KRAS G12C, KRAS G12D, KRAS G12V, KRAS G12S, KRAS G13C, KRAS G13D, KRAS G13V, KRAS Q61H, KRAS Q61E, KRAS Q61P, KRAS A146P, KRAS A146T, KRAS A146V. KRAS may present one or more of these mutations/alterations.

Preferably, the cancer as defined herein (above or below) comprises a BRAF mutation in addition or in alternative to the KRAS mutation. Said BRAF mutation is in particular a class III BRAF mutation, e.g. as defined in Z. Yao, Nature, 2017, 548, 234-238.

Preferably, the cancer as defined herein (above or below) comprises a mutation in a receptor tyrosine kinase (RTK), including EGFR, MET and ERBB2 mutations, in addition or in alternative to the KRAS mutation.

In a further aspect the disclosure relates to a compound of formula **(I), (I*), (Ia), (Ib), (Ic), (Id)**, **(Ie)** or **(If)** - or a pharmaceutically acceptable salt thereof - for use in the treatment and/or prevention of cancer, wherein the cancer comprises a KRAS mutation, said KRAS mutation being preferably selected from the group consisting of: KRAS G12C, KRAS G12D, KRAS G12V, KRAS G13D; or an amplification of KRAS wildtype, amplification of the *KRAS* gene or overexpression of KRAS.

In a further aspect the disclosure relates to a compound of formula **(I), (I*), (Ia), (Ib), (Ic), (Id)**, **(Ie)** or **(If)** - or a pharmaceutically acceptable salt thereof - for use in the treatment and/or prevention of cancer, wherein the cancer comprises a KRAS G12D mutation.

In a further aspect the disclosure relates to a compound of formula **(I), (I*), (Ia)**, **(Ib), (Ic), (Id)**, **(Ie)** or **(If)** - or a pharmaceutically acceptable salt thereof - for use in the treatment and/or prevention of cancer, wherein the cancer comprises a KRAS G12V mutation.

In a further aspect the disclosure relates to a compound of formula **(I), (I*), (Ia), (Ib), (Ic), (Id)**, **(Ie)** or **(If)** - or a pharmaceutically acceptable salt thereof - for use in the treatment and/or prevention of cancer, wherein the cancer comprises a KRAS G13D mutation.

In a further aspect the disclosure relates to a compound of formula **(I), (I*), (Ia), (Ib), (Ic), (Id)**, **(Ie)** or **(If)** - or a pharmaceutically acceptable salt thereof - for use in the treatment and/or prevention of cancer, wherein the cancer comprises wildtype amplified KRAS.

Another aspect is based on identifying a link between the KRAS status of a patient and potential susceptibility to treatment with a compound of formula **(I), (I*), (Ia), (Ib), (Ic), (Id)**, **(Ie)** or **(If).** A KRAS inhibitor, such as a compound of formula **(I), (I*), (Ia), (Ib), (Ic), (Id), (Ie)** or **(If),** may then advantageously be used to treat patients with a disease dependent on KRAS who may be resistant to other therapies. This therefore provides opportunities, methods and tools for selecting patients for treatment with a compound of formula **(I), (I*), (Ia), (Ib), (Ic), (Id), (Ie)** or **(If),** particularly cancer patients. The selection is based on whether the tumor cells to be treated possess wild-type, preferably amplified, or KRAS mutated at residue 12, preferably G12C, G12D or G12V gene, or KRAS mutated at residue 13, preferably G13D gene. The KRAS gene status could therefore be used as a biomarker to indicate that selecting treatment with a compound of formula **(I), (I*), (Ia)**, **(Ib), (Ic), (Id), (Ie)** or **(If)** may be advantageous.

According to one aspect, a compound of formula **(I), (I*), (Ia)**, **(Ib), (Ic), (Id), (Ie)** or **(If)** for use in a method for selecting a patient for treatment with a compound of formula **(I), (I*), (Ia)**, **(Ib), (Ic), (Id), (Ie)** or **(If),** the method comprising
- providing a tumor cell-containing sample from a patient;
- determining whether the KRAS gene in the patient's tumor cell-containing sample encodes for wild-type (glycine at position 12) or mutant (cysteine, aspartic acid, valine, alanine or aginine at position 12, aspartic acid at position 13, amplification and/or overexpression) KRAS protein; and
- selecting a patient for treatment with a compound of formula **(I), (I*), (Ia), (Ib), (Ic), (Id)**, **(Ie)** or **(If)** based thereon.

The method may include or exclude the actual patient sample isolation step.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a KRAS mutation or an amplification of KRAS wildtype.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a G12C mutant, G12D mutant, G12V mutant, G12A mutant, G13D mutant or G12R mutant KRAS gene or an amplification of KRAS wildtype.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a G12C mutant, G12D mutant, G12V mutant or G13D mutant KRAS gene or an amplification of KRAS wildtype.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a G12D mutant KRAS gene.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a G12V mutant KRAS gene.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring a G13D mutant KRAS gene.

According to another aspect, there is provided a compound of formula **(I), (I*), (Ia), (Ib), (Ic), (Id), (Ie)** or **(If)** - or a pharmaceutically acceptable salt thereof - for use in treating a cancer with tumor cells harbouring wildtype amplified KRAS or overexpressed KRAS.

Determining whether a tumor or cancer comprises a G12C KRAS mutation can be undertaken by assessing the nucleotide sequence encoding the KRAS protein, by assessing the amino acid sequence of the KRAS, protein, or by assessing the characteristics of a putative KRAS mutant protein. The sequence of wild-type human KRAS is known in the art. Methods for detecting a mutation in a KRAS nucleotide sequence are known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct sequencing, primer extension reactions, electrophoresis, oligonucleotide ligation assays, hybridization assays, TaqMan assays, SNP genotyping assays, high resolution melting assays and microarray analyses. In some embodiments, samples are evaluated for G12C KRAS mutations by real-time PCR. In real-time PCR, fluorescent probes specific for the KRAS G12C mutation are used. When a mutation is present, the probe binds and fluorescence is detected. In some embodiments, the KRAS G12C mutation is identified using a direct sequencing method of specific regions (e.g. exon 2 and/or exon 3) in the KRAS gene. This technique will identify all possible mutations in the region sequenced. Methods for detecting a mutation in a KRAS protein are known by those of skill in the art. These methods include, but are not limited to, detection of a KRAS mutant using a binding agent (e.g. an antibody) specific for the mutant protein, protein electrophoresis, Western blotting and direct peptide sequencing.

Methods for determining whether a tumor or cancer comprises a G12C KRAS mutation can use a variety of samples. In some embodiments, the sample is taken from a subject having a tumor or cancer. In some embodiments, the sample is a fresh tumor/cancer sample. In some embodiments, the sample is a frozen tumor/cancer sample. In some embodiments, the sample is a formalin-fixed paraffin-embedded sample. In some embodiments, the sample is processed to a cell lysate. In some embodiments, the sample is processed to DNA or RNA. In some embodiments the sample is a liquid biopsy and the test is done on a sample of blood to look for cancer cells from a tumor that are circulating in the blood or for pieces of DNA from tumor cells that are in the blood.

Analogously it can be determined whether a tumor or cancer comprises a KRAS G12D, KRAS G12V, KRAS G12A, KRAS G13D and KRAS G12R mutation or is a KRAS wildtype, preferably amplified.

Preferably, a compound of formula **(I), (I*), (Ia)**, **(Ib), (Ic), (Id)**, **(Ie)** or **(If)** - or a pharmaceutically acceptable salt thereof - for use in the treatment/prevention of a disease/condition/cancer/tumors/cancer cells according to the methods and uses as herein (above and below) defined and disclosed is selected from the group consisting of pancreatic cancer, lung cancer, colorectal cancer, cholangiocarcinoma, appendiceal cancer, multiple myeloma, melanoma, uterine cancer, endometrial cancer, thyroid cancer, acute myeloid leukaemia, bladder cancer, urothelial cancer, gastric cancer, cervical cancer, head and neck squamous cell carcinoma, diffuse large B cell lymphoma, oesophageal cancer, gastroesophageal cancer, chronic lymphocytic leukaemia, hepatocellular cancer, breast cancer, ovarian cancer, prostate cancer, glioblastoma, renal cancer and sarcomas.

Preferably, a compound of formula **(I), (I*), (Ia), (Ib), (Ic), (Id), (Ie)** or **(If)** - or a pharmaceutically acceptable salt thereof - for use in the treatment/prevention of a disease/condition/cancer/tumors/cancer cells to be treated/prevented according to the methods and uses as herein (above and below) defined and disclosed is selected from the group consisting of: pancreatic cancer, lung cancer, ovarian cancer, colorectal cancer (CRC), gastric cancer, gastroesophageal junction cancer (GEJC) and esophageal cancer.

In another aspect, a compound of formula **(I), (I*), (Ia), (Ib), (Ic), (Id), (Ie)** or **(If)** - or a pharmaceutically acceptable salt thereof - for use in the treatment/prevention of a disease/condition/cancer/tumors/cancer cells according to the methods and uses as herein (above and below) defined and disclosed is selected from the group consisting of pancreatic cancer (preferably pancreatic ductal adenocarcinoma (PDAC)), lung cancer (preferably non-small cell lung cancer (NSCLC)), gastric cancer, cholangiocarcinoma and colorectal cancer (preferably colorectal adenocarcinoma). Preferably, said pancreatic cancer, lung cancer, cholangiocarcinoma, colorectal cancer (CRC), pancreatic ductal adenocarcinoma (PDAC), non-small cell lung cancer (NSCLC) or colorectal adenocarcinoma comprises a KRAS mutation, in particular a KRAS G12D or KRAS G12V mutation. Preferably (in alternative or in combination with the previous preferred embodiment), said non-small cell lung cancer (NSCLC) comprises a mutation (in particular a loss-of-function mutation) in the *NF1* gene.

In another aspect, a compound of formula **(I), (I*), (Ia), (Ib), (Ic), (Id), (Ie)** or **(If)** - or a pharmaceutically acceptable salt thereof - for use in the treatment/prevention of a disease/condition/cancer/tumors/cancer cells according to the methods and uses as herein (above and below) defined and disclosed is gastric cancer, ovarian cancer or esophageal cancer, said gastric cancer or esophageal cancer being preferably selected from the group consisting of: gastric adenocarcinoma (GAC), esophageal adenocarcinoma (EAC) and gastroesophageal junction cancer (GEJC). Preferably, said gastric cancer, ovarian cancer, esophageal cancer, gastric adenocarcinoma (GAC), esophageal adenocarcinoma (EAC) or gastroesophageal junction cancer (GEJC) comprises a KRAS mutation or wildtype amplified KRAS.

Particularly preferred, a compound of formula **(I), (I*), (Ia), (Ib), (Ic), (Id), (Ie)** or **(If)** - or a pharmaceutically acceptable salt thereof - for use in the treatment/prevention of cancer according to the methods and uses as herein (above and below) defined and disclosed is selected from the group consisting of:
- lung adenocarcinoma (preferably non-small cell lung cancer (NSCLC)) harbouring a KRAS mutation at position 12 (preferably a G12C, G12D, G12V, G12A, G12R mutation), at position 13 (preferably G13D) or an amplification of KRAS wildtype;
- colorectal adenocarcinoma harbouring a KRAS mutation at position 12 (preferably a G12C, G12D, G12V, G12A, G12R mutation), at position 13 (preferably G13D) or an amplification of KRAS wildtype;
- pancreatic adenocarcinoma (preferably pancreatic ductal adenocarcinoma (PDAC)) harbouring a RAS mutation at position 12 (preferably a KRAS and preferably a G12C, G12D, G12V, G12A, G12R mutation), at position 13 (preferably G13D) or an amplification of KRAS wildtype.

Preferably, "cancer" as used herein (above or below) includes drug-resistant cancer and cancer that has failed one, two or more lines of mono- or combination therapy with one or more anti-cancer agents. In particular, "cancer" (and any embodiment thereof) refers to any cancer (especially the cancer species defined hereinabove and hereinbelow) that is resistant to treatment with a KRAS G12C inhibitor.

Different resistance mechanisms have already been reported. For example, the following articles describe resistance in patients following treatment with a KRAS G12C inhibitor: (i) Awad MM, Liu S, Rybkin, II, Arbour KC, Dilly J, Zhu VW, et al. Acquired resistance to KRAS(G12C) inhibition in cancer. N Engl J Med 2021;384:2382-93 and (ii) Tanaka N, Lin JJ, Li C, Ryan MB, Zhang J, Kiedrowski LA, et al. Clinical acquired resistance to KRAS(G12C) inhibition through a novel KRAS switch-II pocket mutation and polyclonal alterations converging on RAS-MAPK reactivation. Cancer Discov 2021;11:1913-22.

In another aspect, a compound of formula **(I), (I*), (Ia), (Ib), (Ic), (Id), (Ie)** or **(If)** - or a pharmaceutically acceptable salt thereof - for use in the treatment/prevention of a disease/condition/cancer/tumors/cancer cells according to the methods and uses as herein (above and below) defined and disclosed is a RASopathy, preferably selected from the group consisting of Neurofibromatosis type 1 (NF1), Noonan Syndrome (NS), Noonan Syndrome with Multiple Lentigines (NSML) (also referred to as LEOPARD syndrome), Capillary Malformation-Arteriovenous Malformation Syndrome (CM-AVM), Costello Syndrome (CS), Cardio-Facio-Cutaneous Syndrome (CFC), Legius Syndrome (also known as NF1-like Syndrome) and Hereditary gingival fibromatosis.

Additionally, a compound of formula **(I), (I*), (Ia), (Ib), (Ic), (Id), (Ie)** or **(If)** - or a pharmaceutically acceptable salt thereof - for use in the treatment of the following cancers, tumors and other proliferative diseases without being restricted thereto. Preferably, the methods of treatment, methods, uses, compounds for use and pharmaceutical compositions for use as disclosed herein (above and below) are applied in treatments of diseases/conditions/cancers/tumors which (*i.e.* the respective cells) harbour a KRAS mutation at position 12 (preferably a G12C, G12D, G12V, G12A, G12R mutation) or an amplification of KRAS wildtype alternatively they have been identified to harbour a KRAS mutation at position 12 (preferably a G12C, G12D, G12V, G12A, G12R mutation) as herein described and/or referred or an amplification of KRAS wildtype:
cancers/tumors/carcinomas of the head and neck: *e.g*. tumors/carcinomas/cancers of the nasal cavity, paranasal sinuses, nasopharynx, oral cavity (including lip, gum, alveolar ridge, retromolar trigone, floor of mouth, tongue, hard palate, buccal mucosa), oropharynx (including base of tongue, tonsil, tonsillar pilar, soft palate, tonsillar fossa, pharyngeal wall), middle ear, larynx (including supraglottis, glottis, subglottis, vocal cords), hypopharynx, salivary glands (including minor salivary glands);
cancers/tumors/carcinomas of the lung: *e.g*. non-small cell lung cancer (NSCLC) (squamous cell carcinoma, spindle cell carcinoma, adenocarcinoma, large cell carcinoma, clear cell carcinoma, bronchioalveolar), small cell lung cancer (SCLC) (oat cell cancer, intermediate cell cancer, combined oat cell cancer);
neoplasms of the mediastinum: *e.g*. neurogenic tumors (including neurofibroma, neurilemoma, malignant schwannoma, neurosarcoma, ganglioneuroblastoma, ganglioneuroma, neuroblastoma, pheochromocytoma, paraganglioma), germ cell tumors (including seminoma, teratoma, non-seminoma), thymic tumors (including thymoma, thymolipoma, thymic carcinoma, thymic carcinoid), mesenchymal tumors (including fibroma, fibrosarcoma, lipoma, liposarcoma, myxoma, mesothelioma, leiomyoma, leiomyosarcoma, rhabdomyosarcoma, xanthogranuloma, mesenchymoma, hemangioma, hemangioendothelioma, hemangiopericytoma, lymphangioma, lymphangiopericytoma, lymphangiomyoma);
cancers/tumors/carcinomas of the gastrointestinal (GI) tract: *e.g*. tumors/carcinomas/ cancers of the esophagus, stomach (gastric cancer), gastroesophageal junction cancer pancreas, liver and biliary tree (including hepatocellular carcinoma (HCC), *e.g*. childhood HCC, fibrolamellar HCC, combined HCC, spindle cell HCC, clear cell HCC, giant cell HCC, carcinosarcoma HCC, sclerosing HCC; hepatoblastoma; cholangiocarcinoma; cholangiocellular carcinoma; hepatic cystadenocarcinoma; angiosarcoma, hemangioendothelioma, leiomyosarcoma, malignant schwannoma, fibrosarcoma, Klatskin tumor), gall bladder, extrahepatic bile ducts, small intestine (including duodenum, jejunum, ileum), large intestine (including cecum, colon, rectum, anus; colorectal cancer, gastrointestinal stroma tumor (GIST)), genitourinary system (including kidney, e.g. renal pelvis, renal cell carcinoma (RCC), nephroblastoma (Wilms' tumor), hypernephroma, Grawitz tumor; ureter; urinary bladder, e.g. urachal cancer, urothelial cancer; urethra, e.g. distal, bulbomembranous, prostatic; prostate (androgen dependent, androgen independent, castration resistant, hormone independent, hormone refractory), penis) gastric cancer;
cancers/tumors/carcinomas of the testis: *e.g*. seminomas, non-seminomas,
gynecologic cancers/tumors/carcinomas: *e.g*. tumors/carcinomas/cancers of the ovary, fallopian tube, peritoneum, cervix, vulva, vagina, uterine body (including endometrium, fundus);
cancers/tumors/carcinomas of the breast: e.g. mammary carcinoma (infiltrating ductal, colloid, lobular invasive, tubular, adenocystic, papillary, medullary, mucinous), hormone receptor positive breast cancer (estrogen receptor positive breast cancer, progesterone receptor positive breast cancer), Her2 positive breast cancer, triple negative breast cancer, Paget's disease of the breast;
cancers/tumors/carcinomas of the endocrine system: e.g. tumors/carcinomas/cancers of the endocrine glands, thyroid gland (thyroid carcinomas/tumors; papillary, follicular, anaplastic, medullary), parathyroid gland (parathyroid carcinoma/tumor), adrenal cortex (adrenal cortical carcinoma/tumors), pituitary gland (including prolactinoma, craniopharyngioma), thymus, adrenal glands, pineal gland, carotid body, islet cell tumors, paraganglion, pancreatic endocrine tumors (PET; non-functional PET, PPoma, gastrinoma, insulinoma, VIPoma, glucagonoma, somatostatinoma, GRFoma, ACTHoma), carcinoid tumors;
sarcomas of the soft tissues: *e.g.* fibrosarcoma, fibrous histiocytoma, liposarcoma, leiomyosarcoma, rhabdomyosarcoma, angiosarcoma, lymphangiosarcoma, Kaposi's sarcoma, glomus tumor, hemangiopericytoma, synovial sarcoma, giant cell tumor of tendon sheath, solitary fibrous tumor of pleura and peritoneum, diffuse mesothelioma, malignant peripheral nerve sheath tumor (MPNST), granular cell tumor, clear cell sarcoma, melanocytic schwannoma, plexosarcoma, neuroblastoma, ganglioneuroblastoma, neuroepithelioma, extraskeletal Ewing's sarcoma, paraganglioma, extraskeletal chondrosarcoma, extraskeletal osteosarcoma, mesenchymoma, alveolar soft part sarcoma, epithelioid sarcoma, extrarenal rhabdoid tumor, desmoplastic small cell tumor;
sarcomas of the bone: *e.g*. myeloma, reticulum cell sarcoma, chondrosarcoma (including central, peripheral, clear cell, mesenchymal chondrosarcoma), osteosarcoma (including parosteal, periosteal, high-grade surface, small cell, radiation-induced osteosarcoma, Paget's sarcoma), Ewing's tumor, malignant giant cell tumor, adamantinoma, (fibrous) histiocytoma, fibrosarcoma, chordoma, small round cell sarcoma, hemangioendothelioma, hemangiopericytoma, osteochondroma, osteoid osteoma, osteoblastoma, eosinophilic granuloma, chondroblastoma;
mesothelioma: *e.g*. pleural mesothelioma, peritoneal mesothelioma;
cancers of the skin: e.g. basal cell carcinoma, squamous cell carcinoma, Merkel's cell carcinoma, melanoma (including cutaneous, superficial spreading, lentigo maligna, acral lentiginous, nodular, intraocular melanoma), actinic keratosis, eyelid cancer;
neoplasms of the central nervous system and brain: e.g. astrocytoma (cerebral, cerebellar, diffuse, fibrillary, anaplastic, pilocytic, protoplasmic, gemistocytary), glioblastoma, gliomas, oligodendrogliomas, oligoastrocytomas, ependymomas, ependymoblastomas, choroid plexus tumors, medulloblastomas, meningiomas, schwannomas, hemangioblastomas, hemangiomas, hemangiopericytomas, neuromas, ganglioneuromas, neuroblastomas, retinoblastomas, neurinomas (e.g. acoustic), spinal axis tumors;
lymphomas and leukemias: e.g. B-cell non-Hodgkin lymphomas (NHL) (including small lymphocytic lymphoma (SLL), lymphoplasmacytoid lymphoma (LPL), mantle cell lymphoma (MCL), follicular lymphoma (FL), diffuse large cell lymphoma (DLCL), Burkitt's lymphoma (BL)), T-cell non-Hodgkin lymphomas (including anaplastic large cell lymphoma (ALCL), adult T-cell leukemia/lymphoma (ATLL), cutaneous T-cell lymphoma (CTCL), peripheral T-cell lymphoma (PTCL)), lymphoblastic T-cell lymphoma (T-LBL), adult T-cell lymphoma, lymphoblastic B-cell lymphoma (B-LBL), immunocytoma, chronic B-cell lymphocytic leukemia (B-CLL), chronic T-cell lymphocytic leukemia (T-CLL) B-cell small lymphocytic lymphoma (B-SLL), cutaneous T-cell lymphoma (CTLC), primary central nervous system lymphoma (PCNSL), immunoblastoma, Hodgkin's disease (HD) (including nodular lymphocyte predominance HD (NLPHD), nodular sclerosis HD (NSHD), mixed-cellularity HD (MCHD), lymphocyte-rich classic HD, lymphocyte-depleted HD (LDHD)), large granular lymphocyte leukemia (LGL), chronic myelogenous leukemia (CML), acute myelogenous/myeloid leukemia (AML), acute lymphatic/lymphoblastic leukemia (ALL), acute promyelocytic leukemia (APL), chronic lymphocytic/lymphatic leukemia (CLL), prolymphocytic leukemia (PLL), hairy cell leukemia, chronic myelogenous/myeloid leukemia (CML), myeloma, plasmacytoma, multiple myeloma (MM), plasmacytoma, myelodysplastic syndromes (MDS), chronic myelomonocytic leukemia (CMML);
cancers of unknown primary site (CUP);

All cancers/tumors/carcinomas mentioned above which are characterized by their specific location/origin in the body are meant to include both the primary tumors and the metastatic tumors derived therefrom.

All cancers/tumors/carcinomas mentioned above may be further differentiated by their histopathological classification:
Epithelial cancers, e.g. squamous cell carcinoma (SCC) (carcinoma *in situ,* superficially invasive, verrucous carcinoma, pseudosarcoma, anaplastic, transitional cell, lymphoepithelial), adenocarcinoma (AC) (well-differentiated, mucinous, papillary, pleomorphic giant cell, ductal, small cell, signet-ring cell, spindle cell, clear cell, oat cell, colloid, adenosquamous, mucoepidermoid, adenoid cystic), mucinous cystadenocarcinoma, acinar cell carcinoma, large cell carcinoma, small cell carcinoma, neuroendocrine tumors (small cell carcinoma, paraganglioma, carcinoid); oncocytic carcinoma;
Nonepithilial cancers, e.g. sarcomas (fibrosarcoma, chondrosarcoma, rhabdomyosarcoma, leiomyosarcoma, hemangiosarcoma, giant cell sarcoma, lymphosarcoma, fibrous histiocytoma, liposarcoma, angiosarcoma, lymphangiosarcoma, neurofibrosarcoma), lymphoma, melanoma, germ cell tumors, hematological neoplasms, mixed and undifferentiated carcinomas;
The compounds of the disclosure may be used in therapeutic regimens in the context of first line, second line, or any further line treatments.

The compounds of the disclosure may be used for the prevention, short-term or long-term treatment of the above-mentioned diseases/conditions/cancers/tumors, optionally also in combination with radiotherapy and/or surgery.

The methods of treatment, methods, uses and compounds for use as disclosed herein (above and below) can be performed with any compound of formula **(I), (I*), (Ia), (Ib), (Ic), (Id)**, **(Ie)** or **(If)** - or a pharmaceutically acceptable salt thereof - as disclosed or defined herein and with any pharmaceutical composition or kit comprising a compound of formula **(I), (I*), (Ia), (Ib), (Ic), (Id), (Ie)** or **(If)** - or a pharmaceutically acceptable salt thereof (each including all individual embodiments or generic subsets of compounds of formula **(I), (I*), (Ia), (Ib), (Ic), (Id)**, **(Ie)** or **(If)).**

### Combination treatment

The compounds of formula **(I), (I*), (Ia), (Ib), (Ic), (Id)**, **(Ie)** or **(If)** - or the pharmaceutically acceptable salts thereof - and the pharmaceutical compositions comprising such compounds or salts may also be co-administered with other pharmacologically active substances, *e.g*. with other anti-neoplastic compounds (*e.g*. chemotherapy), or used in combination with other treatments, such as radiation or surgical intervention, either as an adjuvant prior to surgery or post-operatively. Preferably, the pharmacologically active substance(s) for co-administration is/are (an) anti-neoplastic compound(s).

Thus, in a further aspect the disclosure relates to a compound of formula **(I), (I*), (Ia)**, **(Ib)**, **(Ic), (Id), (Ie)** or **(If)** - or a pharmaceutically acceptable salt thereof - for use as hereinbefore defined wherein said compound is administered before, after or together with one or more other pharmacologically active substance(s).

In a further aspect the disclosure relates to a compound of formula **(I), (I*), (Ia)**, **(Ib), (Ic), (Id)**, **(Ie)** or **(If)** - or a pharmaceutically acceptable salt thereof - for use as hereinbefore defined, wherein said compound is administered in combination with one or more other pharmacologically active substance(s).

In a further aspect the disclosure relates to a compound of formula **(I), (I*), (Ia)**, **(Ib), (Ic), (Id), (Ie)** or **(If)** - or a pharmaceutically acceptable salt thereof - for use in the treatment and/or prevention of cancer, wherein the compound of formula **(I), (I*), (Ia)**, **(Ib), (Ic), (Id), (Ie)** or **(If)** - or a pharmaceutically acceptable salt thereof - is administered simultaneously, concurrently, sequentially, successively, alternately or separately with the one or more other pharmacologically active substance(s).

In a further aspect the disclosure relates to an inhibitor of a KRAS mutated at residue 12 or 13, such as KRAS G12C, KRAS G12D, KRAS G12V, KRAS G12A, KRAS G13D and/or KRAS G12R inhibitors, preferably KRAS G12C, KRAS G12D or selective KRAS G12D inhibitors - or a pharmaceutically acceptable salt thereof - for use in the treatment and/or prevention of cancer, wherein the inhibitor - or a pharmaceutically acceptable salt thereof - is administered in combination with the one or more other pharmacologically active substance(s).

In a further aspect the disclosure relates to an inhibitor of an inhibitor of KRAS wildtype amplified or overexpressed - or a pharmaceutically acceptable salt thereof - for use in the treatment and/or prevention of cancer, wherein the inhibitor - or a pharmaceutically acceptable salt thereof - is administered in combination with the one or more other pharmacologically active substance(s).

In a further aspect the disclosure relates to a kit comprising
- a first pharmaceutical composition or dosage form comprising a compound of formula **(I), (I*)**, **(Ia)**, **(Ib)**, **(Ic), (Id)**, **(Ie)** or **(If)** - or a pharmaceutically acceptable salt thereof - and, optionally, one or more pharmaceutically acceptable excipient(s), and
- a second pharmaceutical composition or dosage form comprising another pharmacologically active substance, and, optionally, one or more pharmaceutically acceptable excipient(s),
for use in the treatment and/or prevention of cancer, wherein the first pharmaceutical composition is to be administered simultaneously, concurrently, sequentially, successively, alternately or separately with the second and/or additional pharmaceutical composition or dosage form.

In one aspect such kit for said use comprises a third pharmaceutical composition or dosage form comprising a third pharmaceutical composition or dosage form comprising still another pharmacologically active substance, and, optionally, one or more pharmaceutically acceptable excipient(s)

In a further embodiment of the disclosure the components (*i.e.* the combination partners) of the combinations, kits, uses, methods and compounds for use according to the disclosure (including all embodiments) are administered simultaneously.

In a further embodiment of the disclosure the components (*i.e.* the combination partners) of the combinations, kits, uses, methods and compounds for use according to the disclosure (including all embodiments) are administered concurrently.

In a further embodiment of the disclosure the components (*i.e.* the combination partners) of the combinations, kits, uses, methods and compounds for use according to the disclosure (including all embodiments) are administered sequentially.

In a further embodiment of the disclosure the components *(i.e.* the combination partners) of the combinations, kits, uses, methods and compounds for use according to the disclosure (including all embodiments) are administered successively.

In a further embodiment of the disclosure the components *(i.e.* the combination partners) of the combinations, kits, uses, methods and compounds for use according to the disclosure (including all embodiments) are administered alternately.

In a further embodiment of the disclosure the components (*i.e.* the combination partners) of the combinations, kits, uses, methods and compounds for use according to the disclosure (including all embodiments) are administered separately.

The pharmacologically active substance(s) to be used together/in combination with the compound of formula **(I), (I*)**, **(Ia)**, **(Ib), (Ic), (Id), (Ie)** or **(If)** - or a pharmaceutically acceptable salt thereof - (including all individual embodiments or generic subsets of compounds) or in the medical uses, uses, methods of treatment and/or prevention, pharmaceutical compositions as herein (above and below) defined can be selected from any one or more of the following (preferably there is one or two additional pharmacologically active substance used in all these embodiments):
**1. an inhibitor of EGFR and/or ErbB2 (HER2) and/or ErbB3 (HER3) and/or ErbB4 (HER4) or of any mutants thereof**
   a. irreversible inhibitors: *e.g.* afatinib, dacomitinib, canertinib, neratinib, avitinib, poziotinib, AV 412, PF-6274484, HKI 357, olmutinib, osimertinib, almonertinib, nazartinib, lazertinib, pelitinib;
   b. reversible inhibitors: *e.g*. erlotinib, gefitinib, icotinib, sapitinib, lapatinib, varlitinib, vandetanib, TAK-285, AEE788, BMS599626/AC-480, GW 583340;
   c. *anti*-EGFR antibodies: *e.g*. necitumumab, panitumumab, cetuximab, amivantamab;
   d. *anti*-HER2 antibodies: *e.g*. pertuzumab, trastuzumab, trastuzumab emtansine;
   e. inhibitors of mutant EGFR;
   f. an inhibitor of HER2 with exon 20 mutations;
   g. preferred irreversible inhibitor is afatinib;
   h. preferred *anti*-EGFR antibody is cetuximab.
**2. an inhibitor of MEK and/or of mutants thereof**
   a. *e.g*. trametinib, cobimetinib, binimetinib, selumetinib, refametinib;
   b. preferred is trametinib
   c. a MEK inhibitor as disclosed in WO 2013/136249;
   d. a MEK inhibitor as disclosed in WO 2013/136254
3. **an inhibitor of SOS1 and/or of any mutants thereof** (*i.e.* a compound that modulates/inhibits the GEF functionality of SOS1, *e.g*. by binding to SOS1 and preventing protein-protein interaction between SOS1 and a (mutant) Ras protein, *e.g*. KRAS)
   a. *e.g*. BAY-293;
   b. a SOS1 inhibitor as disclosed in WO 2018/115380;
   c. a SOS1 inhibitor as disclosed in WO 2019/122129;
   d. a SOS1 inhibitor as disclosed in WO 2020/180768, WO 2020/180770, WO 2018/172250 and WO 2019/201848.
**4. an oncolytic virus**
**5. a RAS vaccine**
   a. *e.g*. TG02 (Targovax).
**6. a cell cycle inhibitor**
   a. *e.g*. inhibitors of CDK4/6 and/or of any mutants therof
      i. *e.g*. palbociclib, ribociclib, abemaciclib, trilaciclib, PF-06873600;
      ii. preferred are palbociclib and abemaciclib;
      iii. most preferred is abemaciclib.
   b. *e.g*. vinca alkaloids
      i. *e.g*. vinorelbine.
   c. *e.g*. inhibitors of Aurora kinase and/or of any mutants therof
      i. *e.g*. alisertib, barasertib.
**7. an inhibitor of PTK2** (= **FAK) and/or of any mutants thereof**
   a. *e.g*. TAE226, BI 853520.
**8. an inhibitor of SHP2 and/or of any mutants thereof**
   a. *e.g*. SHP099, TNO155, RMC-4550, RMC-4630, IACS-13909.
**9. an inhibitor of PI3 kinase** (= **PI3K) and/or of any mutants thereof**
   a. *e.g*. inhibitors of PI3Kα and/or of any mutants therof
      i. *e.g*. alpelisib, serabelisib, GDC-0077, HH-CYH33, AMG 511, buparlisib, dactolisib, pictilisib, taselisib.
10. **an inhibitor of FGFR1 and/or FGFR2 and/or FGFR3 and/or of any mutants thereof**
   a. *e.g*. ponatinib, infigratinib, nintedanib.
**11. an inhibitor of AXL and/or of any mutants thereof**
**12. a taxane**
   a. *e.g.* paclitaxel, nab-paclitaxel, docetaxel;
   b. preferred is paclitaxel.
**13. a platinum-containing compound**
   a. *e.g.* cisplatin, carboplatin, oxaliplatin
   b. preferred is oxaliplatin.
**14. an anti-metabolite**
   a. *e.g.* 5-fluorouracil, capecitabine, floxuridine, cytarabine, gemcitabine, pemetrexed, combination of trifluridine and tipiracil (= TAS102);
   b. preferred is 5-fluorouracil.
**15. an immunotherapeutic agent**
   a. *e.g.* an immune checkpoint inhibitor
      i. *e.g.* an anti-CTLA4 mAb, *anti*-PD1 mAb, *anti-*PD-L1 mAb, *anti*-PD-L2 mAb, *anti-LAG3* mAb, *anti*-TIM3 mAb;
      ii. preferred is an *anti-*PD1 mAb;
      iii. *e.g.* ipilimumab, nivolumab, pembrolizumab, tislelizumab atezolizumab, avelumab, durvalumab, pidilizumab, PDR-001 (= spartalizumab), AMG-404, ezabenlimab;
      iv. preferred are nivolumab, pembrolizumab, ezabenlimab and PDR-001 (= spartalizumab);
      v. most preferred is ezabenlimab, pembrolizumab and nivolumab.
**16. a topoisomerase inhibitor**
   a. *e.g.* irinotecan, liposomal irinotecan (nal-IRI), topotecan, etoposide;
   b. most preferred is irinotecan and liposomal irinotecan (nal-IRI).
**17. an inhibitor of A-Raf and/or B-Raf and/or C-Raf and/or of any mutants thereof**
   a. *e.g.* encorafenib, dabrafenib, vemurafenib, PLX-8394, RAF-709 (= example 131 in WO 2014/151616), LXH254, sorafenib, LY-3009120 (= example 1 in WO 2013/134243), lifirafenib, TAK-632, agerafenib, CCT196969, RO5126766, RAF265.
**18. an inhibitor of *m*TOR**
   a. *e.g.* rapamycin, temsirolimus, everolimus, ridaforolimus, zotarolimus, sapanisertib, Torin 1, dactolisib, GDC-0349, VS-5584, vistusertib, AZD8055.
**19. an epigenetic regulator**
   a. *e.g.* a BET inhibitor
      i. *e.g.* JQ-1, GSK 525762, OTX-015, CPI-0610, TEN-010, OTX-015, PLX51107, ABBV-075, ABBV-744, BMS986158, TGI-1601, CC-90010, AZD5153, I-BET151, BI 894999;
**20. an inhibitor of IGF1/2 and/or of IGF1-R and/or of any mutants thereof**
   a. *e.g.* xentuzumab (antibody 60833 in WO 2010/066868), MEDI-573 (= dusigitumab), linsitinib.
**21. an inhibitor of a Src family kinase and/or of any mutants thereof**
   a. *e.g.* an inhibitor of a kinase of the SrcA subfamily and/or of any mutants thereof, *i.e.* an inhibitor of Src, Yes, Fyn, Fgr and/or of any mutants thereof;
   b. *e.g.* an inhibitor of a kinase of the SrcB subfamily and/or of any mutants thereof, *i.e.* an inhibitor of Lck, Hck, Blk, Lyn and/or of any mutants thereof;
   c. *e.g.* an inhibitor of a kinase of the Frk subfamily and/or of any mutants thereof, *i.e.* an inhibitor of Frk and/or of any mutants thereof;
   *d. e.g.* dasatinib, ponatinib, bosutinib, vandetanib, KX-01, saracatinib, KX2-391, SU 6656, WH-4-023.
**22. an apoptose regulator**
   a. *e.g.* an MDM2 inhibitor, e.g. an inhibitor of the interaction between p53 (preferably functional p53, most preferably *wt* p53) and MDM2 and/or of any mutants thereof;
      i. *e.g.* HDM-201, NVP-CGM097, RG-7112, MK-8242, RG-7388, SAR405838, AMG-232, DS-3032, RG-7775, APG-115;
      ii. preferred are HDM-201, RG-7388 and AMG-232;
      iii. an MDM2 inhibitor as disclosed in WO 2015/155332;
      iv. an MDM2 inhibitor as disclosed in WO 2016/001376;
      v. an MDM2 inhibitor as disclosed in WO 2016/026937;
      vi. an MDM2 inhibitor as disclosed in WO 2017/060431;
   b. *e.g.* a PARP inhibitor;
   c. *e.g.* an MCL-1 inhibitor;
      i. *e.g.* AZD-5991, AMG-176, AMG-397, S64315, S63845, A-1210477;
**23. an inhibitor of c-MET and/or of any mutants thereof**
   a. *e.g.* savolitinib, cabozantinib, foretinib;
   b. MET antibodies, *e.g.* emibetuzumab, amivantamab;
**24. an inhibitor of ERK and/or of any mutants thereof**
   a. *e.g.* ulixertinib, LTT462;
**25. an inhibitor of farnesyl transferase and/or of any mutants thereof**
   a. *e.g.* tipifarnib;
**26. an inhibitor of YAP1, WWTR1, TEAD1, TEAD2, TEAD3 and / or TEAD4**
   a. reversible inhibitors of TEAD transcription factors (e.g. disclosed in WO 2018/204532);
   b. irreversible inhibitors of TEAD transcription factors (e.g. disclosed in WO 2020/243423);
   c. protein-protein interaction inhibitors of the YAP/TAZ::TEAD interaction (e.g. disclosed in WO 2021/186324);
   d. inhibitors of TEAD palmitoylation.

In a further embodiment the compound of formula **(I), (I*), (Ia), (Ib), (Ic), (Id), (Ie)** or **(If)** - or a pharmaceutically acceptable salt thereof - for (combined) use in a method (*e.g*. method for the treatment and/or prevention) as hereinbefore described, wherein one other pharmacologically active substance is to be administered before, after or together with the compound of formula **(I), (I*), (Ia), (Ib), (Ic), (Id), (Ie)** or **(If)** - or a pharmaceutically acceptable salt thereof - wherein said one other pharmacologically active substance is
- a SOS1 inhibitor; or
- a MEK inhibitor; or
- trametinib, or
- an *anti*-PD-1 antibody; or
- ezabenlimab; or
- cetuximab; or
- afatinib; or
- standard of care (SoC) in a given indication; or
- a PI3 kinase inhibitor; or
- an inhibitor of TEAD palmitoylation; or
- a YAP/TAZ::TEAD inhibitor.

In a further embodiment the compound of formula **(I), (I*)**, **(Ia)**, **(Ib), (Ic), (Id), (Ie)** or **(If)** - or a pharmaceutically acceptable salt thereof - for (combined) use in a method (*e.g*. method for the treatment and/or prevention) as hereinbefore described, wherein one other pharmacologically active substance is to be administered in combination with the compound of formula **(I), (I*), (Ia), (Ib), (Ic), (Id), (Ie)** or **(If)** - or a pharmaceutically acceptable salt thereof - wherein said one other pharmacologically active substance is
- a SOS1 inhibitor; or
- a MEK inhibitor; or
- trametinib; or
- an *anti*-PD-1 antibody; or
- ezabenlimab; or
- cetuximab; or
- afatinib; or
- standard of care (SoC) in a given indication; or
- a PI3 kinase inhibitor; or
- an inhibitor of TEAD palmitoylation; or
- a YAP/TAZ::TEAD inhibitor.

In a further aspect the compound of formula **(I), (I*), (Ia), (Ib), (Ic), (Id), (Ie)** or **(If)** - or a pharmaceutically acceptable salt thereof - for (combined) use in a method (e.g. method for the treatment and/or prevention) as hereinbefore described, wherein two other pharmacologically active substances are to be administered before, after or together with the compound of formula **(I), (I*), (Ia), (Ib), (Ic), (Id), (Ie)** or **(If)** - or a pharmaceutically acceptable salt thereof - wherein said two other pharmacologically active substances are
- a MEK inhibitor and a SOS1 inhibitor; or
- trametinib and a SOS1 inhibitor; or
- an *anti*-PD-1 antibody (preferably ezabenlimab) and an *anti*-LAG-3 antibody; or
- an *anti*-PD-1 antibody (preferably ezabenlimab) and a SOS1 inhibitor; or
- a MEK inhibitor and an inhibitor selected from the group consisting of an EGFR inhibitor and/or ErbB2 (HER2) inhibitor and/or inhibitor of any mutants thereof; or
- a SOS1 inhibitor and an inhibitor selected from the group consisting of an EGFR inhibitor and/or ErbB2 (HER2) inhibitor and/or inhibitor of any mutants thereof; or
- a MEK inhibitor and afatinib; or
- a MEK inhibitor and cetuximab; or
- trametinib and afatinib; or
- trametinib and cetuximab; or
- a SOS1 inhibitor and afatinib; or
- a SOS1 inhibitor and cetuximab; or
- a SOS1 inhibitor and an inhibitor of TEAD palmitoylation; or
- a SOS1 inhibitor and a YAP/TAZ::TEAD inhibitor.

In a further aspect the compound of formula **(I), (I*), (Ia), (Ib), (Ic), (Id), (Ie)** or **(If)** - or a pharmaceutically acceptable salt thereof - for (combined) use in a method (*e.g*. method for the treatment and/or prevention) as hereinbefore described, wherein two other pharmacologically active substances are to be administered in combination with the compound of formula **(I), (I*), (Ia), (Ib), (Ic), (Id), (Ie)** or **(If)** - or a pharmaceutically acceptable salt thereof - wherein said two other pharmacologically active substances are
- a MEK inhibitor and a SOS1 inhibitor; or
- trametinib and a SOS1 inhibitor; or
- an *anti*-PD-1 antibody (preferably ezabenlimab) and an *anti*-LAG-3 antibody; or
- an *anti*-PD-1 antibody (preferably ezabenlimab) and a SOS1 inhibitor; or
- a MEK inhibitor and an inhibitor selected from the group consisting of an EGFR inhibitor and/or ErbB2 (HER2) inhibitor and/or inhibitor of any mutants thereof; or
- a SOS1 inhibitor and an inhibitor selected from the group consisting of an EGFR inhibitor and/or ErbB2 (HER2) inhibitor and/or inhibitor of any mutants thereof; or
- a MEK inhibitor and afatinib; or
- a MEK inhibitor and cetuximab; or
- trametinib and afatinib; or
- trametinib and cetuximab; or
- a SOS1 inhibitor and afatinib; or
- a SOS1 inhibitor and cetuximab; or
- a SOS1 inhibitor and an inhibitor of TEAD palmitoylation; or
- a SOS1 inhibitor and a YAP/TAZ::TEAD inhibitor.

Additional the compound of formula **(I)**, **(I*), (Ia), (Ib), (Ic), (Id), (Ie)** or **(If)** - or a pharmaceutically acceptable salt thereof - (including all individual embodiments or generic subsets of compounds of formula **(I), (I*)**, **(Ia)**, **(Ib), (Ic), (Id)**, **(Ie)** or **(If))** for use in combination with other pharmacologically active substance(s) or in the medical uses, uses, methods of treatment and/or prevention, pharmaceutical compositions, kits as herein (above and below) defined include, without being restricted thereto, hormones, hormone analogues and antihormones (*e.g*. tamoxifen, toremifene, raloxifene, fulvestrant, megestrol acetate, flutamide, nilutamide, bicalutamide, aminoglutethimide, cyproterone acetate, finasteride, buserelin acetate, fludrocortisone, fluoxymesterone, medroxyprogesterone, octreotide), aromatase inhibitors (*e.g*. anastrozole, letrozole, liarozole, vorozole, exemestane, atamestane), LHRH agonists and antagonists (*e.g.* goserelin acetate, luprolide), inhibitors of growth factors and/or of their corresponding receptors (growth factors such as for example platelet derived growth factor (PDGF), fibroblast growth factor (FGF), vascular endothelial growth factor (VEGF), epidermal growth factor (EGF), insuline-like growth factors (IGF), human epidermal growth factor (HER, *e.g*. HER2, HER3, HER4) and hepatocyte growth factor (HGF) and/or their corresponding receptors), inhibitors are for example (*anti*-)growth factor antibodies, (*anti-*)growth factor receptor antibodies and tyrosine kinase inhibitors, such as for example cetuximab, gefitinib, afatinib, nintedanib, imatinib, lapatinib, bosutinib, bevacizumab and trastuzumab); antimetabolites (*e.g*. antifolates such as methotrexate, raltitrexed, pyrimidine analogues such as 5-fluorouracil (5-FU), ribonucleoside and deoxyribonucleoside analogues, capecitabine and gemcitabine, purine and adenosine analogues such as mercaptopurine, thioguanine, cladribine and pentostatin, cytarabine (ara C), fludarabine); antitumor antibiotics (*e.g*. anthracyclins such as doxorubicin, doxil (pegylated liposomal doxorubicin hydrochloride, myocet (non-pegylated liposomal doxorubicin), daunorubicin, epirubicin and idarubicin, mitomycin-C, bleomycin, dactinomycin, plicamycin, streptozocin); platinum derivatives (*e.g.* cisplatin, oxaliplatin, carboplatin); alkylation agents (*e.g.* estramustin, meclorethamine, melphalan, chlorambucil, busulphan, dacarbazin, cyclophosphamide, ifosfamide, temozolomide, nitrosoureas such as for example carmustin and lomustin, thiotepa); antimitotic agents (*e.g.* Vinca alkaloids such as for example vinblastine, vindesin, vinorelbin and vincristine; and taxanes such as paclitaxel, docetaxel); angiogenesis inhibitors (*e.g*. tasquinimod), tubuline inhibitors; DNA synthesis inhibitors, PARP inhibitors, topoisomerase inhibitors (*e.g*. epipodophyllotoxins such as for example etoposide and etopophos, teniposide, amsacrin, topotecan, irinotecan, mitoxantrone), serine/threonine kinase inhibitors (*e.g*. PDK 1 inhibitors, Raf inhibitors, A-Raf inhibitors, B-Raf inhibitors, C-Raf inhibitors, mTOR inhibitors, mTORC1/2 inhibitors, PI3K inhibitors, PI3Kα inhibitors, dual mTOR/PI3K inhibitors, STK 33 inhibitors, AKT inhibitors, PLK 1 inhibitors, inhibitors of CDKs, Aurora kinase inhibitors), tyrosine kinase inhibitors (*e.g*. PTK2/FAK inhibitors), protein protein interaction inhibitors (*e.g*. IAP inhibitors/SMAC mimetics, Mcl-1, MDM2/MDMX), MEK inhibitors, ERK inhibitors, FLT3 inhibitors, BRD4 inhibitors, IGF-1R inhibitors, TRAILR2 agonists, Bcl-xL inhibitors, Bcl-2 inhibitors (*e.g*. venetoclax), Bcl-2/Bcl-xL inhibitors, ErbB receptor inhibitors, BCR-ABL inhibitors, ABL inhibitors, Src inhibitors, rapamycin analogs (*e.g*. everolimus, temsirolimus, ridaforolimus, sirolimus), androgen synthesis inhibitors, androgen receptor inhibitors, DNMT inhibitors, HDAC inhibitors, ANG1/2 inhibitors, CYP17 inhibitors, radiopharmaceuticals, proteasome inhibitors (*e.g*. carfilzomib), immunotherapeutic agents such as immune checkpoint inhibitors (*e.g*. CTLA4, PD1, PD-L1, PD-L2, LAG3, and TIM3 binding molecules/immunoglobulins, such as *e.g*. ipilimumab, nivolumab, pembrolizumab), ADCC (antibody-dependent cell-mediated cytotoxicity) enhancers (*e.g*. anti-CD33 antibodies, anti-CD37 antibodies, anti-CD20 antibodies), t-cell engagers (*e.g*. bi-specific T-cell engagers (BiTEs^{®}) like *e.g*. CD3 x BCMA, CD3 x CD33, CD3 x CD19), PSMA x CD3), tumor vaccines, immunomodulator, e.g. STING agonist, and various chemotherapeutic agents such as amifostin, anagrelid, clodronat, filgrastin, interferon, interferon alpha, leucovorin, procarbazine, levamisole, mesna, mitotane, pamidronate and porfimer.

It is to be understood that the combinations, compositions, kits, methods, uses, pharmaceutical compositions or compounds for use according to this disclosure may envisage the simultaneous, concurrent, sequential, successive, alternate or separate administration of the active ingredients or components. It will be appreciated that the compound of formula **(I)**, **(I*), (Ia), (Ib), (Ic), (Id), (Ie)** or **(If)** - or a pharmaceutically acceptable salt thereof - and the one or more other pharmacologically active substance(s) can be administered formulated either dependently or independently, such as e.g. the compound of formula **(I), (I*), (Ia), (Ib), (Ic), (Id), (Ie)** or **(If)** - or a pharmaceutically acceptable salt thereof - and the one or more other pharmacologically active substance(s) may be administered either as part of the same pharmaceutical composition/dosage form or, preferably, in separate pharmaceutical compositions/dosage forms.

In this context, "combination" or "combined" within the meaning of this disclosure includes, without being limited, a product that results from the mixing or combining of more than one active ingredient and includes both fixed and non-fixed (e.g. free) combinations (including kits) and uses, such as e.g. the simultaneous, concurrent, sequential, successive, alternate or separate use of the components or ingredients. The term "fixed combination" means that the active ingredients are administered to a patient simultaneously in the form of a single entity or dosage. The term "non-fixed combination" means that the active ingredients are administered to a patient as separate entities either simultaneously, concurrently or sequentially with no specific time limits, wherein such administration provides therapeutically effective levels of the compounds in the body of the patient.

The administration of the compound of formula **(I), (I*), (Ia), (Ib), (Ic), (Id), (Ie)** or **(If)** - or a pharmaceutically acceptable salt thereof - and the one or more other pharmacologically active substance(s) may take place by co-administering the active components or ingredients, such as *e.g*. by administering them simultaneously or concurrently in one single or in two or more separate formulations or dosage forms. Alternatively, the administration of the compound of formula **(I)**, **(I*)**, **(Ia)**, **(Ib), (Ic), (Id), (Ie)** or **(If)** - or a pharmaceutically acceptable salt thereof - and the one or more other pharmacologically active substance(s) may take place by administering the active components or ingredients sequentially or in alternation, such as *e.g*. in two or more separate formulations or dosage forms.

For example, simultaneous administration includes administration at substantially the same time. This form of administration may also be referred to as "concomitant" administration. Concurrent administration includes administering the active agents within the same general time period, for example on the same day(s) but not necessarily at the same time. Alternate administration includes administration of one agent during a time period, for example over the course of a few days or a week, followed by administration of the other agent(s) during a subsequent period of time, for example over the course of a few days or a week, and then repeating the pattern for one or more cycles. Sequential or successive administration includes administration of one agent during a first time period (for example over the course of a few days or a week) using one or more doses, followed by administration of the other agent(s) during a second and/or additional time period (for example over the course of a few days or a week) using one or more doses. An overlapping schedule may also be employed, which includes administration of the active agents on different days over the treatment period, not necessarily according to a regular sequence. Variations on these general guidelines may also be employed, *e.g*. according to the agents used and the condition of the subject.

### Definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to:

The use of the prefix **C_{x-y}**, wherein **x** and **y** each represent a positive integer **(x < y),** indicates that the chain or ring structure or combination of chain and ring structure as a whole, specified and mentioned in direct association, may consist of a maximum of **y** and a minimum of **x** carbon atoms.

The indication of the number of members in groups that contain one or more heteroatom(s) (*e.g*. heteroaryl, heteroarylalkyl, heterocyclyl, heterocycylalkyl) relates to the total number of atoms of all the ring members or the total of all the ring and carbon chain members.

The indication of the number of carbon atoms in groups that consist of a combination of carbon chain and carbon ring structure (*e.g*. cycloalkylalkyl, arylalkyl) relates to the total number of carbon atoms of all the carbon ring and carbon chain members. Obviously, a ring structure has at least three members.

In general, for groups comprising two or more subgroups (*e.g*. heteroarylalkyl, heterocycylalkyl, cycloalkylalkyl, arylalkyl) the last named subgroup is the radical attachment point, for example, the substituent aryl-C₁₋₆alkyl means an aryl group which is bound to a C₁₋₆alkyl group, the latter of which is bound to the core or to the group to which the substituent is attached.

In groups like HO, H₂N, (O)S, (O)₂S, NC (cyano), HOOC, F₃C or the like, the skilled artisan can see the radical attachment point(s) to the molecule from the free valences of the group itself.

The expression "compound of the disclosure" and grammatical variants thereof comprises compounds of formula **(I)**, **(I*)**, **(Ia)**, **(Ib), (Ic), (Id), (Ie)** and **(If),** including all salts, aspects and preferred embodiments thereof as herein defined. Any reference to a compound of the invention or to a compound of formula **(I)**, **(I*)**, **(Ia)**, **(Ib), (Ic), (Id), (Ie)** and **(If)** is intended to include a reference to the respective (sub)aspects and embodiments.

**Alkyl** denotes monovalent, saturated hydrocarbon chains, which may be present in both straight-chain (unbranched) and branched form. If an **alkyl** is substituted, the substitution may take place independently of one another, by mono- or polysubstitution in each case, on all the hydrogen-carrying carbon atoms.

The term "C₁₋₅alkyl" includes for example H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-. Further examples of **alkyl** are methyl (Me; -CH₃), ethyl (Et; -CH₂CH₃), 1-propyl (*n*-propyl; *n-*Pr; -CH₂CH₂CH₃), 2-propyl (*i*-Pr; *iso*-propyl; -CH(CH₃)₂), 1-butyl (*n*-butyl; *n*-Bu; -CH₂CH₂CH₂CH₃), 2-methyl-1-propyl (*iso*-butyl; *i*-Bu; -CH₂CH(CH₃)₂), 2-butyl (*sec*-butyl; *sec*-Bu; -CH(CH₃)CH₂CH₃), 2-methyl-2-propyl (*tert-*butyl; *t*-Bu; -C(CH₃)₃), 1-pentyl (*n*-pentyl; -CH₂CH₂CH₂CH₂CH₃), 2-pentyl (-CH(CH₃)CH₂CH₂CH₃), 3-pentyl (-CH(CH₂CH₃)₂), 3-methyl-1-butyl (*iso*-pentyl; -CH₂CH₂CH(CH₃)₂), 2-methyl-2-butyl (-C(CH₃)₂CH₂CH₃), 3-methyl-2-butyl (-CH(CH₃)CH(CH₃)₂), 2,2-dimethyl-1-propyl (*neo*-pentyl; -CH₂C(CH₃)₃), 2-methyl-1-butyl (-CH₂CH(CH₃)CH₂CH₃), 1-hexyl (*n*-hexyl; -CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), 2,3-dimethyl-1-butyl (-CH₂CH(CH₃)CH(CH₃)CH₃), 2,2-dimethyl-1-butyl (-CH₂C(CH₃)₂CH₂CH₃), 3,3-dimethyl-1-butyl (-CH₂CH₂C(CH₃)₃), 2-methyl-1-pentyl (-CH₂CH(CH₃)CH₂CH₂CH₃), 3-methyl-1-pentyl (-CH₂CH₂CH(CH₃)CH₂CH₃), 1-heptyl (*n*-heptyl), 2-methyl-1-hexyl, 3-methyl-1-hexyl, 2,2-dimethyl-1-pentyl, 2,3-dimethyl-1-pentyl, 2,4-dimethyl-1-pentyl, 3,3-dimethyl-1-pentyl, 2,2,3-trimethyl-1-butyl, 3-ethyl-1-pentyl, 1-octyl (*n*-octyl), 1-nonyl (*n*-nonyl); 1-decyl (*n*-decyl).

By the terms propyl, butyl, pentyl, hexyl, heptyl, octyl, nonyl, decyl *etc.* without any further definition are meant saturated hydrocarbon groups with the corresponding number of carbon atoms, wherein all isomeric forms are included.

The above definition for **alkyl** also applies if **alkyl** is a part of another (combined) group such as for example C_{x-y}**alkyl**amino or C_{x-y}**alkyl**oxy.

The term **alkylene** can also be derived from **alkyl. Alkylene** is bivalent, unlike **alkyl,** and requires two binding partners. Formally, the second valency is produced by removing a hydrogen atom in an **alkyl.** Corresponding groups are for example -CH₃ and -CH₂-, -CH₂CH₃ and -CH₂CH₂- or >CHCH₃.

The term "C₁₋₄alkylene" includes for example -(CH₂)-, -(CH₂-CH₂)-, -(CH(CH₃))-, -(CH₂-CH₂-CH₂)-, -(C(CH₃)₂)-, -(CH(CH₂CH₃))-, -(CH(CH₃)-CH₂)-, -(CH₂-CH(CH₃))-, -(CH₂-CH₂-CH₂-CH₂)-, -(CH₂-CH₂-CH(CH₃))-, -(CH(CH₃)-CH₂-CH₂)-, -(CH₂-CH(CH₃)-CH₂)-, -(CH₂-C(CH₃)₂)-, -(C(CH₃)₂-CH₂)-, -(CH(CH₃)-CH(CH₃))-, -(CH₂-CH(CH₂CH₃))-, -(CH(CH₂CH₃)-CH₂)-, -(CH(CH₂CH₂CH₃))-, -(CH(CH(CH₃))₂)- and -C(CH₃)(CH₂CH₃)-.

Other examples of **alkylene** are methylene, ethylene, propylene, 1-methylethylene, butylene, 1-methylpropylene, 1,1-dimethylethylene, 1,2-dimethylethylene, pentylene, 1,1-dimethylpropylene, 2,2-dimethylpropylene, 1,2-dimethylpropylene, 1,3-dimethylpropylene, hexylene.

By the generic terms propylene, butylene, pentylene, hexylene *etc.* without any further definition are meant all the conceivable isomeric forms with the corresponding number of carbon atoms, *i.e.* propylene includes 1-methylethylene and butylene includes 1-methylpropylene, 2-methylpropylene, 1,1-dimethylethylene and 1,2-dimethylethylene.

The above definition for **alkylene** also applies if **alkylene** is part of another (combined) group such as for example in HO-C_{x-y}**alkylene**amino or H₂N-C_{x-y}**alkylene**oxy.

Unlike **alkyl, alkenyl** consists of at least two carbon atoms, wherein at least two adjacent carbon atoms are joined together by a C-C double bond and a carbon atom can only be part of one C-C double bond. If in an **alkyl** as hereinbefore defined having at least two carbon atoms, two hydrogen atoms on adjacent carbon atoms are formally removed and the free valencies are saturated to form a second bond, the corresponding **alkenyl** is formed.

Examples of **alkenyl** are vinyl (ethenyl), prop-1-enyl, allyl (prop-2-enyl), isopropenyl, but-1-enyl, but-2-enyl, but-3-enyl, 2-methyl-prop-2-enyl, 2-methyl-prop-1-enyl, 1-methyl-prop-2-enyl, 1-methyl-prop-1-enyl, 1-methylidenepropyl, pent-1-enyl, pent-2-enyl, pent-3-enyl, pent-4-enyl, 3-methyl-but-3-enyl, 3-methyl-but-2-enyl, 3-methyl-but-1-enyl, hex-1-enyl, hex-2-enyl, hex-3-enyl, hex-4-enyl, hex-5-enyl, 2,3-dimethyl-but-3-enyl, 2,3-dimethyl-but-2-enyl, 2-methylidene-3-methylbutyl, 2,3-dimethyl-but-1-enyl, hexa-1,3-dienyl, hexa-1,4-dienyl, penta-1,4-dienyl, penta-1,3-dienyl, buta-1,3-dienyl, 2,3-dimethylbuta-1,3-diene.

By the generic terms propenyl, butenyl, pentenyl, hexenyl, butadienyl, pentadienyl, hexadienyl, heptadienyl, octadienyl, nonadienyl, decadienyl etc. without any further definition are meant all the conceivable isomeric forms with the corresponding number of carbon atoms, *i.e.* propenyl includes prop-1-enyl and prop-2-enyl, butenyl includes but-1-enyl, but-2-enyl, but-3-enyl, 1-methyl-prop-1-enyl, 1-methyl-prop-2-enyl.

**Alkenyl** may optionally be present in the *cis* or *trans* or *E* or *Z* orientation with regard to the double bond(s).

The above definition for **alkenyl** also applies when **alkenyl** is part of another (combined) group such as for example in C_{x-y}**alkenyl**amino or C_{x-y}**alkenyl**oxy.

Unlike **alkylene, alkenylene** consists of at least two carbon atoms, wherein at least two adjacent carbon atoms are joined together by a C-C double bond and a carbon atom can only be part of one C-C double bond. If in an **alkylene** as hereinbefore defined having at least two carbon atoms, two hydrogen atoms at adjacent carbon atoms are formally removed and the free valencies are saturated to form a second bond, the corresponding **alkenylene** is formed. Examples of **alkenylene** are ethenylene, propenylene, 1-methylethenylene, butenylene, 1-methylpropenylene, 1,1-dimethylethenylene, 1,2-dimethylethenylene, pentenylene, 1,1-dimethylpropenylene, 2,2-dimethylpropenylene, 1,2-dimethylpropenylene, 1,3-dimethylpropenylene, hexenylene.

By the generic terms propenylene, butenylene, pentenylene, hexenylene *etc.* without any further definition are meant all the conceivable isomeric forms with the corresponding number of carbon atoms, *i.e.* propenylene includes 1-methylethenylene and butenylene includes 1-methylpropenylene, 2-methylpropenylene, 1,1-dimethylethenylene and 1,2-dimethylethenylene.

**Alkenylene** may optionally be present in the *cis* or *trans* or *E* or *Z* orientation with regard to the double bond(s).

The above definition for **alkenylene** also applies when **alkenylene** is a part of another (combined) group as for example in HO-C_{x-y}**alkenylene**amino or H₂N-C_{x-y}**alkenylene**oxy.

Unlike **alkyl, alkynyl** consists of at least two carbon atoms, wherein at least two adjacent carbon atoms are joined together by a C-C triple bond. If in an **alkyl** as hereinbefore defined having at least two carbon atoms, two hydrogen atoms in each case at adjacent carbon atoms are formally removed and the free valencies are saturated to form two further bonds, the corresponding **alkynyl** is formed.

Examples of **alkynyl** are ethynyl, prop-1-ynyl, prop-2-ynyl, but-1-ynyl, but-2-ynyl, but-3-ynyl, 1-methyl-prop-2-ynyl, pent-1-ynyl, pent-2-ynyl, pent-3-ynyl, pent-4-ynyl, 3-methyl-but-1-ynyl, hex-1-ynyl, hex-2-ynyl, hex-3-ynyl, hex-4-ynyl, hex-5-ynyl.

By the generic terms propynyl, butynyl, pentynyl, hexynyl, heptynyl, octynyl, nonynyl, decynyl etc. without any further definition are meant all the conceivable isomeric forms with the corresponding number of carbon atoms, *i.e*. propynyl includes prop-1-ynyl and prop-2-ynyl, butynyl includes but-1-ynyl, but-2-ynyl, but-3-ynyl, 1-methyl-prop-1-ynyl,1-methyl-prop-2-ynyl.

If a hydrocarbon chain carries both at least one double bond and also at least one triple bond, by definition it belongs to the **alkynyl** subgroup.

The above definition for **alkynyl** also applies if **alkynyl** is part of another (combined) group, as for example in C_{x-y}**alkynyl**amino or C_{x-y}**alkynyl**oxy.

Unlike **alkylene, alkynylene** consists of at least two carbon atoms, wherein at least two adjacent carbon atoms are joined together by a C-C triple bond. If in an **alkylene** as hereinbefore defined having at least two carbon atoms, two hydrogen atoms in each case at adjacent carbon atoms are formally removed and the free valencies are saturated to form two further bonds, the corresponding **alkynylene** is formed.

Examples of **alkynylene** are ethynylene, propynylene, 1-methylethynylene, butynylene, 1-methylpropynylene, 1,1-dimethylethynylene, 1,2-dimethylethynylene, pentynylene, 1,1-dimethylpropynylene, 2,2-dimethylpropynylene, 1,2-dimethylpropynylene, 1,3-dimethylpropynylene, hexynylene.

By the generic terms propynylene, butynylene, pentynylene, hexynylene etc. without any further definition are meant all the conceivable isomeric forms with the corresponding number of carbon atoms, *i.e.* propynylene includes 1-methylethynylene and butynylene includes 1-methylpropynylene, 2-methylpropynylene, 1,1-dimethylethynylene and 1,2-dimethylethynylene.

The above definition for **alkynylene** also applies if **alkynylene** is part of another (combined) group, as for example in HO-C_{x-y}**alkynylene**amino or H₂N-C_{x-y}**alkynylene**oxy.

By **heteroatoms** are meant oxygen, nitrogen and sulphur atoms.

**Haloalkyl (haloalkenyl, haloalkynyl)** is derived from the previously defined **alkyl (alkenyl, alkynyl)** by replacing one or more hydrogen atoms of the hydrocarbon chain independently of one another by halogen atoms, which may be identical or different. If a **haloalkyl (haloalkenyl, haloalkynyl)** is to be further substituted, the substitutions may take place independently of one another, in the form of mono- or polysubstitutions in each case, on all the hydrogen-carrying carbon atoms.

Examples of **haloalkyl (haloalkenyl, haloalkynyl)** are -CF₃, -CHF₂, -CH₂F, -CF₂CF₃, -CHFCF₃, -CH₂CF₃, -CF₂CH₃, -CHFCH₃, -CF₂CF₂CF₃, -CF₂CH₂CH₃, -CF=CF₂, -CCl=CH₂, -CBr=CH₂, -C≡C-CF₃, -CHFCH₂CH₃, -CHFCH₂CF₃.

From the previously defined **haloalkyl (haloalkenyl, haloalkynyl)** are also derived the terms **haloalkylene (haloalkenylene, haloalkynylene). Haloalkylene (haloalkenylene, haloalkynylene),** unlike **haloalkyl (haloalkenyl, haloalkynyl),** is bivalent and requires two binding partners. Formally, the second valency is formed by removing a hydrogen atom from a **haloalkyl (haloalkenyl, haloalkynyl).**

Corresponding groups are for example -CH₂F and -CHF-, -CHFCH₂F and -CHFCHF- or >CFCH₂F.

The above definitions also apply if the corresponding halogen-containing groups are part of another (combined) group.

**Halogen** denotes fluorine, chlorine, bromine and/or iodine atoms.

**Cycloalkyl** is made up of the subgroups **monocyclic cycloalkyl, bicyclic cycloalkyl** and **spiro-cycloalkyl.** The ring systems are saturated and formed by linked carbon atoms. In bicyclic cycloalkyl two rings are joined together so that they have at least two carbon atoms in common. In spiro-cycloalkyl one carbon atom (spiroatom) belongs to two rings together.

If a **cycloalkyl** is to be substituted, the substitutions may take place independently of one another, in the form of mono- or polysubstitutions in each case, on all the hydrogen-carrying carbon atoms. **Cycloalkyl** itself may be linked as a substituent to the molecule *via* every suitable position of the ring system.

Examples of **cycloalkyl** are cyclopropyl, cyclobutyl, cyclopentyl, cyclohexyl, cycloheptyl, bicyclo[2.2.0]hexyl, bicyclo[3.2.0]heptyl, bicyclo[3.2.1]octyl, bicyclo[2.2.2]octyl, bicyclo[4.3.0]nonyl (octahydroindenyl), bicyclo[4.4.0]decyl (decahydronaphthyl), bicyclo[2.2.1]heptyl (norbornyl), bicyclo[4.1.0]heptyl (norcaranyl), bicyclo[3.1.1]heptyl (pinanyl), spiro[2.5]octyl, spiro[3.3]heptyl.

The above definition for **cycloalkyl** also applies if **cycloalkyl** is part of another (combined) group as for example in C_{x-y}**cycloalkyl**amino, C_{x-y}**cycloalkyl**oxy or C_{x-y}**cycloalkyl**alkyl.

If the free valency of a **cycloalkyl** is saturated, then an **alicycle** is obtained.

The term **cycloalkylene** can thus be derived from the previously defined **cycloalkyl. Cycloalkylene,** unlike **cycloalkyl,** is bivalent and requires two binding partners. Formally, the second valency is obtained by removing a hydrogen atom from a **cycloalkyl.** Corresponding groups are for example:
cyclohexyl and 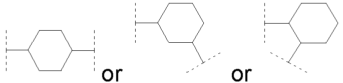 (cyclohexylene).

The above definition for **cycloalkylene** also applies if **cycloalkylene** is part of another (combined) group as for example in HO-C_{x-y}**cycloalkylene**amino or H₂N-C_{x-y}**cycloalkylene**oxy.

**Cycloalkenyl** is made up of the subgroups **monocyclic cycloalkenyl, bicyclic cycloalkeny** and **spiro-cycloalkenyl.** However, the systems are unsaturated, *i.e.* there is at least one C-C double bond but no aromatic system. If in a **cycloalkyl** as hereinbefore defined two hydrogen atoms at adjacent cyclic carbon atoms are formally removed and the free valencies are saturated to form a second bond, the corresponding **cycloalkenyl** is obtained.

If a **cycloalkenyl** is to be substituted, the substitutions may take place independently of one another, in the form of mono- or polysubstitutions in each case, on all the hydrogen-carrying carbon atoms. **Cycloalkenyl** itself may be linked as a substituent to the molecule *via* every suitable position of the ring system.

Examples of **cycloalkenyl** are cycloprop-1-enyl, cycloprop-2-enyl, cyclobut-1-enyl, cyclobut-2-enyl, cyclopent-1-enyl, cyclopent-2-enyl, cyclopent-3-enyl, cyclohex-1-enyl, cyclohex-2-enyl, cyclohex-3-enyl, cyclohept-1-enyl, cyclohept-2-enyl, cyclohept-3-enyl, cyclohept-4-enyl, cyclobuta-1,3-dienyl, cyclopenta-1,4-dienyl, cyclopenta-1,3-dienyl, cyclopenta-2,4-dienyl, cyclohexa-1,3-dienyl, cyclohexa-1,5-dienyl, cyclohexa-2,4-dienyl, cyclohexa-1,4-dienyl, cyclohexa-2,5-dienyl, bicyclo[2.2.1]hepta-2,5-dienyl (norborna-2,5-dienyl), bicyclo[2.2.1]hept-2-enyl (norbornenyl), spiro[4,5]dec-2-enyl.

The above definition for **cycloalkenyl** also applies when **cycloalkenyl** is part of another (combined) group as for example in C_{x-y}**cycloalkenyl**amino, C_{x-y}**cycloalkenyl**oxy or C_{x-y}**cycloalkenyl**alkyl.

If the free valency of a **cycloalkenyl** is saturated, then an **unsaturated alicycle** is obtained.

The term **cycloalkenylene** can thus be derived from the previously defined **cycloalkenyl. Cycloalkenylene,** unlike **cycloalkenyl,** is bivalent and requires two binding partners. Formally, the second valency is obtained by removing a hydrogen atom from a **cycloalkenyl.** Corresponding groups are for example:
cyclopentenyl and 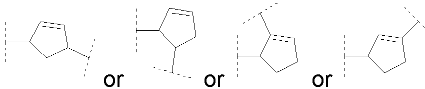 (cyclopentenylene) *etc.*

The above definition for **cycloalkenylene** also applies if **cycloalkenylene** is part of another (combined) group as for example in HO-C_{x-y}**cycloalkenylene**amino or H₂N-C_{x-y}**cycloalkenylene**oxy.

**Aryl** denotes mono-, bi- or tricyclic carbocycles with at least one aromatic carbocycle. Preferably, it denotes a monocyclic group with six carbon atoms (phenyl) or a bicyclic group with nine or ten carbon atoms (two six-membered rings or one six-membered ring with a five-membered ring), wherein the second ring may also be aromatic or, however, may also be partially saturated.

If an **aryl** is to be substituted, the substitutions may take place independently of one another, in the form of mono- or polysubstitutions in each case, on all the hydrogen-carrying carbon atoms. **Aryl** itself may be linked as a substituent to the molecule *via* every suitable position of the ring system.

Examples of **aryl** are phenyl, naphthyl, indanyl (2,3-dihydroindenyl), indenyl, anthracenyl, phenanthrenyl, tetrahydronaphthyl (1,2,3,4-tetrahydronaphthyl, tetralinyl), dihydronaphthyl (1,2- dihydronaphthyl), fluorenyl. Most preferred is phenyl.

The above definition of **aryl** also applies if **aryl** is part of another (combined) group as for example in **aryl**amino, **aryl**oxy or **aryl**alkyl.

If the free valency of an **aryl** is saturated, then an **aromatic group** is obtained.

The term **arylene** can also be derived from the previously defined **aryl. Arylene,** unlike **aryl,** is bivalent and requires two binding partners. Formally, the second valency is formed by removing a hydrogen atom from an **aryl.** Corresponding groups are for example:
phenyl and 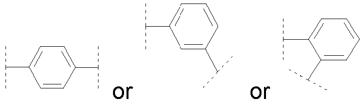 (*o*, *m*, *p*-phenylene),
naphthyl and 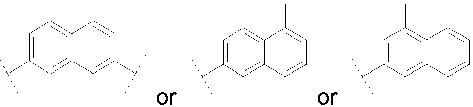 *etc.*

The above definition for **arylene** also applies if **arylene** is part of another (combined) group as for example in HO-**arylene**amino or H₂N-**arylene**oxy.

**Heterocyclyl** denotes ring systems, which are derived from the previously defined **cycloalkyl, cycloalkenyl** and **aryl** by replacing one or more of the groups -CH₂- independently of one another in the hydrocarbon rings by the groups -O-, -S- or -NH- or by replacing one or more of the groups =CH- by the group =N-, wherein a total of not more than five heteroatoms may be present, at least one carbon atom must be present between two oxygen atoms and between two sulphur atoms or between an oxygen and a sulphur atom and the ring as a whole must have chemical stability. Heteroatoms may optionally be present in all the possible oxidation stages (sulphur → sulfoxide -SO-, sulphone -SO₂-; nitrogen → N-oxide). In a **heterocyclyl** there is no heteroaromatic ring, *i.e.* no heteroatom is part of an aromatic system.

A direct result of the derivation from **cycloalkyl, cycloalkenyl** and **aryl** is that **heterocyclyl** is made up of the subgroups **monocyclic heterocyclyl, bicyclic heterocyclyl, tricyclic heterocyclyl** and **spiro-heterocyclyl,** which may be present in saturated or unsaturated form.

By unsaturated is meant that there is at least one double bond in the ring system in question, but no heteroaromatic system is formed. In bicyclic heterocyclyl two rings are linked together so that they have at least two (hetero)atoms in common. In spiro-heterocyclyl one carbon atom (spiroatom) belongs to two rings together.

If a **heterocyclyl** is substituted, the substitutions may take place independently of one another, in the form of mono- or polysubstitutions in each case, on all the hydrogen-carrying carbon and/or nitrogen atoms. **Heterocyclyl** itself may be linked as a substituent to the molecule *via* every suitable position of the ring system. Substituents on **heterocyclyl** do not count for the number of members of a **heterocyclyl.**

Examples of **heterocyclyl** are tetrahydrofuryl, pyrrolidinyl, pyrrolinyl, imidazolidinyl, thiazolidinyl, imidazolinyl, pyrazolidinyl, pyrazolinyl, piperidinyl, piperazinyl, oxiranyl, aziridinyl, azetidinyl, 1,4-dioxanyl, azepanyl, diazepanyl, morpholinyl, thiomorpholinyl, homomorpholinyl, homopiperidinyl, homopiperazinyl, homothiomorpholinyl, thiomorpholinyl-S-oxide, thiomorpholinyl-S, S-dioxide, 1,3-dioxolanyl, tetrahydropyranyl, tetrahydrothiopyranyl, [1,4]-oxazepanyl, tetrahydrothienyl, homothiomorpholinyl-*S*, *S*-dioxide, oxazolidinonyl, dihydropyrazolyl, dihydropyrrolyl, dihydropyrazinyl, dihydropyridyl, dihydropyrimidinyl, dihydrofuryl, dihydropyranyl, tetrahydrothienyl-*S*-oxide, tetrahydrothienyl-*S*,*S-*dioxide, homothiomorpholinyl-*S*-oxide, 2,3-dihydroazet, 2*H*-pyrrolyl, 4*H*-pyranyl, 1,4-dihydropyridinyl, 8-aza-bicyclo[3.2.1]octyl, 8-aza-bicyclo[5.1.0]octyl, 2-oxa-5-azabicyclo[2.2.1]heptyl, 8-oxa-3-aza-bicyclo[3.2.1]octyl, 3,8-diaza-bicyclo[3.2.1]octyl, 2,5-diaza-bicyclo[2.2.1]heptyl, 1-aza-bicyclo[2.2.2]octyl, 3,8-diaza-bicyclo[3.2.1]octyl, 3,9-diazabicyclo[4.2.1]nonyl, 2,6-diaza-bicyclo[3.2.2]nonyl, 1,4-dioxa-spiro[4.5]decyl, 1-oxa-3,8-diazaspiro[4.5]decyl, 2,6-diaza-spiro[3.3]heptyl, 2,7-diaza-spiro[4.4]nonyl, 2,6-diazaspiro[3.4]octyl, 3,9-diaza-spiro[5.5]undecyl, 2.8-diaza-spiro[4,5]decyl.

Further examples are the structures illustrated below, which may be attached *via* each hydrogen-carrying atom (exchanged for hydrogen):

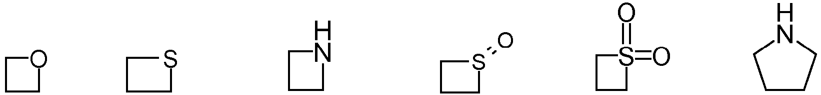

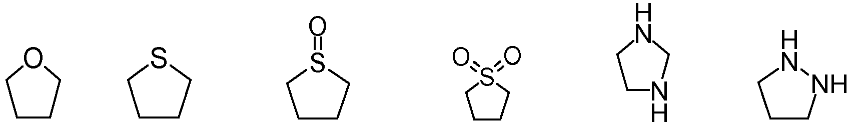

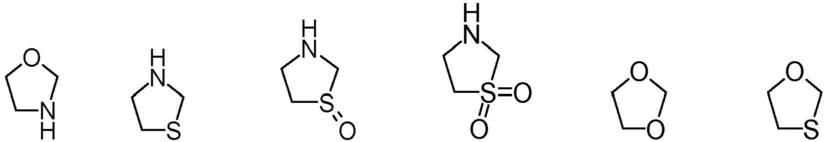

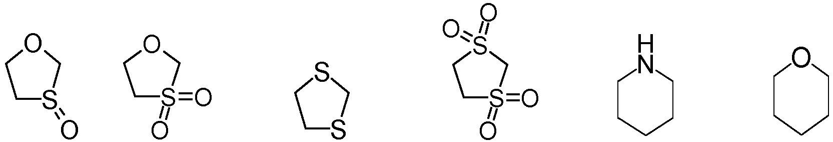

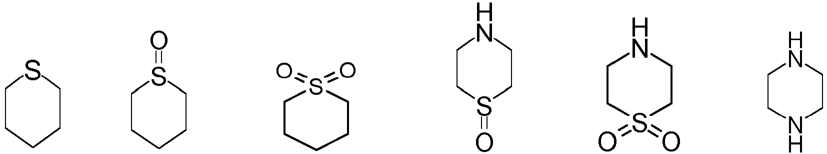

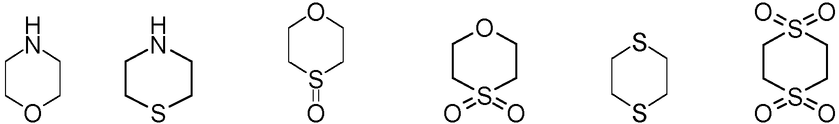

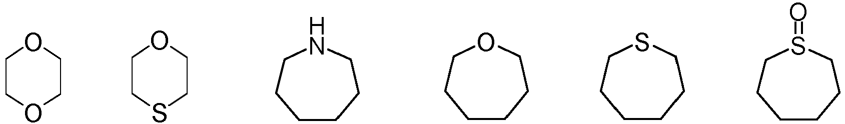

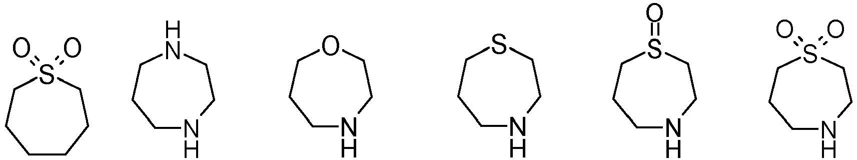

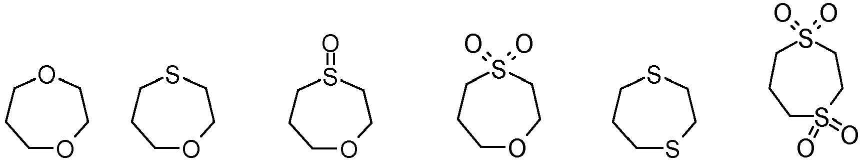

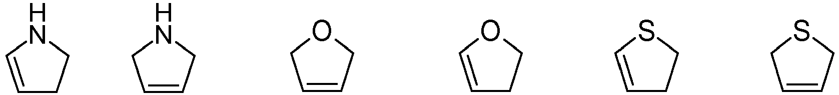

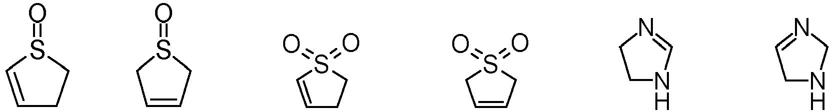

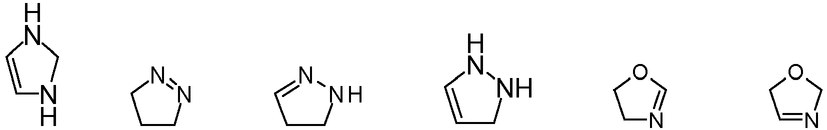

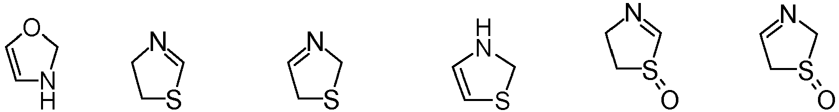

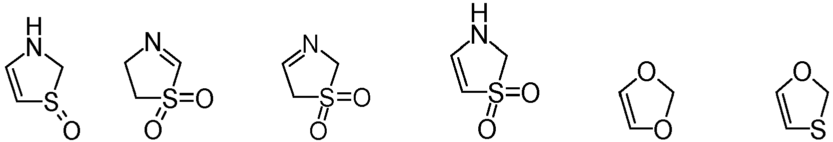

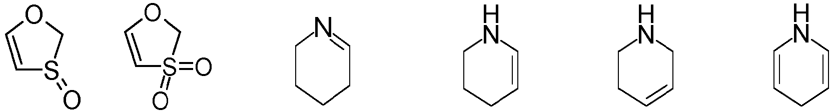

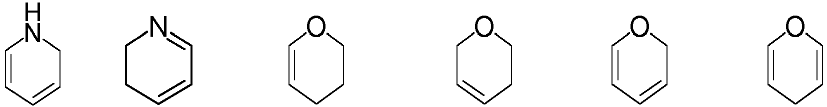

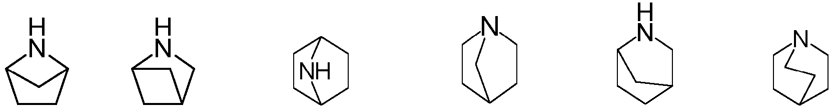

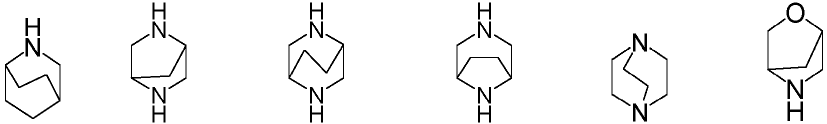

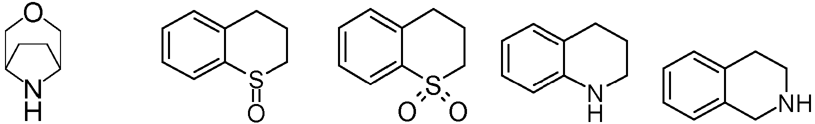

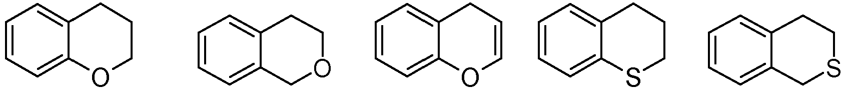

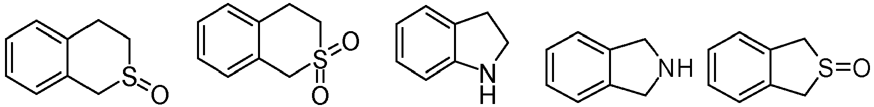

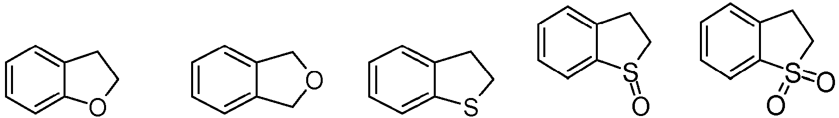

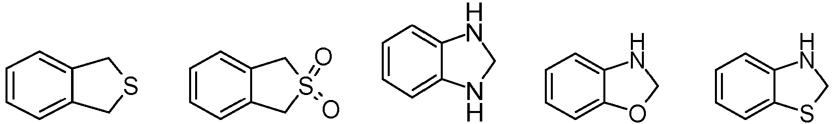

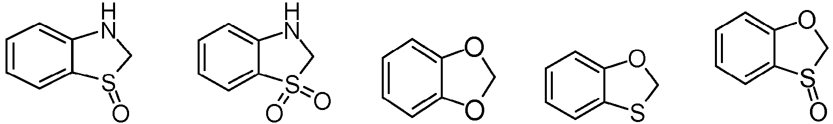

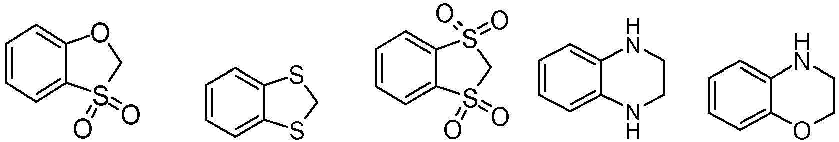

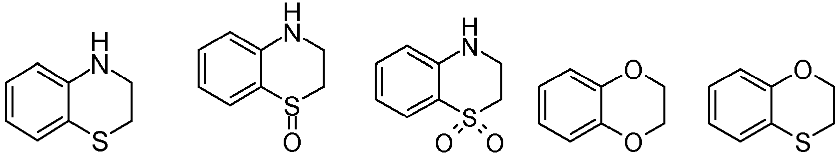

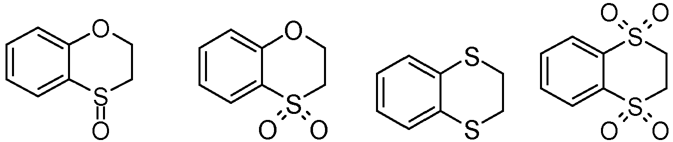

Preferred monocyclic heterocyclyl is 4 to 7 membered and has one or two heteroatoms independently selected from oxygen, nitrogen and sulfur.

Preferred monocyclic heterocyclyls are: piperazinyl, piperidinyl, morpholinyl, pyrrolidinyl, and azetidinyl.

Preferred bicyclic heterocyclyl is 6 to 10 membered and has one or two heteroatoms independently selected from oxygen, nitrogen and sulfur.

Preferred tricyclic heterocyclyl is 9 membered and has one or two heteroatoms independently selected from oxygen, nitrogen and sulfur.

Preferred spiro-heterocyclyl is 7 to 11 membered and has one or two heteroatoms independently selected from oxygen, nitrogen and sulfur.

The above definition of **heterocyclyl** also applies if **heterocyclyl** is part of another (combined) group as for example in **heterocyclylamino, heterocyclyloxy or heterocyclylalkyl.**

If the free valency of a **heterocyclyl** is saturated, then a **heterocycle** is obtained.

The term **heterocyclylene** is also derived from the previously defined **heterocyclyl. Heterocyclylene,** unlike **heterocyclyl,** is bivalent and requires two binding partners. Formally, the second valency is obtained by removing a hydrogen atom from a **heterocyclyl.** Corresponding groups are for example:
piperidinyl and 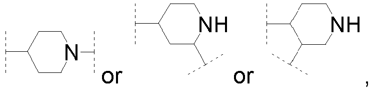
2,3-dihydro-1*H*-pyrrolyl and 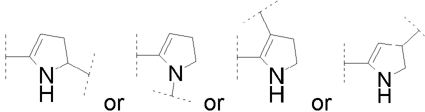 *etc.*

The above definition of **heterocyclylene** also applies if **heterocyclylene** is part of another (combined) group as for example in HO-**heterocyclylene**amino or H₂N-**heterocyclylene**oxy.

**Heteroaryl** denotes monocyclic heteroaromatic rings or polycyclic rings with at least one heteroaromatic ring, which compared with the corresponding **aryl** or **cycloalkyl (cycloalkenyl)** contain, instead of one or more carbon atoms, one or more identical or different heteroatoms, selected independently of one another from among nitrogen, sulphur and oxygen, wherein the resulting group must be chemically stable. The prerequisite for the presence of **heteroaryl** is a heteroatom and a heteroaromatic system.

If a **heteroaryl** is to be substituted, the substitutions may take place independently of one another, in the form of mono- or polysubstitutions in each case, on all the hydrogen-carrying carbon and/or nitrogen atoms. **Heteroaryl** itself may be linked as a substituent to the molecule *via* every suitable position of the ring system, both carbon and nitrogen. Substituents on **heteroaryl** do not count for the number of members of a **heteroaryl.**

Examples of **heteroaryl** are furyl, thienyl, pyrrolyl, oxazolyl, thiazolyl, isoxazolyl, isothiazolyl, pyrazolyl, imidazolyl, triazolyl, tetrazolyl, oxadiazolyl, thiadiazolyl, pyridyl, pyrimidyl, pyridazinyl, pyrazinyl, triazinyl, pyridyl-*N*-oxide, pyrrolyl-*N*-oxide, pyrimidinyl-*N*-oxide, pyridazinyl-*N*-oxide, pyrazinyl-*N*-oxide, imidazolyl-*N*-oxide, isoxazolyl-*N*-oxide, oxazolyl-*N-*oxide, thiazolyl-*N*-oxide, oxadiazolyl-*N*-oxide, thiadiazolyl-*N*-oxide, triazolyl-*N*-oxide, tetrazolyl-*N*-oxide, indolyl, isoindolyl, benzofuryl, benzothienyl, benzoxazolyl, benzothiazolyl, benzisoxazolyl, benzisothiazolyl, benzimidazolyl, indazolyl, isoquinolinyl, quinolinyl, quinoxalinyl, cinnolinyl, phthalazinyl, quinazolinyl, benzotriazinyl, indolizinyl, oxazolopyridyl, imidazopyridyl, naphthyridinyl, benzoxazolyl, pyridopyridyl, pyrimidopyridyl, purinyl, pteridinyl, benzothiazolyl, imidazopyridyl, imidazothiazolyl, quinolinyl-*N*-oxide, indolyl-*N-*oxide, isoquinolyl-*N*-oxide, quinazolinyl-*N*-oxide, quinoxalinyl-*N*-oxide, phthalazinyl-*N*-oxide, indolizinyl-*N*-oxide, indazolyl-*N*-oxide, benzothiazolyl-*N*-oxide, benzimidazolyl-*N*-oxide.

Further examples are the structures illustrated below, which may be attached *via* each hydrogen-carrying atom (exchanged for hydrogen):

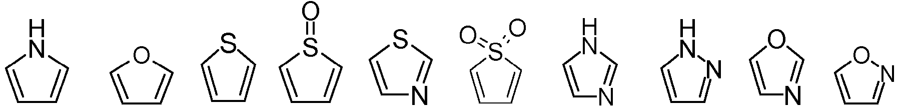

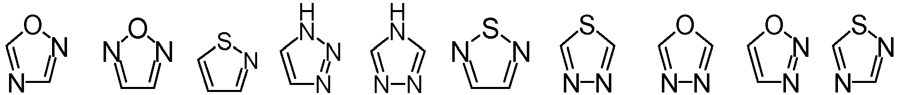

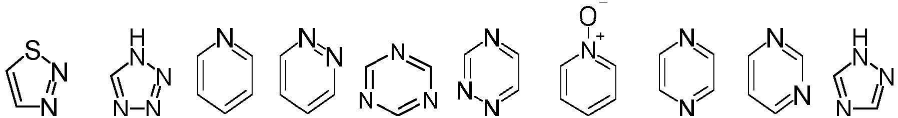

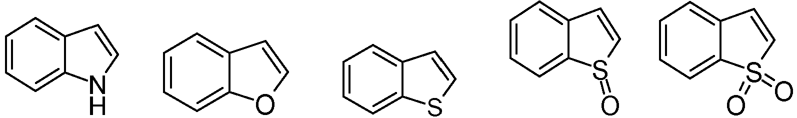

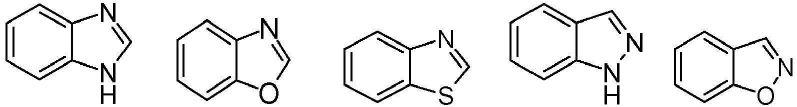

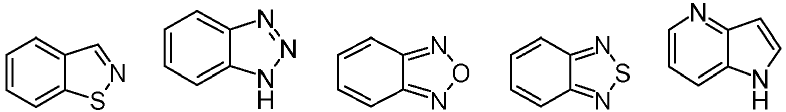

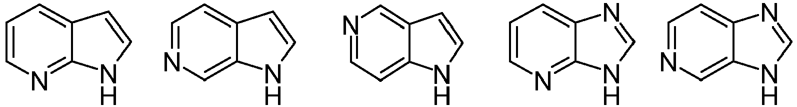

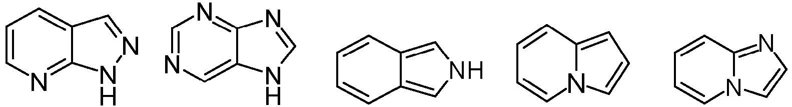

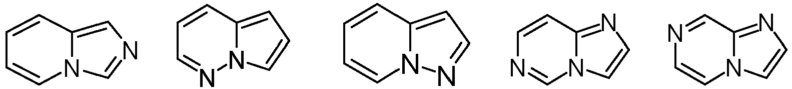

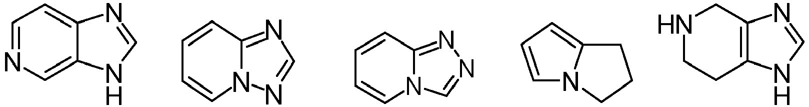

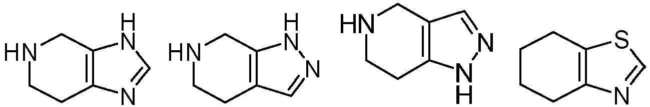

Preferably, heteroaryls are 5-6 membered monocyclic or 9-10 membered bicyclic, each with 1 to 4 heteroatoms independently selected from oxygen, nitrogen and sulfur.

The above definition of **heteroaryl** also applies if **heteroaryl** is part of another (combined) group as for example in **heteroaryl**amino, **heteroaryl**oxy or **heteroaryl**alkyl.

If the free valency of a **heteroaryl** is saturated, a **heteroaromatic group** is obtained.

The term **heteroarylene** is also derived from the previously defined **heteroaryl. Heteroarylene,** unlike **heteroaryl,** is bivalent and requires two binding partners. Formally, the second valency is obtained by removing a hydrogen atom from a **heteroaryl.** Corresponding groups are for example:
pyrrolyl and 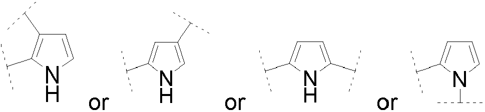 *etc.*

The above definition of **heteroarylene** also applies if **heteroarylene** is part of another (combined) group as for example in HO-**heteroarylene**amino or H₂N-**heteroarylene**oxy.

By **substituted** is meant that a hydrogen atom which is bound directly to the atom under consideration, is replaced by another atom or another group of atoms **(substituent).** Depending on the starting conditions (number of hydrogen atoms) mono- or polysubstitution may take place on one atom. Substitution with a particular substituent is only possible if the permitted valencies of the substituent and of the atom that is to be substituted correspond to one another and the substitution leads to a stable compound (*i.e*. to a compound which is not converted spontaneously, *e.g*. by rearrangement, cyclisation or elimination).

Bivalent substituents such as =S, =NR, =NOR, =NNRR, =NN(R)C(O)NRR, =N₂ or the like, may only be substituents on carbon atoms, whereas the bivalent substituents =O and =NR may also be a substituent on sulphur. Generally, substitution may be carried out by a bivalent substituent only at ring systems and requires replacement of two geminal hydrogen atoms, *i.e.* hydrogen atoms that are bound to the same carbon atom that is saturated prior to the substitution. Substitution by a bivalent substituent is therefore only possible at the group -CH₂- or sulphur atoms (=O group or =NR group only, one or two =O groups possible or, *e.g*., one =O group and one =NR group, each group replacing a free electron pair) of a ring system.

**Isotopes:** It is to be understood that all disclosures of an atom or compound of the invention include all suitable isotopic variations. In particular, a reference to hydrogen also includes deuterium.

**Stereochemistry/solvates/hydrates:** Unless specifically indicated, throughout the specification and appended claims, a given chemical formula or name shall encompass tautomers and all stereo, optical and geometrical isomers (*e.g*. enantiomers, diastereomers, *E*/*Z* isomers, *etc*.) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates and hydrates of the free compound or solvates and hydrates of a salt of the compound.

In general, substantially pure stereoisomers can be obtained according to synthetic principles known to a person skilled in the field, *e.g*. by separation of corresponding mixtures, by using stereochemically pure starting materials and/or by stereoselective synthesis. It is known in the art how to prepare optically active forms, such as by resolution of racemic forms or by synthesis, *e.g*. starting from optically active starting materials and/or by using chiral reagents.

Enantiomerically pure compounds of this invention or intermediates may be prepared *via* asymmetric synthesis, for example by preparation and subsequent separation of appropriate diastereomeric compounds or intermediates which can be separated by known methods (*e.g*. by chromatographic separation or crystallization) and/or by using chiral reagents, such as chiral starting materials, chiral catalysts or chiral auxiliaries.

Further, it is known to the person skilled in the art how to prepare enantiomerically pure compounds from the corresponding racemic mixtures, such as by chromatographic separation of the corresponding racemic mixtures on chiral stationary phases, or by resolution of a racemic mixture using an appropriate resolving agent, *e.g*. by means of diastereomeric salt formation of the racemic compound with optically active acids or bases, subsequent resolution of the salts and release of the desired compound from the salt, or by derivatization of the corresponding racemic compounds with optically active chiral auxiliary reagents, subsequent diastereomer separation and removal of the chiral auxiliary group, or by kinetic resolution of a racemate (*e.g*. by enzymatic resolution); by enantioselective crystallization from a conglomerate of enantiomorphous crystals under suitable conditions, or by (fractional) crystallization from a suitable solvent in the presence of an optically active chiral auxiliary.

**Salts:** The phrase **"pharmaceutically acceptable"** is employed herein to refer to those compounds, materials, compositions, and/or dosage forms which are, within the scope of sound medical judgement, suitable for use in contact with the tissues of human beings and animals without excessive toxicity, irritation, allergic response, or other problem or complication, and commensurate with a reasonable benefit/risk ratio.

As used herein **"pharmaceutically acceptable salts"** refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like.

For example, such salts include salts from benzenesulfonic acid, benzoic acid, citric acid, ethanesulfonic acid, fumaric acid, gentisic acid, hydrobromic acid, hydrochloric acid, maleic acid, malic acid, malonic acid, mandelic acid, methanesulfonic acid, 4-methyl-benzenesulfonic acid, phosphoric acid, salicylic acid, succinic acid, sulfuric acid and tartaric acid.

Further pharmaceutically acceptable salts can be formed with cations from ammonia, L-arginine, calcium, 2,2'-iminobisethanol, L-lysine, magnesium, *N*-methyl-D-glucamine, potassium, sodium and tris(hydroxymethyl)-aminomethane.

The pharmaceutically acceptable salts of the present invention can be synthesized from the parent compound which contains a basic or acidic moiety by conventional chemical methods. Generally, such salts can be prepared by reacting the free acid or base form of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention (*e.g.* trifluoro acetate salts), also comprise a part of the invention.

In a representation such as for example 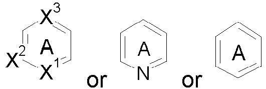 the letter A has the function of a ring designation in order to make it easier, for example, to indicate the attachment of the ring in question to other rings.

For bivalent groups in which it is crucial to determine which adjacent groups they bind and with which valency, the corresponding binding partners are indicated in brackets where necessary for clarification purposes, as in the following representations: 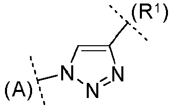 or (R²) -C(=O)NH- or (R²) -NHC(=O)-.

If such a clarification is missing then the bivalent group can bind in both directions, *i.e., e.g., -* C(=O)NH- also includes -NHC(=O)- (and vice versa).

Groups or substituents are frequently selected from among a number of alternative groups/substituents with a corresponding group designation (*e.g*. **R^{a}**, **R^{b}** etc). If such a group is used repeatedly to define a compound according to the invention in different parts of the molecule, it is pointed out that the various uses are to be regarded as totally independent of one another.

By a **therapeutically effective amount** for the purposes of this invention is meant a quantity of substance that is capable of obviating symptoms of illness or of preventing or alleviating these symptoms, or which prolong the survival of a treated patient.

### List of abbreviations

| | |
|---|---|
| Ac | acetyl |
| ACN | acetonitrile |
| aq. | aqueous |
| ATP | adenosine triphosphate |
| Bn | benzyl |
| Boc | *tert*-butyloxycarbonyl |
| Bu | butyl |
| c | concentration |
| CDI | 1,1'-carbonyldiimidazole |
| d | day(s) |
| TLC | thin layer chromatography |
| Davephos | 2-dimethylamino-2'-dicyclohexylaminophosphinobiphenyl |
| DBU | 1,8-Diazabicyclo(5.4.0)undec-7-ene |
| DCE | dichloroethane |
| DCM | dichloromethane |
| DEA | diethyl amine |
| DIPEA | *N*-ethyl-*N*,*N*-diisopropylamine (Hünig's base) |
| DMA | dimethylacetamide |
| DMAP | 4-*N*,*N*-dimethylaminopyridine |
| DME | 1,2-dimethoxyethane |
| DMF | *N*,*N*-dimethylformamide |
| DMSO | dimethylsulphoxide |
| DPPA | diphenylphosphorylazide |
| dppf | 1.1'-bis(diphenylphosphino)ferrocene |
| EDTA | ethylenediaminetetraacetic acid |
| EGTA | ethyleneglycoltetraacetic acid |
| equiv. | equivalent(s) |
| ESI | electron spray ionization |
| Et | ethyl |
| Et₂O | diethyl ether |
| EtOAc | ethyl acetate |
| EtOH | ethanol |
| h | hour(s) |
| HATU | O-(7-azabenzotriazol-1-yl)-*N*,*N*,*N*',*N*'-tetramethyl-uronium hexafluorophosphate |
| HPLC | high performance liquid chromatography |
| *i* | iso |
| conc. | concentrated |
| LC | liquid chromatography |
| LiHMDS | lithium bis(trimethylsilyl)amide |
| sln. | solution |
| Me | methyl |
| MeOH | methanol |
| min | minute(s) |
| MPLC | medium pressure liquid chromatography |
| MS | mass spectrometry |
| MTBE | methyl *tert-*butyl ether |
| NMM | *N*-methylmorpholine |
| NMP | *N*-methylpyrrolidone |
| NP | normal phase |
| n.a. | not available |
| PBS | phosphate-buffered saline |
| Ph | phenyl |
| Pr | propyl |
| PTSA | *p*-toluenesulfonic acid |
| Py | pyridine |
| rac | racemic |
| red. | reduction |
| Rf (R_{f}) | retention factor |
| RP | reversed phase |
| RRLC | Rapid resolution liquid chromatography |
| rt | ambient temperature |
| SFC | supercritical fluid chromatography |
| S_{N} | nucleophilic substitution |
| TBAF | tetrabutylammonium fluoride |
| TBDMS | *tert*-butyldimethylsilyl |
| TBME | *tert*-butylmethylether |
| TBTU | O-(benzotriazol-1-yl)-*N,N,N',N'*-tetramethyl-uronium tetrafluoroborate |
| tBu | *tert-*butyl |
| TEA | triethyl amine |
| temp. | temperature |
| *tert* | tertiary |
| Tf | triflate |
| TFA | trifluoroacetic acid |
| THF | tetrahydrofuran |
| t_{Ret}. | retention time (HPLC) |
| TRIS | tris(hydroxymethyl)-aminomethane |
| TsOH | *p*-toluenesulphonic acid |
| UPLC | ultra performance liquid chromatography |
| UV | ultraviolet |
| wt | weight |

### Examples

Features and advantages of the present invention will become apparent from the following detailed examples which illustrate the principles of the invention by way of example without restricting its scope:

### Preparation of the compounds according to the invention

### General

Unless stated otherwise, all the reactions are carried out in commercially obtainable apparatus using methods that are commonly used in chemical laboratories. Starting materials that are sensitive to air and/or moisture are stored under protective gas and corresponding reactions and manipulations therewith are carried out under protective gas (nitrogen or argon).

If a compound is to be represented both by a structural formula and by its nomenclature, in the event of a conflict the structural formula is decisive.

**Microwave reactions** are carried out in an initiator/reactor made by Biotage or in an Explorer made by CEM or in Synthos 3000 or Monowave 3000 made by Anton Paar in sealed containers (preferably 2, 5 or 20 mL), preferably with stirring.

### Chromatography

The thin layer chromatography is carried out on ready-made silica gel 60 TLC plates on glass (with fluorescence indicator F-254) made by Merck.

The **preparative high pressure chromatography (RP HPLC)** of the example compounds according to the invention is carried out on Agilent or Gilson systems with columns made by Waters (names: SunFire^{™} Prep C18, OBD^{™} 10 µm, 50 x 150 mm or SunFire^{™} Prep C18 OBD^{™} 5 µm, 30 x 50 mm or XBridge^{™} Prep C18, OBD^{™} 10 µm, 50 x 150 mm or XBridge^{™} Prep C18, OBD^{™} 5 µm, 30 x 150 mm or XBridge^{™} Prep C18, OBD^{™} 5 µm, 30 x 50 mm) and YMC (names: Actus-Triart Prep C18, 5 µm, 30 x 50 mm).

Different gradients of H₂O/acetonitrile are used to elute the compounds, while for Agilent systems 5 % acidic modifier (20 mL HCOOH to 1 L H₂O/acetonitrile (1/1)) is added to the water (acidic conditions). For Gilson systems the water is added 0.1 % HCOOH.

For the chromatography under basic conditions for Agilent systems H₂O/acetonitrile gradients are used as well, while the water is made alkaline by addition of 5 % basic modifier (50 g NH₄HCO₃ + 50 mL NH₃ (25 % in H₂O) to 1 L with H₂O). For Gilson systems the water is made alkaline as follows: 5mL NH₄HCO₃ solution (158 g in 1 L H₂O) and 2 mL NH₃ (28 % in H₂O) are replenished to 1 L with H₂O.

The **supercritical fluid chromatography (SFC)** of the intermediates and example compounds according to the invention is carried out on a JASCO SFC-system with the following colums: Chiralcel OJ (250 x 20 mm, 5 µm), Chiralpak AD (250 x 20 mm, 5 µm), Chiralpak AS (250 x 20 mm, 5 µm), Chiralpak IC (250 x 20 mm, 5 µm), Chiralpak IA (250 x 20 mm, 5 µm), Chiralcel OJ (250 x 20 mm, 5 µm), Chiralcel OD (250 x 20 mm, 5 µm), Phenomenex Lux C2 (250 x 20 mm, 5 µm).

The **analytical HPLC (reaction control)** of intermediate and final compounds is carried out using columns made by Waters (names: XBridge^{™} C18, 2.5 µm, 2.1 x 20 mm or XBridge^{™} C18, 2.5 µm, 2.1 x 30 mm or Aquity UPLC BEH C18, 1.7 µm, 2.1 x 50mm) and YMC (names: Triart C18, 3.0 µm, 2.0 x 30 mm) and Phenomenex (names: Luna C18, 5.0 µm, 2.0 x 30 mm). The analytical equipment is also equipped with a mass detector in each case.

### HPLC-mass spectroscopy/UV-spectrometry

The retention times/MS-ESI⁺ for characterizing the example compounds according to the invention are produced using an HPLC-MS apparatus (high performance liquid chromatography with mass detector). Compounds that elute at the injection peak are given the retention time t_{Ret.} = 0.00.

### Method A

| | | |
|---|---|---|
| HPLC | Agilent 1100 system | |
| MS | 1200Series LC/MSD(API-ES+/-3000V, Quadrupol, G6140) | |
| MSD signal settings | Scan pos/neg 120 - 900m/z | |
| Detection signal | 315 nm (bandwidth 170nm, reference off) | |
| Spectrum range | 230 - 400 nm | |
| Peak width | <0.01 min | |
| Column | Waters, Xbridge C18, 2.5 µm, 2.1x20 mm column | |
| Column temperature | 60°C | |
| Solvent | A: 20mM NH₄HCO₃/ NH₃ in H₂O pH 9 | |
| | B: ACN HPLC grade | |
| Flow | 1.00 mL/min | |
| Gradient | 0.00 - 1.50 min | 10 % to 95 % B |
| | 1.50 - 2.00 min | 95 % B |
| | 2.00 - 2.10 min | 95 % to 10 % B |

### Method B

| | |
|---|---|
| HPLC | Agilent 1260 system |
| MS | 1200 Series LC/MSD (MM-ES+APCI +/- 3000 V, Quadrupol, G6130) |
| Detection | UV: 254 nm (bandwidth 8, reference off) |
| | UV: 230 nm (bandwidth 8, reference off) |
| | UV spectrum range: 190 - 400 nm; step: 4 nm |
| | MS: positive and negative mode |
| Mass range | 100 - 800 m/z |
| Column | Waters; Part. No. 186003389; XBridge BEH C18, 2,5 µm, 30 x 2.1 mm |
| Column temperature | 45 °C |
| Solvent | A: 5 mM NH₄HCO₃/19 mM NH₃ in H₂O; B: ACN (HPLC grade) |
| Flow | 1.40 mL/min |
| Gradient | 0.00 - 1.00 min: 5 % B to 100 % B |
| | 1.00 - 1.37 min: 100 % B |
| | 1.37 - 1.40 min: 100 % B to 5 % B |

### Method C

| | |
|---|---|
| HPLC | Agilent 1260 Series |
| MS | Agilent LC/MSD Quadrupole |
| Detection | MS: positive and negative mode |
| Mass range | 100 - 750 m/z |
| Column | Waters X-Bridge BEH C18, 2.5 µm, 2.1 x 30 mm XP |
| Column temperature | 45 °C |
| Solvent | A: 20 mM NH₄HCO₃/30 mM NH₃ in H₂O; B: ACN (HPLC grade) |
| Flow | 1.40 mL/min |
| Gradient | 0.00 - 1.00 min: 15% B to 95% B |
| | 1.00 - 1.30 min: 95 % B |

### Method D

| | | |
|---|---|---|
| HPLC | Agilent 1100/1200 system | |
| MS | 1200 Series LC/MSD (MM-ES + APCI +/- 3000 V, Quadrupol, G6130B) | |
| MSD signal settings | Scan pos 150 - 750 | |
| Detection signal | UV 254 nm, 230 nm, 214 nm (bandwidth 8, reference off) | |
| Spectrum | range: 190 - 400 nm; slit: 4 nm | |
| Peak width | > 0.0031 min (0.063 s response time, 80Hz) | |
| Column | Waters, Part. No. 186003389, XBridge BEH C18, 2.5 µm, 2.1 x 30 mm) column | |
| Column temperature | 45 °C | |
| Solvent | A: 5 mM NH₄HCO₃/18 mM NH₃ in H2O (pH = 9.2) | |
| | B: ACN (HPLC grade) | |
| Flow | 1.4 mL/min | |
| Gradient | 0.0 - 1.0 min | 15 % to 95 % B |
| | 1.0 - 1.1 min | 95 % B |
| | Stop time: 1.3 min | |

### Method E

| | | |
|---|---|---|
| HPLC | Agilent 1100/1200 system | |
| MS | 1200 Series LC/MSD (MM-ES + APCI +/- 3000 V, Quadrupol, G6130B) | |
| MSD signal settings | Scan pos/neg 150 - 750 | |
| Detection signal | UV 254 nm, 230 nm, 214 nm (bandwidth 8, reference off) | |
| Spectrum | range: 190 - 400 nm; slit: 4 nm | |
| Peak width | > 0.0031 min (0.063 s response time, 80Hz) | |
| Column | Waters, Part. No. 186003389, XBridge BEH C18, 2.5 µm, 2.1 x 30 mm) column | |
| Column temperature | 45 °C | |
| Solvent | A: 5 mM NH₄HCO₃/18 mM NH₃ in H2O (pH = 9.2) | |
| | B: ACN (HPLC grade) | |
| Flow | 1.4 mL/min | |
| Gradient | 0.0 - 1.0 min | 15 % to 95 % B |
| | 1.0 - 1.1 min | 95 % B |
| | Stop time: 1.3 min | |

### Method F

| | | |
|---|---|---|
| HPLC | Agilent 1100/1200 system | |
| MS | 1200 Series LC/MSD (API-ES +/- 3000/3500 V, Quadrupol,G6140A) | |
| MSD signal settings | Scan pos/neg 150 - 750 | |
| Detection signal | UV 254 nm, 230 nm, 214 nm (bandwidth 10, reference off) | |
| Spectrum | range: 190 - 400 nm; slit: 4 nm | |
| Peak width | > 0.0031 min (0.063 s response time, 80Hz) | |
| Column | YMC; Part. No. TA12S03-0302WT; Triart C18, 3 µm, 12 nm; 30 x 2.0 mm column | |
| Column temperature | 45 °C | |
| Solvent | A: H₂O + 0,11% formic acid | |
| | B: ACN + 0,1% formic acid (HPLC grade) | |
| Flow | 1.4 mL/min | |
| Gradient | 0.0 - 1.0 min | 15 % to 95 % B |
| | 1.0 - 1.1 min | 95 % B |
| Stop time: | 1.23 min | |

### Method G

| | | |
|---|---|---|
| UPLC-MS | Waters Acquity-UPLC-SQ Detector-2 | |
| MSD signal settings | Scan pos & Neg 100 - 1500, | |
| | Source Votage: Capillary Vol(kV)- 3.50, Cone(V): 50 | |
| | Source Temp: Desolvation Temp(°C): 350 | |
| | Source Gas Flow: Desolvation (L/Hr): 750, Cone(L/Hr): 50 | |
| Detection signal | Diode Array | |
| Spectrum | Range: 200 - 400 nm; Resolution: 1.2nm | |
| Sampling rate | 10 point/sec | |
| Column | AQUITY UPLC BEH C18 1.7µm, 2.1X50mm | |
| Column temperature | 35 °C | |
| Solvent | A: 0.07% formic acid in ACN | |
| | B: 0.07% formic acid in water | |
| Flow | 0.6 mL/min | |
| Gradient | 0.0 - 0.30 min | 97% B |
| | 0.30 - 2.20 min | 97 % to 2 % B |
| | 2.20 - 3.30 min | 2 % B |
| | 3.30 - 4.50 min | 2 % to 97 % B |
| | 4.50 - 4.51 min | 97 % B |

### Method H

| | | |
|---|---|---|
| UPLC-MS | Waters Acquity-Binary Solvent Manager-UPLC-SQ Detector-2 | |
| MSD signal settings | Scan pos & Neg 100 - 1500, | |
| | Source Votage: Capillary Vol(kV)- 3.50, Cone(V): 50 | |
| | Source Temp: Desolvation Temp(°C): 350 | |
| | Source Gas Flow: Desolvation (L/Hr): 750, Cone(L/Hr): 50 | |
| Detection signal | Diode Array | |
| Spectrum | Range: 200 - 400 nm; Resolution: 1.2nm | |
| Sampling rate | 10 point/sec | |
| Column | AQUITY UPLC BEH C18 1.7µm, 2.1X50mm | |
| Column temperature | 35 °C | |
| Solvent | A: 0.07% formic acid in ACN | |
| | B: 0.07% formic acid in water | |
| Flow | 0.6 mL/min | |
| Gradient | 0.0 - 0.40 min | 97% B |
| | 0.40 - 2.50 min | 97 % to 2 % B |
| | 2.50 - 3.40 min | 2 % B |
| | 3.40 - 3.50 min | 2 % to 97 % B |
| | 3.50 - 4.0 min | 97 % B |

### Method I

| | | |
|---|---|---|
| LC-MS | Waters Arc-HPLC-SQ Detector-2 | |
| MSD signal settings | ESI Scan pos & neg | |
| | Capillary Voltage 3.50 Kv cone voltage 30V Desolvation gas 750L/hr Desolvation Temp 350°c | |
| Column | X-Bridge C18, 4.6x 75mm, 3.5µ | |
| Column temperature | 35 °C | |
| Solvent | A: 10mM Ammonium Acetate in water | |
| | B: ACN | |
| Flow | 1.0 mL/min | |
| Gradient | 0.0 - 0.75 min | 5% B |
| | 0.75 - 1.50 min | 5 % to 40 % B |
| | 1.50 - 5.0 min | 40 % to 98 % B |
| | 5.0 - 7.0 min | 98 % B |

### Method J

| | | |
|---|---|---|
| LC-MS | Waters Acquity-UPLC-SQ Detector-2 | |
| MSD signal settings | ESI Scan pos & neg | |
| | Capillary Voltage 3.50 Kv cone voltage 50V Desolvation gas 750L/h Desolvation Temp 350°C | |
| Column | Waters Acquity-UPLC-SQ Detector-2 | |
| Column temperature | 35 °C | |
| Solvent | A: 0.05% TFA in ACN | |
| | B: 0.05% TFA in water | |
| Flow | 0.6 mL/min | |
| Gradient | 0.0 - 0.3 min | 97% B |
| | 0.3 - 2.2 min | 97 % to 2 % B |
| | 2.2 - 3.3 min | 2 % B |

### Method K

| | | |
|---|---|---|
| LC-MS | Waters Arc-HPLC-SQ Detector-2 | |
| MSD signal settings | ESI Scan pos & neg | |
| | Capillary Voltage 3.50 Kv cone voltage 30V Desolvation gas | |
| | 750L/hr Desolvation Temp 350°C | |
| Column | X-Bridge C18, 4.6x 50mm, 3.5µ | |
| Column temperature | 35 °C | |
| Solvent | A: 10mM Ammonium Acetate in water | |
| | B: ACN | |
| Flow | 2.0 mL/min | |
| Gradient | 0.0 - 0.2 min | 10% B |
| | 0.2 - 2.50 min | 10 % to 75 % B |
| | 2.50 - 3.0 min | 75 % to 100 % B |
| | 3.0 - 4.8 min | 100 % B |

### Method L

| | |
|---|---|
| HPLC | Agilent 1260 Series |
| MS | Agilent LC/MSD Quadrupole |
| Detection | MS: positive and negative mode |
| Mass range | 550 - 1200 m/z |
| Column | Waters X-Bridge BEH C18, 2.5 µm, 2.1 x 30 mm XP |
| Column temperature | 45 °C |
| Solvent | A: 20 mM NH₄HCO₃/30 mM NH₃ in H₂O; B: ACN (HPLC grade) |
| Flow | 1.40 mL/min |
| Gradient | 0.00 - 1.50 min: 50% B to 95% B |
| | 1.50 - 2.00 min: 95 % B |

### Method M

| | |
|---|---|
| HPLC | Agilent 1260 Series |
| MS | Agilent LC/MSD Quadrupole |
| Detection | MS: positive and negative mode |
| Mass range | 550 - 1200 m/z |
| Column | Waters X-Bridge BEH C18, 2.5 µm, 2.1 x 30 mm XP |
| Column temperature | 45 °C |
| Solvent | A: 20 mM NH₄HCO₃/30 mM NH₃ in H₂O; B: ACN (HPLC grade) |
| Flow | 1.40 mL/min |
| Gradient | 0.00 - 1.00 min: 50% B to 95% B |
| | 1.00 - 1.30 min: 95 % B |

### Method N

| | |
|---|---|
| HPLC | Agilent 1260 Series |
| MS | Agilent LC/MSD Quadrupole |
| Detection | MS: positive and negative mode |
| Mass range | 100 - 750 m/z |
| Column | YMC-Triart C18, 3µm, 12nm, 2.0x30mm |
| Column temperature: | 45 °C |
| Solvent | A: H₂O+0,11% formic acid; B: ACN (HPLC grade)+0,1% formic acid |
| Flow: | 1.40 mL/min |
| Gradient: | 0.00 - 1.00 min: 15% B to 95% B |
| | 1.00- 1.30 min: 95 % B |

### Method O

| | | |
|---|---|---|
| HPLC | Waters - Alliance 2996 | |
| Detection signal | PDA Detector | |
| Spectrum | Range: 200 - 400 nm; Resolution: 1.2nm | |
| Sampling rate | 1 point/sec | |
| ELSD Parameters | Gas Pressure:50 PSI, Drift tube Temp: 50°C, Gain:500 | |
| Column | Atlantis T3 (4.6 x 250mm) 5.0µm | |
| Column temperature | Ambient | |
| Solvent | A: 10 mM ammonium acetate in water | |
| | B: ACN | |
| Flow | 0.7 mL/min | |
| Gradient | 0.0 - 1.20 min | 2% B |
| | 1.2 - 10.0 min | 2% to 98 % B |
| | 10.0 - 12.0 min | 98% B |
| | 12.0 - 14.0 min | 97% to 2 % B |
| | 14.0 - 16.0 min | 2 % B |

### Method P

| | | |
|---|---|---|
| UPLC-MS | Waters Acquity-UPLC-SQ Detector-2 | |
| MSD signal settings | Scan Positive & Negative 100 - 1500, | |
| | Source Voltage: Capillary Voltage(kV)- 3.50, Cone(V): 50 | |
| | Source Temp: Desolvation Temp(°C): 350 | |
| | Source Gas Flow: Desolvation (L/Hr): 650 | |
| Detection signal | Diode Array | |
| Spectrum | Range: 200 - 400 nm; Resolution: 1.2nm | |
| Sampling rate | 10 point/sec | |
| ELSD Parameters: | GAS:2.0 SLM, Nebulizer Temp:40°C, Evaporative Temp:45°C | |
| Column | AQUITY UPLC BEH C18 1.7µm, 2.1X50mm | |
| Column temperature | 50 °C | |
| Solvent | A: 0.05% formic acid in water | |
| | B: 0.05% formic acid in ACN | |
| Flow | 0.6 mL/min | |
| Gradient | 0.0 - 2.20 min | 3% to 98% B |
| | 2.20 - 3.20 min | 98% B |
| | 3.20 - 3.50 min | 98% to 3% B |
| | 3.50 - 4.20 min | 2% B |

### Method Q

| | | |
|---|---|---|
| HPLC-MS | Waters Arc-HPLC with 2998PDA Detector and SQ Detector-2 | |
| MSD signal settings | Scan Pos & Neg 100 - 1500, | |
| | Source Votage: Capillary Vol(kV)- 3.50, Cone(V): 30 | |
| | Source Temp: Desolvation Temp(°C): 350 | |
| | Source Gas Flow: Desolvation (L/h): 750 | |
| Detection signal | PDA Detector | |
| Spectrum | Range: 200 - 400 nm; Resolution: 1.2nm | |
| Sampling rate | 10 point/sec | |
| Column | X-Bridge C18, 4.6x 50mm, 3.5µm | |
| Column temperature | 35 °C | |
| Solvent | A: 10 mM ammonium acetate in water | |
| | B: ACN | |
| Flow | 1.0 mL/min | |
| Gradient | 0.0 - 0.75 min | 5% B |
| | 0.75 - 1.50 min | 5 % to 40 % B |
| | 1.50 - 5.0 min | 40 % to 98 % B |
| | 5.0 - 7.0 min | 98 % B |
| | 7.0 - 9.0 min | 98 % to 5% B |
| | 9.0 - 10.01 min | 5% B |

### Method R

| | | |
|---|---|---|
| HPLC | | Agilent 1200 system |
| Column | | Chiralpak IE, 5.0 µm, 2.1x150 mm column |
| Column temperature | | 40°C |
| Solvent | | EtOH/Heptane 1:1 + 0.1% diethylamine (isocratic) |
| Flow | | 0.60 mL/min |

### GCMS

### Method U

| | |
|---|---|
| GC | Agilent Technologies-7890B GC System with 7693 Auto Sampler and 5977A MSD |
| Injection Temperature | 230°C |
| Column Flow | 2.0 mL/min |
| Solvent delay | 1.5 min |
| Split Ratio | 10:01 |
| Column Oven Temperature Program | 100°C/1 min, 20°C/min/310°/5min |
| Total run time | 16 min |
| Interface Temperature | 150°C |
| Ion Source Temperature | 230°C |
| Gas | He |
| Column & Column dimension | ZB-5MS (30m X 0.32mm; 1µm) |
| MSD Scan Range | 50-900 |

### Method V

| | |
|---|---|
| GC | Agilent Technologies-7890B GC System with 7693 Auto Sampler and 5977A MSD |
| Injection Temperature | 230°C |
| Column Flow | 2.0 mL/min |
| Solvent delay | 1.5 min |
| Split Ratio | 10:01 |
| Column Oven Temperature Program | 40°C/2 min, 15°C /min/200°/1min, |
| | 25°C/min/310°/0min, |
| Total run time | 18min |
| Interface Temperature | 150°C |
| Ion Source Temperature | 230°C |
| Gas | He |
| Column & Column dimension | ZB-5MS (30m X 0.32mm; 1µm) |
| MSD Scan Range | 50-900 |

### Method W

| | |
|---|---|
| GC | Agilent Technologies-7890B GC System with 7693 Auto Sampler and 5977A MSD |
| Injection Temperature | 230°C |
| Column Flow | 2.0 mL/min |
| Solvent delay | 1.5 min |
| Split Ratio | 10:01 |
| Column Oven Temperature Program | 60°C/3 min, 20°C/min/310°/2min |
| Total run time | 18 min |
| Interface Temperature | 150°C |
| Ion Source Temperature | 230°C |
| Gas | He |
| Column & Column dimension | ZB-5MS (30m X 0.32mm; 1µm) |

### Method SFC-1

| | |
|---|---|
| Make | Waters UPC²-MS |
| Soft | Empower3 |
| MS | QDa |
| Column | CHIRALCEL OX-3(4.6*150MM) 3µm |
| A-Solvent | CO2 |
| B-solvent | ACN |
| Total Flow | 3g/min |
| % of Co-Solvent | 15 |
| ABPR | 1500psi |
| Colum temp | 30°C |
| PDA range | 200nm to 400nm |
| Resolution | 1.2nm |
| MS Parameters | - |
| QDa MS scan range | 100Da to 1000Da |
| | Cone voltage |
| Positive scan | 20V |
| Negative Scan | 15V |

The compounds according to the invention and intermediates are prepared by the methods of synthesis described hereinafter in which the substituents of the general formulae have the meanings given hereinbefore. These methods are intended as an illustration of the invention without restricting its subject matter and the scope of the compounds claimed to these examples. Where the preparation of starting compounds is not described, they are commercially obtainable or their synthesis is described in the prior art or they may be prepared analogously to known prior art compounds or methods described herein, *i.e.* it is within the skills of an organic chemist to synthesize these compounds. Substances described in the literature can be prepared according to the published methods of synthesis. If a chemical structure in the following is depicted without exact configuration of a stereo center, e.g. of an asymmetrically substituted carbon atom, then both configurations shall be deemed to be included and disclosed in such a representation. The representation of a stereo center in racemic form shall always deem to include and disclose both enantiomers (if no other defined stereo center(s) exists) or all other potential diastereomers and enantiomers (if additional, defined or undefined, stereo centers exist).

### Synthesis of spiroketone intermediates A

### Experimental procedure for the synthesis of A-2a

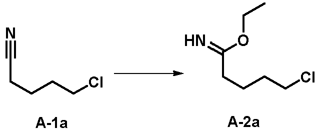

To a suspension of 5-chloropentanenitrile (22.9 g, 195 mmol, 1.00 equiv.) in EtOH (136 mL) is added acetyl chloride (111 mL, 1.56 mol, 8.00 equiv.) dropwise at 0 °C. The reaction mixture is allowed to warm to rt and stirred for 12 h. The mixture is concentrated under reduced pressure and washed with Et₂O and the crude product **A-2a** is used as the HCl salt directly in the next step without further purification (HPLC method: A; tᵣₑₜ = 1.03 min; [M+H]⁺ = 164).

### Experimental procedure for the synthesis of A-3a

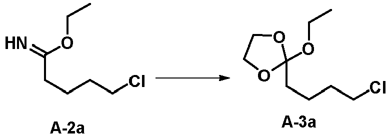

Crude **A-2a** (HCl salt) (28.0 g, 140 mmol, 1.00 equiv.) and ethylene glycol (7.38 g, 119 mmol, 0.90 equiv.) are dissolved in DCM (300 mL) and stirred at rt for 6 d. The resulting suspension is concentrated under reduced pressure, diluted with Et₂O (200 mL) and filtered. The filtrate is concentrated under reduced pressure, taken up in DCM (200 mL) and treated with a KOH solution (2 M in water, 150 mL). The mixture is stirred at rt overnight keeping the phases intact. The phases are separated, the water phase is extracted with DCM (2x) and the combined organic phases are dried over magnesium sulfate, filtered and concentrated under reduced pressure. The crude orthoester **A-3a** is used for the next step without further purification (HPLC method: A; tᵣₑₜ = 1.37 min; [M+H]⁺ = 163).

### Experimental procedure for the synthesis of A-4a

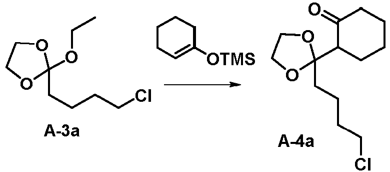

Crude **A-3a** (22.3 g, 107 mmol, 1.00 equiv.), 1-cyclohexenyloxytrimethylsilane (16.4 mL, 82.3 mmol, 0.80 equiv.) and zinc chloride (10.2 g, 74.8 mmol, 0.70 equiv.) are dissolved in DCM (120 mL) and stirred at rt for 5 h. The reaction mixture is treated by addition of saturated sodium hydrogencarbonate solution. The organic phase is separated, dried over magnesium sulfate, filtered and concentrated under reduced pressure. The crude product is purified by NP-chromatography to give the desired compound **A-4a** (HPLC method: A; tᵣₑₜ = 1.25 min; [M+Na]⁺ = 283).

### Experimental procedure for the synthesis of A-8a

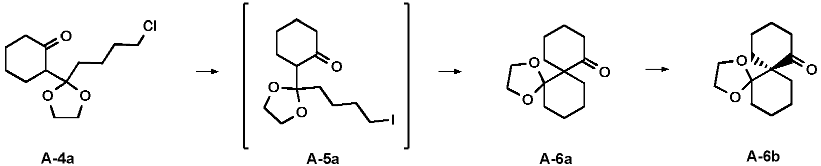

**A-4a** (14.9 g, 57.1 mmol, 1.0 equiv.) and sodium iodide (26.0 g, 171 mmol, 3.0 equiv.) are dissolved in acetone (120 mL) and stirred under reflux for 16 h. The reaction mixture is concentrated under reduced pressure, diluted with DCM and washed with a saturated sodium thiosulfate solution. The organic phase is separated, dried over MgSO₄, filtered and concentrated under reduced pressure. The crude product **A-5a** is used for the next step without further purification.

**A-5a** (30 g, 85.0 mmol, 1.0 equiv.) is dissolved in THF. The mixture is treated with potassium tert.-butoxide (28.7 g, 256 mmol, 3.0 equiv.) at 0 °C and stirred at rt overnight. The reaction mixture is quenched by addition of water (2 mL), diluted by addition of Et₂O and a saturated sodium hydrogencarbonate solution. The organic phase is separated, dried over MgSO₄, filtered and concentrated under reduced pressure. The crude product is purified by NP-chromatography to give (racemic) compound **A-6a** (The reaction sequence **A-1a** → **A-6a** is based on Marko et al., THL 2003, 44, 3333-3336 and Maulide et al., Eur. J. Org. Chem. 2004, 19:3962-3967.

Enantiomer **A-6b** can then be obtained after chiral separation via SFC using the following conditions: Column: Lux;Cellulose-4 (250mmX30mmX5µm), 90% CO2, 10% ACN, Flow: 90g/min, Temp: 30°C, enantiomer A-6b (SFC-method: SFC-1, tᵣₑₜ=2.99min) as peak 2 after enantiomer elutes.

### Synthesis of alcohol-intermediates B

### Experimental procedure for the synthesis of B-2a

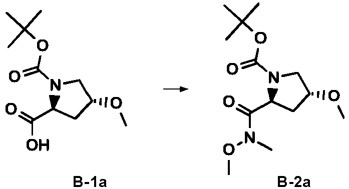

**B-1a** (4.92 g, 19.1 mmol, 1.00 equiv.), *N*,*N*-carbonyldiimidazole (5.14 g, 28.6 mmol, 1.50 equiv.) and mol. sieves (3 Å, 500 mg) are dissolved in DCM (29.5 mL) and stirred for 40 min at rt. After complete activation, *N,O*-dimethylhydroxylamine hydrochloride (2.79 g, 28.6 mmol, 1.50 equiv.) is added and the reaction is stirred again for 2 h at rt. When complete, water (100 mL) and DCM (150 mL) are added and the phases are separated, the water phase is extracted with DCM (2 x). The combined organic phases are washed with brine and concentrated under reduce pressure. The residue is purified by NP chromatography to give the product **B-2a.**

The following intermediates **B-2** (Table 1) are available in an analogous manner. The crude products are purified by chromatography if necessary.

**Table 1**

| **#** | **structure** | **tᵣₑₜ [min]** | **[M+H]⁺** | **HPLC method** |
|---|---|---|---|---|
| **B-2a** | 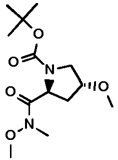 | 0.43 | 189 ([M+H-Boc]⁺) | C |
| **B-2b** | 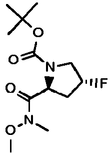 | 0.47 | 177 ([M+H-Boc]⁺) | C |

### Experimental procedure for the synthesis of B-3a

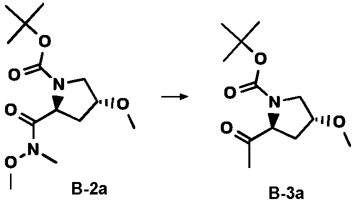

**B-2a** (4.88 g, 16.9 mmol, 1.00 equiv.) is dissolved in THF (15 mL) under an argon atmosphere and cooled to -10 °C. Bromo(methyl)magnesium (3.4 M in MeTHF, 6.46 mL, 22.0 mmol, 1.3 equiv.) is added and stirred for 1 h at -10 °C. After complete conversion, the reaction mixture is cooled to -20 °C and quenched by addition of brine. The resulting mixture is extracted with DCM (3 x). The combined organic phases are concentrated under reduced pressure to obtain **B-3a.**

The following intermediates **B-3** (Table 2) are available in an analogous manner. The crude products are purified by chromatography if necessary.

**Table 2**

| **#** | **structure** | **tᵣₑₜ [min]** | **[M+H]⁺** | **HPLC method** |
|---|---|---|---|---|
| **B-3a** | 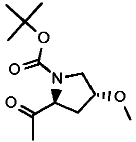 | 0.97 | 144 ([M+H-Boc]⁺) | A |
| **B-3b** | 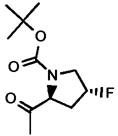 | 0.50 | 132 ([M+H-Boc]⁺) | C |

### Experimental procedure for the synthesis of B-4a

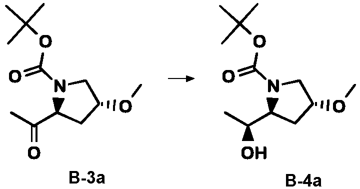

(*R*)-Methyl oxazaborolidine (0.99 g, 3.3 mmol, 0.20 equiv.) is dissolved in THF (2 mL) under an argon atmosphere and cooled to -5 °C. Borane-dimethyl sulfide complex (1.0 M, 22 mL 22 mmol, 1.3 equiv.) is added. The mixture is stirred for 30 min at rt. The mixture is cooled to -5 °C and **B-3a** (4.1 g, 17 mmol, 1 equiv.) is added slowly dropwise. The reaction is stirred at rt for 1 h. After complete conversion of starting material, the reaction is cooled to -10 °C and quenched by addition of MeOH. The mixture is concentrated under reduced pressure. The residue is dissolved in water (150 mL) and formic acid (0.5 mL) and extracted with DCM (3 x). The combined organic phases are concentrated under reduced pressure and purified by NP chromatography to give the product **B-4a.**

The following intermediates **B-4** (Table 3) are available in an analogous manner. The crude products are purified by chromatography if necessary.

**Table 3**

| **#** | **structure** | **tᵣₑₜ [min]** | **[M+H]⁺** | **HPLC method** |
|---|---|---|---|---|
| **B-4a** | 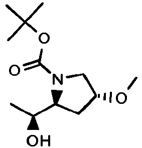 | 0.44 | 190 ([M+H-tBu]⁺) | C |
| **B-4b** | 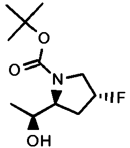 | 0.46 | 178 ([M+H-tBu]⁺) | C |

### Experimental procedure for the synthesis of B-5a

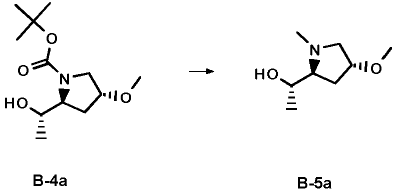

**B-4a** (306 mg, 12.5 mmol, 1.00 equiv.) is dissolved in THF, (30.6 mL) under an argon atmosphere. Lithium aluminium hydride (1 M in THF, 24.9 mL, 25.0 mmol, 2.00 equiv.) is added slowly. Reaction is stirred at 60 °C for 1 h. After complete conversion, the reaction is cooled to rt, Rochelle salt solution and KOH is added and stirred for 1 h. The existing suspension is extracted with DCM (3 x), the combined organic phases are concentrated under reduce pressure to yield **B-5a.**

The following intermediates **B-5** (Table 4) are available in an analogous manner. The crude products are purified by chromatography if necessary.

**Table 4**

| **#** | **structure** | **tᵣₑₜ [min]** | **[M+H]⁺** | **HPLC method** |
|---|---|---|---|---|
| **B-5a** | 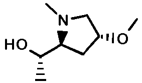 | 0.92 | 160 | A |
| **B-5b** | 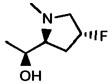 | 0.92 | 148 | A |

### Synthesis of pyrimidine derivatives C

### Experimental procedure for the synthesis of C-3a:

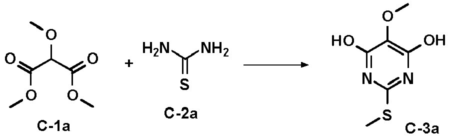

To a stirred solution of 2-methoxy-malonic acid dimethyl ester (36.0 g, 222 mmol, 1.00 equiv.) and thiourea (25.4 g, 333 mmol, 1.50 equiv.) in MeOH (360 mL) is added sodium methoxide (27.8 g, 555 mmol, 2.5 equiv.) at rt and the mixture is stirred at 80 °C for 24 h. After complete conversion, iodomethane (41.0 g, 289 mmol, 1.30 equiv.) is added slowly at rt and the mixture is stirred at rt for 16 h. After complete conversion, the reaction mixture is concentrated, water is added, and the reaction mixture is stirred for 30 min. The product is collected by filtration, washed with water, and dried under vacuum. The crude product **C-3a** is used for the next step without purification. (HPLC method: H, tᵣₑₜ = 0.89 min; [M+H]⁺ = 189).

### Experimental procedure for the synthesis of C-4a:

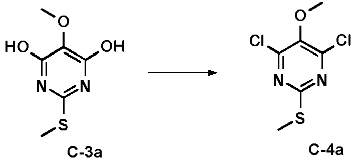

To a stirred mixture of **C-3a** (3.1 g, 16 mmol, 1.0 equiv.) and N,N-diethylaniline (0.4 mL) at 0 °C, POCl₃ (13 g, 81 mmol, 5.0 equiv.) is added slowly and the resulting mixture is stirred for 16 h at 90 °C. After complete conversion the mixture is cooled to rt, excess POCl₃ is evaporated, water is added, and the product is isolated by extraction with EtOAc. The crude product is purified via NP chromatography to obtain **C-4a** (HPLC method: H, tᵣₑₜ = 2.12 min; [M+H]⁺ = 225).

### Experimental procedure for the synthesis of C-5a:

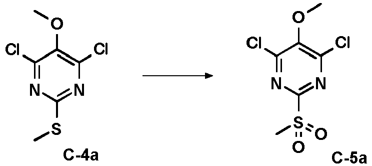

To a stirred solution of **C-4a** (24.0 g, 107 mmol, 1.00 equiv.) in DCM (240 mL) at 0 °C, m-CPBA (55.0 g, 321 mmol, 3.0 equiv.) is added and the mixture is allowed to reach rt and stirred for additional 16 h. After complete conversion, the mixture is diluted with DCM washed with saturated NaHCO₃, and the organic layer is dried, filtered, and concentrated to yield **C-5a** which is used for the next step without purification. (HPLC method: H, tᵣₑₜ = 1.68 min; [M+H]⁺ = 257).

### Synthesis of nitrile intermediates D

### Experimental procedure for the synthesis of D-1a:

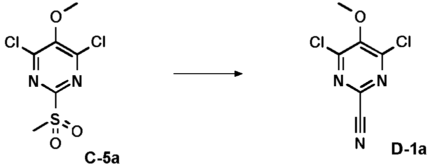

To a stirred solution of **C-5a** (24.0 g, 93.4 mmol, 1.0 equiv.) in ACN (216 mL) and water (24 mL) under nitrogen at 0 °C, NaCN (5.49 g, 112 mmol, 1.2 equiv.) is added and the mixture is allowed to reach rt and stirred for additional 1 h. After complete conversion, water and EtOAc is added, the organic layer is separated, washed with water, dried, filtered, and concentrated and the crude product is purified via NP chromatography yielding D-1a.

### Synthesis of esters and acids E

### Experimental procedure for the synthesis of E-2a:

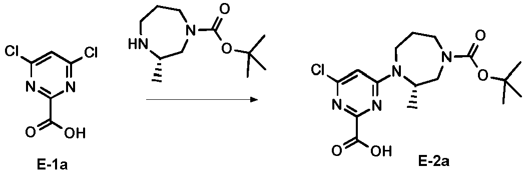

4,6-dichloropyrimidine-2-carboxylic acid **E-1a** (900 mg, 4.66 mmol, 1.0 equiv.) is dissolved in DMSO (2 mL) and DIPEA (1.5 mL, 8.8 mmol, 2.0 equiv.) and (S)-tert-butyl 3-methyl-1,4-diazepane-1-carboxylate (1049 mg, 4.896 mmol, 1.05 equiv.) is added dropwise. The reaction mixture is then stirred at 40 °C fo18 h. The mixture is diluted with acetonitrile and purified by acidic RP chromatography to give the desired product **E-2a** (HPLC method: A, tᵣₑₜ = 0.82 min, [M+H]⁺ = 371).

### Experimental procedures for the synthesis of intermediates E-3a:

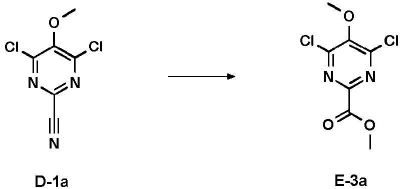

**D-1a** (7.00 g, 34.3 mmol, 1.0 equiv.) is added to a stirred solution of HCl (4 M in MeOH, 105 mL, 420 mmol, 12.4 equiv.) at 0 °C. The mixture is allowed to reach rt and stirred for an additional 16 h. After complete conversion, the reaction mixture is concentrated and the crude product is purified via NP chromatography to give the desired product **E-3a** (HPLC method: H, tᵣₑₜ = 1.48 min; [M+H]⁺ = 237).

### Experimental procedure for the synthesis of intermediates E-5 (Method I):

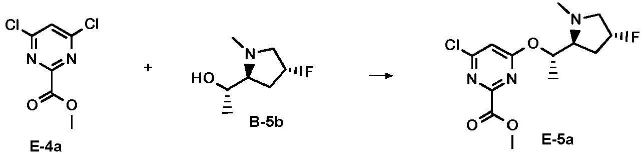

Methyl 4,6-dichloropyrimidine-2-carboxylate **E-4a** (3.00 g, 14.5 mmol, 1.0 equiv.) is dissolved in DCM (30 mL) and DIPEA (5.34 mL, 29.0 mmol, 2.0 equiv.) and **B-5b** (3.20 g, 21.8 mmol, 1.5 equiv.) are added. The reaction mixture is then stirred at rt fo18 h. After complete conversion, the mixture is concentrated, water is added, and the mixture is extracted with EtOAc and the organic phases are washed with brine, dried filtered and concentrated. The crude product is purified by NP chromatography yielding **E-5a.**

The following intermediates **E-5** (Table 5) are available in an analogous manner. The crude products are purified by chromatography if necessary.

**Table 5**

| **#** | **structure** | **tᵣₑₜ [min]** | **[M+H]⁺** | **HPLC method** |
|---|---|---|---|---|
| **E-5a** | 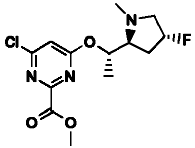 | 1.07 | 318 | H |
| **E-5b** | 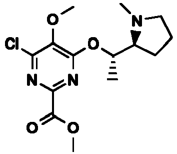 | 1.09 | 330 | H |

### Experimental procedure for the synthesis of intermediates E-5c (Method II):

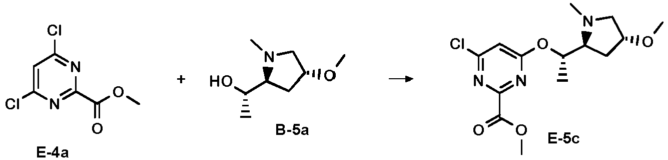

**B-5a** (100 mg, 0.48 mmol, 1 equiv.) is dissolved in THF (500 µL), LiHMDS (591 µL, 0.59 mmol, 1.1 equiv.) is added and stirred for 5 min. Meanwhile methyl 4,6-dichloropyrimidine-2-carboxylate **E-4a** (170 mg, 0.81 mmol, 1.5 equiv.) is dissolved in THF (500 µL). Solution of **B-5a** is added dropwise over 5 min to the methyl 4,6-dichloropyrimidine-2-carboxylate solution. The reaction is stirred for 25 min. After complete conversion, the reaction mixture is filtered and purified by RP chromatography to give **E-5c** (HPLC method: A, tᵣₑₜ = 1.08 min, [M+H]⁺ = 330).

### Synthesis of diketones F

When multiple HPLC retention times are reported it means that different tautomers are present.

### Experimental procedure for the synthesis of F-1a

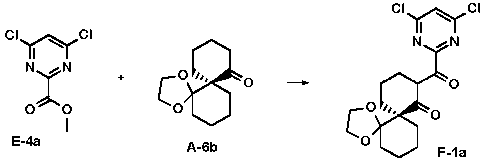

4,6-Dichloropyrimidine-2-carboxylic acid methyl ester **E-4a** (2.00 g, 9.67 mmol, 1.00 equiv.) is dissolved in dry ACN (5 mL) under nitrogen atmosphere. Magnesium bromide diethyl etherate (2.99 g, 11.6 mmol, 1.20 equiv.), a solution of **A-6b** (2.38 g, 10.6 mmol, 1.10 equiv.) in ACN (5 mL) and DIPEA (2.67 mL, 14.5 mmol, 1.50 equiv.) is added and the reaction mixture is stirred at 50 °C for 20 h. After complete conversion, the reaction mixture is carefully quenched with 1 M HCl, diluted with water, extracted with DCM, the organic phases are dried, filtered, and concentrated to obtain crude **F-1a.** The crude compound is purified by NP chromatography. (HPLC method: H, tᵣₑₜ = 2.50 min; [M+H] = 399/401).

### Experimental procedure for the synthesis of F-2a

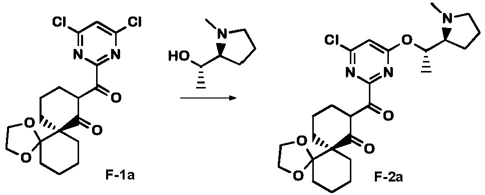

**F-1a** (10.0 g, 19.4 mmol, 1.00 equiv.) is dissolved in DMSO (10 mL), (1S)-1-[(2S)-1-methylpyrorolidin-2-yl]ethanol (2.76 g, 21.4 mmol, 1.10 equiv.) and DIPEA (6.78 mL, 38.8 mmol, 2.0 equiv.) are added and the solution is stirred at rt overnight. The reaction mixture is diluted with DCM and water. The organic phase is separated, evaporated and the resulting residue is purified by RP chromatography to afford **F-2a.** (HPLC-method: A, tᵣₑₜ = 1.58/1.66 min; [M+H] = 492).

### Experimental procedure for the synthesis of F-3a

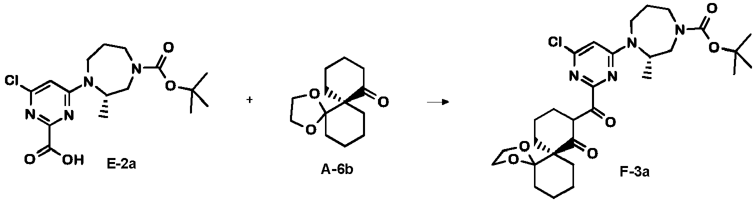

**E-2a** (1.05 g, 2.83 mmol, 1.00 equiv.) and 1-(1H-imidazole-1-carbonyl)-1H-imidazole (918 mg, 5.66 mmol, 2.00 equiv.) under an argon atmosphere are dissolved in dry THF (5 mL) and stirred 1 h at rt. After complete activation of the acid a solution of **A-6b** (1.34 g, 5.98 mmol, 2.00 equiv.) and LiHMDS (1.0 M in THF, 5.95 mL, 5.95 mmol, 2.10 equiv.) is added to the reaction mixture and washed with dry THF (5 mL). The resulting mixture is stirred overnight at 60 °C. After full conversion, the reaction is diluted with an aqueous saturated NaHCO₃ solution and extracted three times with DCM. The organic phases are combined, dried, filtered and concentrated under reduced pressure to give the crude product. The crude product is dissolved in acetonitrile and water, filtered and purified by basic RP chromatography to give the desired product **F-3a.** (HPLC method: C, tᵣₑₜ = 0.888/0.936/0.978 min, [M+H] = 557).

### Experimental procedure for the synthesis of intermediates F-4

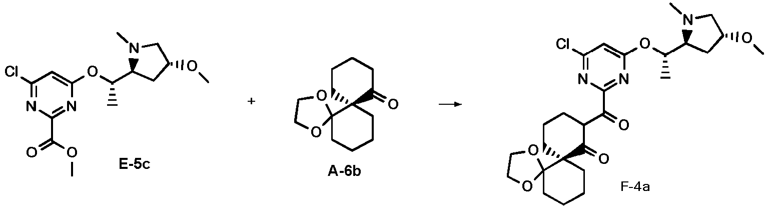

**E-5c** (1.80 g, 0.01 mol, 1.00 equiv.) is dissolved in dry THF (18 mL), activated molecular sieves (3 Å) are added (200 mg per 1 mL solvent) and stirred at 50 °C for 20 min under an argon atmosphere. Then magnesium bromide ethyl etherate (2.11 g, 0.01 mol, 1.5 equiv.) is added and further stirred at 50 °C for 30 min. Meanwhile a second solution is prepared using the **A-6b** (1.47 g, 0.01 mol, 1.5 equiv.), which is also predried using activated molecular sieves (3 Å) at 50 °C for 20 min in THF (8 mL). Then LiHMDS (1 M in THF, 13.7 mL, 0.01 mol, 2.5 equiv.) is added and stirred for 15 min. After that the second solution is added to the first solution and stirred for 1 h at 50 °C until complete conversion to the product is observed. The reaction mixture is carefully quenched with water, THF is removed under reduced pressure. The residue pH is adjusted to 7-8 by using 1 M HCl and extracted with 5 % MeOH in DCM, combined organic layer is washed with brine, dried, filtered and concentrated. The crude compound **F-4a** is purified by NP chromatography.

The following intermediates **F-4** (Table 6) are available in an analogous manner. The crude products are purified by chromatography if necessary.

**Table 6**

| **#** | **structure** | **tᵣₑₜ [min]** | **[M+H]⁺** | **HPLC method** |
|---|---|---|---|---|
| **F-4a** | 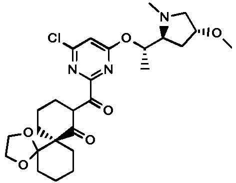 | 5.69 | 522 | I |
| | | 6.00 | | |
| | | 6.13 | | |
| **F-4b** | 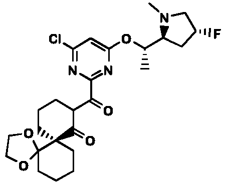 | 1.62 | 510 | H |
| | | 1.75 | | |
| **F-4c** | 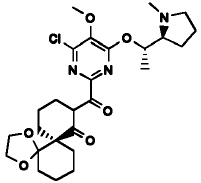 | 1.54 | 522 | H |
| | | 1.68 | | |

### Synthesis of isoxazole intermediates G

### Experimental procedure for the synthesis of intermediates G3 and G4

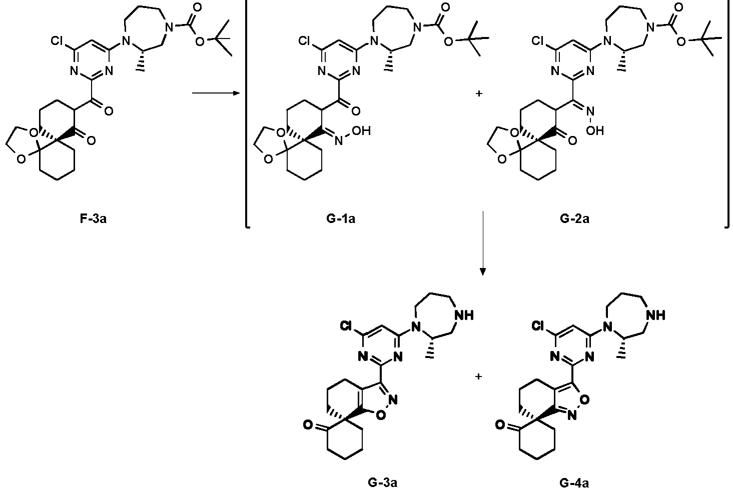

**F-3a** (1.10 g, 1.91 mmol, 1.0 equiv.) is dissolved in 1,4-dioxane (3 mL) and 50 % aq. Hydroxylamine is added (140 µL, 2.29 mmol, 1.2 equiv.). The reaction mixture is stirred overnight at rt. After full conversion of starting material, the reaction is diluted with aq. satd. NaHCO₃ solution and extracted three times with DCM. The organic phases are combined, dried, filtered and concentrated under reduced pressure to give the crude product. The crude mixture of **G-1a** and **G-2a** (1.00 g, 1.68 mmol, 1.0 equiv.) is dissolved in 1,4-dioxane (6 mL) and 4 M HCl aq. (2.11 mL, 8.44 mmol, 5.0 equiv.) is added. The reaction mixture is stirred 3 h at rt. After full conversion of starting material is observed, the reaction is diluted with aq. saturated NaHCO₃ solution and extracted three times with DCM. The organic phase is combined, dried, filtered and concentrated under reduced pressure to give the crude product. The crude product is dissolved in ACN and water, filtered, and purified by basic RP chromatography to give the desired product **G-3a** besides the corresponding isoxazole regioisomer **G-4a.**

The following intermediates **G-3** and **G-4** (Table 7) are available in an analogous manner from suitable intermediates **F.** The crude products are purified by chromatography if necessary.

**Table 7**

| **#** | **structure** | **tᵣₑₜ [min]** | **[M+H]⁺** | **HPLC method** |
|---|---|---|---|---|
| **G-3a** | 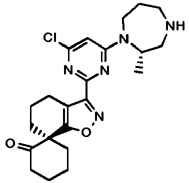 | 0.67 | 430 | C |
| **G-3b** | 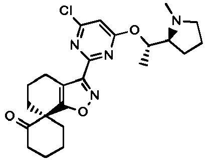 | 1.57 | 445 | A |
| **G-3c** | 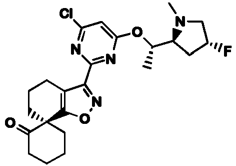 | 1.52 | 463 | A |
| **G-3d** | 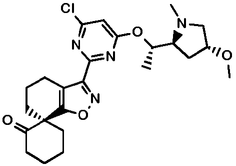 | 3.01 | 475 | K |
| **G-4b** | 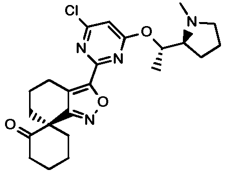 | 1.62 | 445 | A |

### Experimental procedure for the synthesis of G-7a

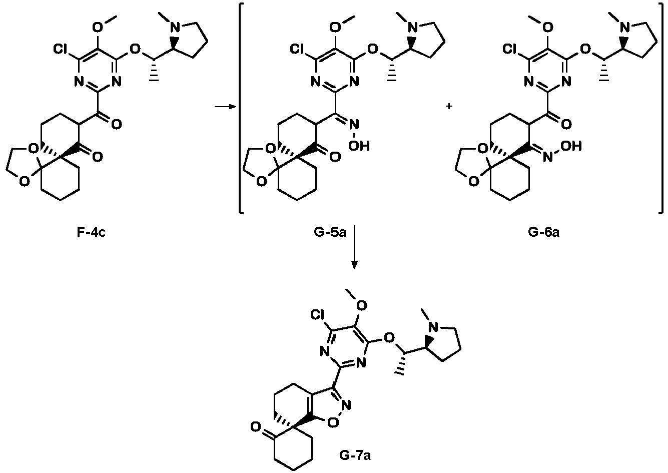

**F-4c** (1.40 g, 2.68 mmol, 1.0 equiv.), is dissolved in dioxane (1 mL), formic acid (103 µL, 2.95 mmol, 1.10 equiv.) and 50 % aq. hydroxylamine solution (50 % in water, 165 µL, 2.95 mmol, 1.10 equiv.) is added and stirred overnight. After complete conversion of starting material is observed the reaction is concentrated under reduced pressure and purified by NP chromatography to give **G-5a** (HPLC method: C, tᵣₑₜ = 1.60 min; [M+H]⁺ = 537).

**G-5a** (1.60 g, 2.98 mmol, 1.0 equiv.) is dissolved in HCl (4 M in dioxane, 2.9 mL) and stirred for 5 min at rt. Then HCl 4M in water (2.5 mL) is added and the reaction is stirred for 30 min at rt. After complete conversion, the reaction mixture is basified with NaHCO₃ and extracted with DCM. The combined organic phases are concentrated under reduced pressure to give the product **G-7a** (HPLC method: H, tᵣₑₜ = 1.59 min; [M+H]⁺ = 537).

### Experimental procedure for the synthesis of G-8a

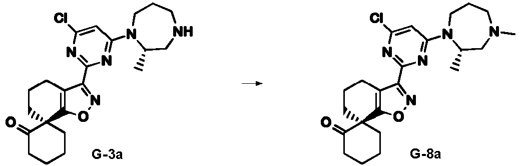

**G-3a** (500 mg, 1.07 mmol, 1.0 equiv.) is dissolved in dry MeOH (10 mL) and formaldehyde (403 µL, 5.36 mmol, 5.0 equiv.) and acetic acid (27 µL, 0.54 mmol, 0.5 equiv.) are added. This is followed by the addition of sodium cyanoborohydride (141 mg, 2.14 mmol, 2.0 equiv.). The solution is stirred for 1 h at rt. After complete consumption of starting material, the reaction is quenched by the addition of water and sat NaHCO₃. The aqueous phase is extracted with DCM (3 x). The combined organic phases are filtered and concentrated under reduced pressure. The residue is dissolved in acetonitrile and purified by RP chromatography to give the desired product **G-8a** (HPLC method: A, tᵣₑₜ = 1.39 min; [M+H]⁺ = 444).

### Experimental procedure for the synthesis of G-9 (Method I)

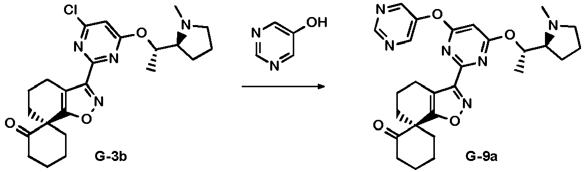

**G-3b** (150 mg, 309 µmol, 1.0 equiv.), 5-hydroxypyrimidine (44.5 mg, 463 µmol, 1.5 equiv.) and Cs₂CO₃ (201 mg, 617 µmol, 2.0 equiv.) are dissolved in dry DMSO (2 mL) and stirred under an argon atmosphere for 18 h at 80 °C. After complete conversion the mixture is diluted with DCM and extracted with water and brine. The combined organic phases are concentrated under reduce pressure and purified by RP chromatography to give the desired product G-9a.

The following intermediates **G-9** (Table 8) are available in an analogous manner. The crude products are purified by chromatography if necessary.

**Table 8**

| **#** | **Structure** | **tᵣₑₜ [min]** | **[M+H]⁺** | **HPLC method** |
|---|---|---|---|---|
| **G-9a** | 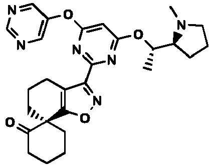 | 1.41 | 505 | A |
| **G-9b** | 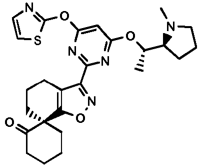 | 0.91 | 510 | C |
| **G-9c** | 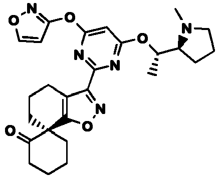 | 1.50 | 494 | A |

### Experimental procedure for the synthesis of G-9 (Method II)

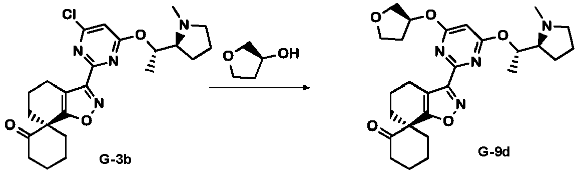

(S)-(+)-3-hydroxytetrahydrofuran (41.7 mg, 0.45 mmol, 2.0 equiv.) is added at 0 °C to a solution of KOtBu/THF (449 µL, 0.45 mmol, 2.0 equiv.) and stirred for 5 min. **G-3b** (100 mg, 0.22 mmol, 1.0 equiv.) dissolved in THF (1 mL) is added. The reaction is stirred for 5 min at rt. After complete conversion, the reaction mixture is extracted with DCM/water and the organic phase is concentrated under reduced pressure. The residue is purified by RP chromatography to give **G-9d.**

The following intermediates **G-9** (Table 9) are available in an analogous manner. The crude products are purified by chromatography if necessary.

**Table 9**

| **#** | **Structure** | **tᵣₑₜ [min]** | **[M+H]⁺** | **HPLC method** |
|---|---|---|---|---|
| **G-9d** | 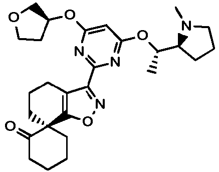 | 0.88 | 497 | C |
| **G-9e** | 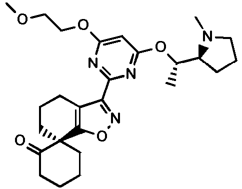 | 1.48 | 485 | A |
| **G-9f** | 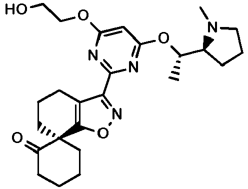 | 1.30 | 471 | A |

### Experimental procedure for the synthesis of G-9g (method III)

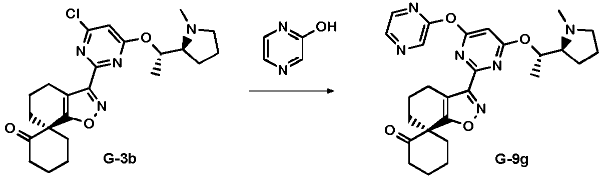

**G-3b** (250 mg, 272 µmol, 1.0 equiv.), 2-hydroxypyrazine (31.3 mg, 326 µmol, 1.2 equiv.) and Cs₂CO₃ (177 mg, 543 µmol, 2.0 equiv.) are dissolved in toluol (1 mL) and stirred under an argon atmosphere 18 h at 110°C. After complete conversion, the reaction mixture is filtered and concentrated under reduce pressure. The residue is dissolved in DCM and extracted with water and brine. The combined organic phases are concentrated under reduce pressure and purified by RP chromatography to give the desired product **G-9g** (HPLC method: A; tᵣₑₜ = 1.43 min; [M+H]⁺ = 505).

### Experimental procedure for the synthesis of G-9 and G-10 (method IV)

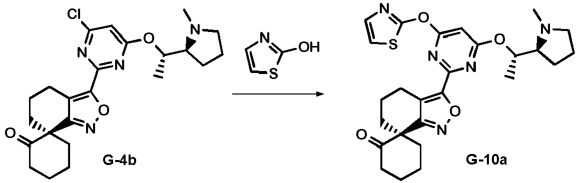

**G-4b** (150 mg, 0.3 mmol, 1.0 equiv.), 2-hydroxythiazole (39.4 mg, 0.39 mmol, 1.30 equiv.) t-BuONa (2 M in THF, 210 µL, 0.42 mmol, 1.4 equiv.) is dissolved in THF (1.5 mL) and stirred at 80 °C for 18 h. After complete conversion, the reaction mixture is extracted 3 times with DCM/H₂O. The combined organic phases are concentrated under reduced pressure and purified by RP chromatography to give the desired product **G-10a.**

The following intermediates **G-9** and **G-10** (Table 10) are available in an analogous manner from **G-3b** and **G-4b.** The crude products are purified by chromatography if necessary.

**Table 10**

| **#** | **structure** | **tᵣₑₜ [min]** | **[M+H]⁺** | **HPLC method** |
|---|---|---|---|---|
| **G-10a** | 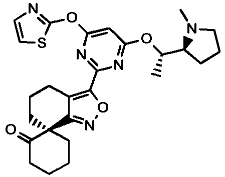 | 0.92 | 504 | C |
| **G-10b** | 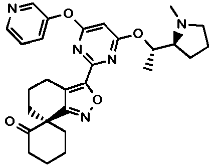 | 0.82 | 504 | C |
| **G-10c** | 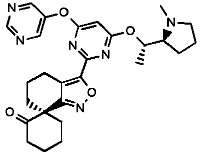 | 0.78 | 505 | C |
| **G-9h** | 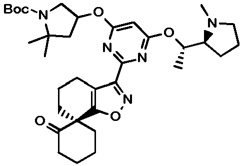 | 1.05 | 624 | C |
| **G-9i** | 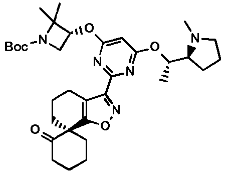 | 1.04 | 610 | C |

### Experimental procedure for the synthesis of G-9 and G-10 (method V)

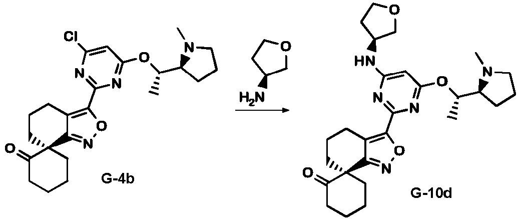

**G-4b** (100 mg, 225 µmol, 1.0 equiv.), (S)-3-aminotetrahydrofuran (39.0 mg, 448 µmol, 2.0 equiv.) and DIPEA (235 µL, 1.35 mmol, 6.0 equiv.) are dissolved in dry DMSO (1.0 mL) and stirred at 90 °C for 18 h. The reaction mixture is diluted with DCM and washed with water. The organic phase is dried with magnesium sulfate, evaporated and the resulting residue is purified by RP chromatography to afford **G-10d.**

The following intermediates **G-9** and **G-10** (Table 11) are available in an analogous manner from **G-3b** and **G-4b.** The crude products are purified by chromatography if necessary.

**Table 11**

| **#** | **structure** | **tᵣₑₜ [min]** | **[M+H]⁺** | **HPLC method** |
|---|---|---|---|---|
| **G-10d** | 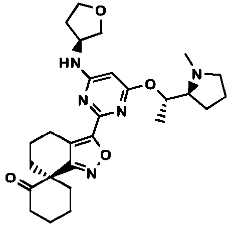 | 1.46 | 496 | A |
| **G-10e** | 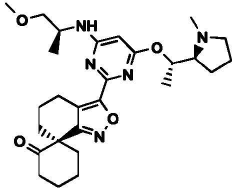 | 1.51 | 498 | A |
| **G-9j** | 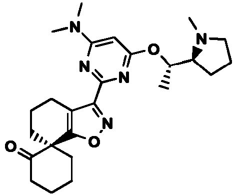 | 0.84 | 454 | E |
| **G-9k** | 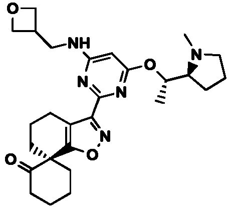 | 0.70 | 496 | C |

### Experimental procedure for the synthesis of G-9 and G-10 (method VI)

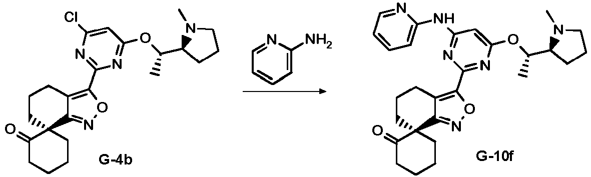

**G-4b** (120 mg, 0.27 mmol, 1.0 equiv.), 2-aminopyridine (31.7 mg, 0.76 mmol, 1.25 equiv.), Xantphos (4.68 mg, 0.01 mmol, 0.03 equiv.), Cs₂CO₃ (131 mg, 0.40 mmol, 1.5 equiv.) and tris(dibenzylideneacetone)dipalladium (0) (2.47 mg, 0.26 µmol, 0.01 equiv.) are dissolved in dioxane (1.2 mL) and stirred under an argon atmosphere for 16 h at 110 °C. After complete conversion, the reaction is filtered and purified by RP chromatography to give the desired product **G-10f.**

The following intermediates **G-9** and **G-10** (Table 12) are available in an analogous manner from **G-3b** and **G-4b.** The crude products are purified by chromatography if necessary.

**Table 12**

| **#** | **structure** | **tᵣₑₜ [min]** | **[M+H]⁺** | **HPLC method** |
|---|---|---|---|---|
| **G-10f** | 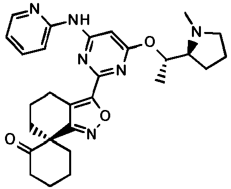 | 1.59 | 503 | A |
| **G-9l** | 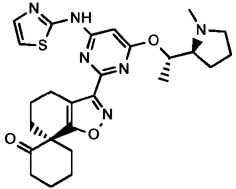 | 1.45 | 509 | A |
| **G-9m** | 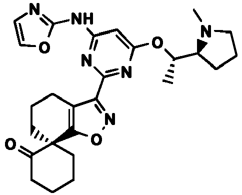 | 1.43 | 493 | A |

### Experimental procedure for the synthesis of G-9 (method VII)

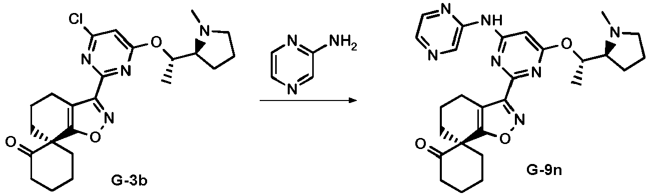

**G-3b** (125 mg, 0.28 mmol, 1.0 equiv.), aminopyrazine (66.8 mg, 702 µmol, 2.5 equiv.), Cs₂CO₃ (275mg, 0.84mmol, 3equiv.), palladium(II)acetate (5 mg, 0.02 mmol, 0.08 equiv.), (S)-(-)-2,2-bis(diphenylphosphino)-1-binaphtyl (14 mg, 0.02 mmol, 0.08 equiv.) are dissolved in dry toluol (5 mL) and stirred for 2 d at 110 °C. After complete conversion, the reaction mixture is allowed to cool to rt, filtered, and concentrated under reduced pressure. The reaction is purified by RP chromatography to give the desired product **G-9n.**

The following intermediates **G-9** and **G-10** (Table 13) are available in an analogous manner from **G-3b** and **G-4b.** The crude products are purified by chromatography if necessary.

**Table 13**

| **#** | **structure** | **tᵣₑₜ [min]** | **[M+H]⁺** | **HPLC method** |
|---|---|---|---|---|
| **G-9n** | 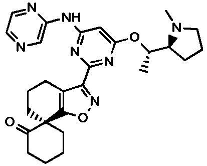 | 0.77 | 504 | C |
| **G-9o** | 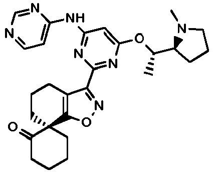 | 0.72 | 504 | C |
| **G-9p** | 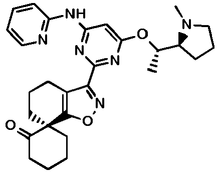 | 0.89 | 503 | C |
| **G-9q** | 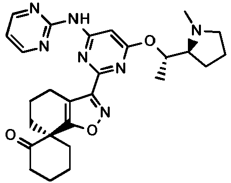 | 0.81 | 504 | C |
| **G-9r** | 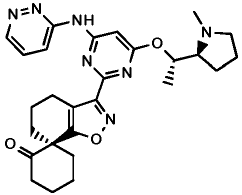 | 0.68 | 504 | C |

### Experimental procedure for the synthesis of G-10g (method VIII)

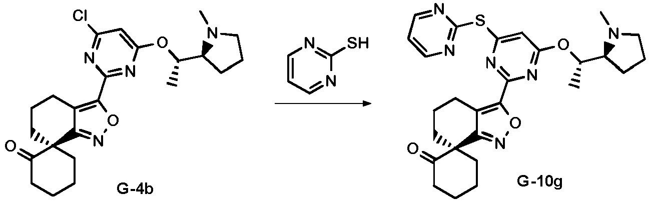

**G-4b** (250 mg, 272 µmol, 1.0 equiv.), 2-mercaptopyrimidine (50.4 mg, 449 µmol, 2.0 equiv.) and Cs₂CO₃ (145 mg, 449 µmol, 2.0 equiv.) are dissolved in dry DMA (0.9 mL) and stirred under an argon atmosphere 3 h at 100 °C. After complete conversion, the reaction mixture is diluted with DCM and extracted with water and brine. The combined organic phases are concentrated under reduce pressure and purified by RP chromatography to give the desired product **G-10g** (HPLC method: A; tᵣₑₜ = 1.53 min; [M+H]⁺ = 521).

### Experimental procedure for the synthesis of G-9

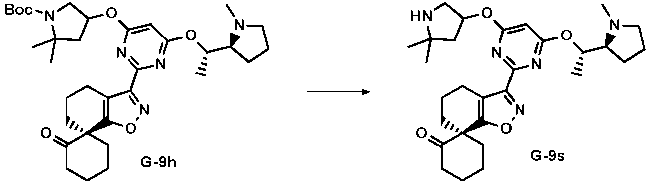

**G-9h** (297 mg, 476 µmol, 1.0 equiv.) is dissolved in DCM (0.91 mL) and trifluoracetic acid (0.99 mL, 4.76 mmol, 10.0 equiv.). The reaction is stirred 4 h at rt. After complete conversion, the dissolved is removed under reduced pressure. The residue is dissolved in DCM and extract with aq. saturated Na₂CO₃. The combined organic phases are dried, filtered, and concentrated under reduced pressure. The residue is purified by RP chromatography to give **G-9s.**

The following intermediates **G-9** (Table 14) are available in an analogous manner from **G-9h** and **G-9i.** The crude products are purified by chromatography if necessary.

**Table 14**

| **#** | **structure** | **tᵣₑₜ [min]** | **[M+H]⁺** | **HPLC method** |
|---|---|---|---|---|
| **G-9s** | 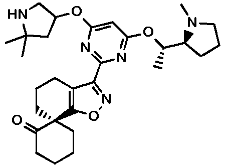 | 1.53 | 524 | A |
| **G-9t** | 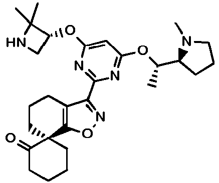 | 0.58 | 510 | C |
| | | 0.73 | | |

### Synthesis of aminocyanothiophenes H, I, and II

### Experimental procedure for the conversion of G-10 to I

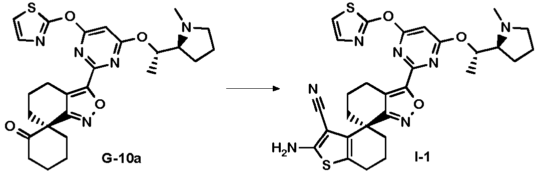

**G-10a** (52.9 mg, 104 µmol, 1.0 equiv.), malononitrile (20 mg, 288 µmol, 2.77 equiv.), sulfur (6.2 mg, 193 µmol, 1.86 equiv.), *beta*-alanine (11.9 mg, 127 µmol, 1.22 equiv.) and mol. sieves (3Å) are suspended in methanol (1.0 mL) and stirred at 80 °C for 18 h. The reaction mixture is diluted with DCM, filtered and washed with aq. saturated NaHCO₃. The organic phase is separated, and the remaining aq. phase is extracted with DCM. The combined organic phases are dried with magnesium sulfate, evaporated and the resulting residue is purified by RP chromatography to afford **I-1.**

The following compounds **I** (Table 15) are available in an analogous manner from the corresponding ketones **G-10.** The crude products are purified by chromatography if necessary.

**Table 15**

| **#** | **structure** | **tᵣₑₜ [min]** | **[M+H]⁺** | **HPLC method** |
|---|---|---|---|---|
| **I-1** | 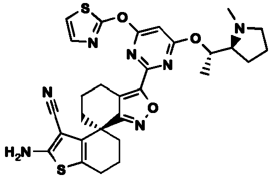 | 1.61 | 590 | A |
| **I-2** | 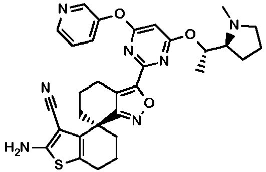 | 1.50 | 584 | A |
| **I-3** | 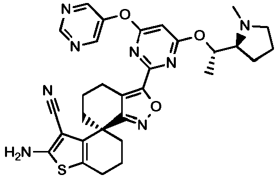 | 1.45 | 585 | A |
| **I-4** | 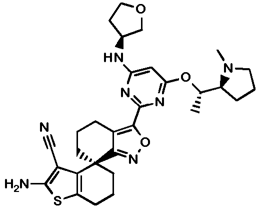 | 1.43 | 576 | A |
| **I-5** | 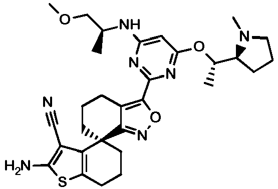 | 1.52 | 578 | A |
| **I-6** | 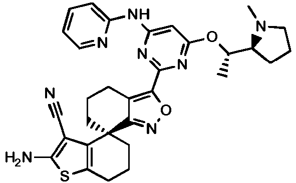 | 1.65 | 583 | A |
| **I-7** | 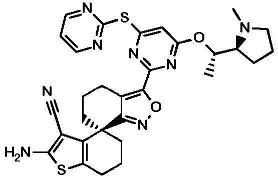 | 1.55 | 601 | A |

### Experimental procedure for the conversion of G-9 to II

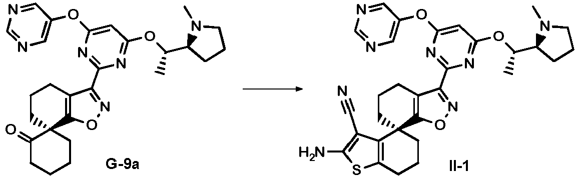

To a solution of **G-9a** (75.0 mg, 0.149 mmol, 1.0 equiv.) and mol. sieves (3Å) in anhydrous methanol (4 mL) under an argon atmosphere, are added malononitrile (20.7 mg, 0.297 mmol, 2.0 equiv.), sulfur (7.15 mg, 0.223 mmol, 1.5 equiv.) and ß-alanine (16.7 mg, 0.178 mmol, 1.2 equiv.). The reaction mixture is stirred at 80 °C overnight. After complete conversion, the mixture is cooled to rt, filtered and extracted with DCM and aq. saturated NaHCO₃. The organic phases are combined and concentrated under reduced pressure. The residue is dissolved in in acetonitrile and water and purified by basic RP chromatography to give the desired product **II-1.**

The following compounds **II** (Table 16) are available in an analogous manner from the corresponding ketones **G-9.** The crude products are purified by chromatography if necessary.

After conversion of the diastereomeric mixture of corresponding ketones **G-9h** followed by RP chromatography (Waters XBridge C18 30x50mm 5µm, gradient 40-70% ACN in aq. NH₄HCO₃/NH₃) **II-8** is obtained as a single diastereomer besides the undesired isomer.

**Table 16**

| **#** | **structure** | **tᵣₑₜ [min]** | **[M+H]⁺** | **HPLC method** |
|---|---|---|---|---|
| **II-1** | 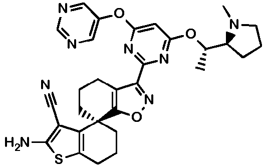 | 1.46 | 585 | A |
| **II-2** | 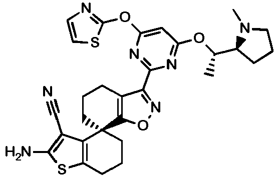 | 1.63 | 590 | A |
| **II-3** | 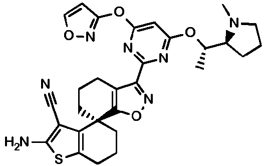 | 1.53 | 574 | A |
| **II-4** | 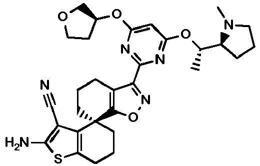 | 1.52 | 577 | A |
| **II-5** | 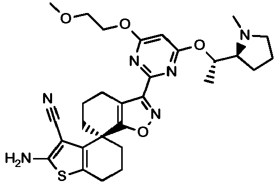 | 1.52 | 565 | A |
| **II-6** | 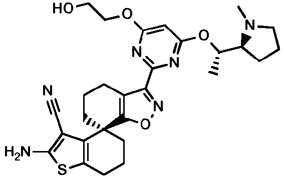 | 1.36 | 551 | A |
| **II-7** | 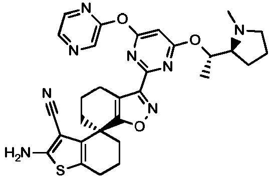 | 1.47 | 585 | A |
| **II-8** | 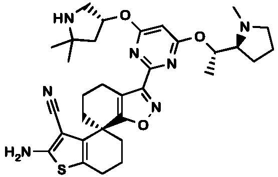 | 1.60 | 604 | A |
| **II-9** | 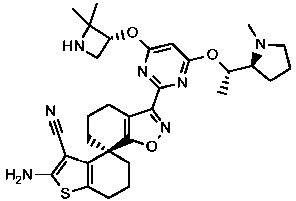 | 1.49 | 590 | A |
| **II-10** | 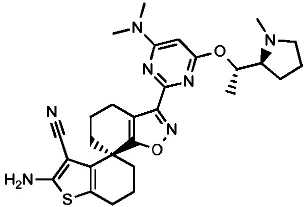 | 1.53 | 534 | A |
| **II-11** | 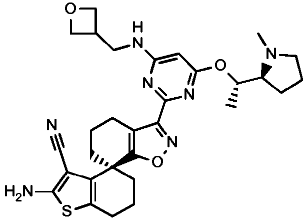 | 1.38 | 576 | A |
| **II-12** | 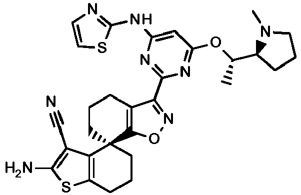 | 1.5 | 589 | A |
| **II-13** | 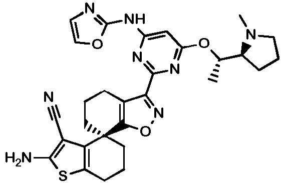 | 1.47 | 573 | A |
| **II-14** | 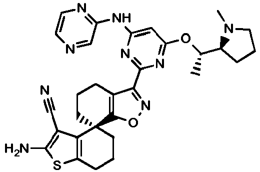 | 1.49 | 584 | A |
| **II-15** | 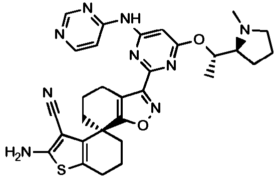 | 1.56 | 584 | A |
| **II-16** | 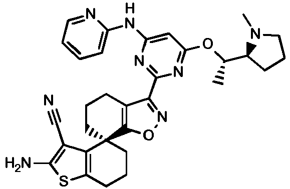 | 1.62 | 583 | A |
| **II-17** | 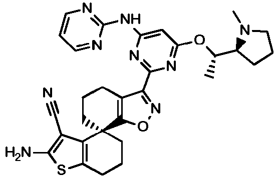 | 1.54 | 584 | A |
| **II-18** | 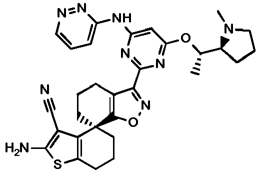 | 1.4 | 584 | A |

### Experimental procedure for the synthesis of H-1a

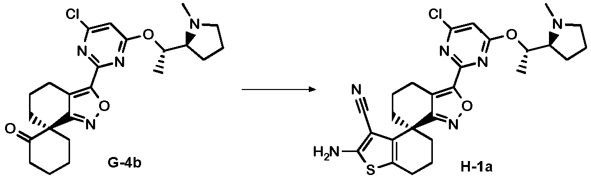

To a solution of **G-4b** (76.1 mg, 0.16 mmol, 1.0 equiv.) and mol. sieves (3Å) in anhydrous methanol (2 mL) under an argon atmosphere, are added malononitrile (14.5 mg, 0.21 mmol, 1.33 equiv.), sulfur (10.1 mg, 0.31 mmol, 1.98 equiv.) and ß-alanine (19.4 mg, 0.22 mmol, 1.38 equiv.). The reaction mixture is stirred at 80 °C overnight. After complete conversion, the mixture is cooled to rt, filtered and extracted with DCM and aq. satd. NaHCO₃. The organic phases are combined and concentrated under reduced pressure. The residue is dissolved in in acetonitrile and water and purified by basic RP chromatography to give the desired product **H-1a.** (HPLC method: A, tᵣₑₜ = 2.16 min; [M+H]⁺ = 525).

### Experimental procedure for the synthesis of H-2

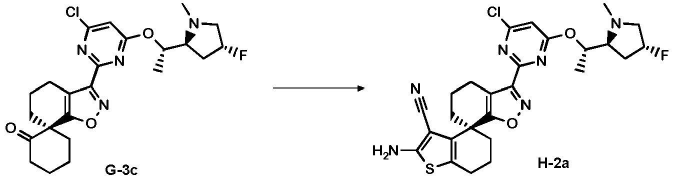

**G-3c** (1.2 g, 2.59 mmol, 1.0 equiv.), ammonium acetate (319 mg, 4.15 mmol, 1.6 equiv.), and sulfur (133 mg, 4.15 mmol, 1.6 equiv.) are dissolved in ethanol (12 mL) and stirred at 60 °C for 15 min. Malonitrile as a solution in ethanol (3.77 mL, 4.28 mmol, 1.65 equiv.) is added slowly dropwise (8mL/h). Reaction is stirred for 5 h at 80 °C. After full conversion, the reaction is concentrated and purified by NP chromatography. Product fractions are diluted with DCM and washed with aq. saturated NaHCO₃. The organic phase is dried filtered and concentrated under reduce pressure to obtain **H-2a.**

The following intermediates **H-2** (Table 17) are available in an analogous manner. The crude products are purified by chromatography if necessary.

**Table 17**

| **#** | **structure** | **tᵣₑₜ [min]** | **[M+H]⁺** | **HPLC method** |
|---|---|---|---|---|
| **H-2a** | 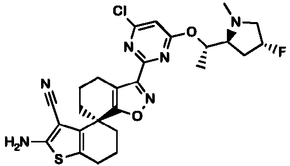 | 1.59 | 543 | A |
| **H-2b** | 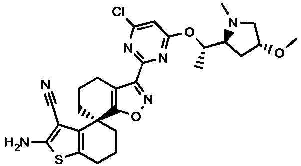 | 1.54 | 555 | A |
| **H-2c** | 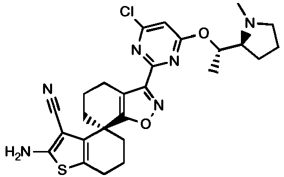 | 0.83 | 525 | E |
| **H-2d** | 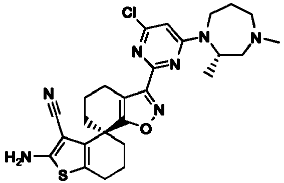 | 0.89 | 525 | E |
| **H-2e** | 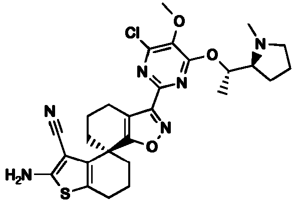 | 1.62 | 555 | A |

### Experimental procedure for the synthesis of II-19

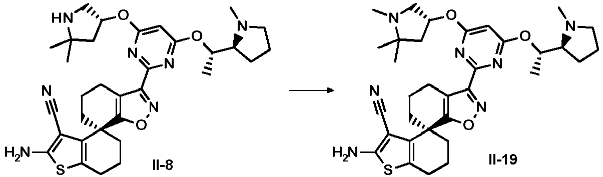

**II-8** (23.0 mg, 0.038 mmol, 1.0 equiv.) is dissolved in dry DCM (0.5 mL) under argon and cooled down to -30 °C. Formaldehyde (37 % in water, 3.4 µL, 0.046 mmol, 1.2 equiv.) is added followed by the addition of sodium triacetoxyborohydride (17.0 mg, 0.076 mmol, 2.0 equiv.). The solution is stirred for 30 min at -30 °C. After complete consumption of starting material, the reaction is quenched by the addition of water. The aqueous phase is extracted with DCM (3 x). The combined organic phases are filtered and concentrated under reduced pressure. The residue is dissolved in acetonitrile and purified by RP chromatography to give the desired product **II-19.** (HPLC method: A, tᵣₑₜ = 1.66 min; [M+H]⁺ = 618).

### Experimental procedure for the conversion of H-2c to II (method I)

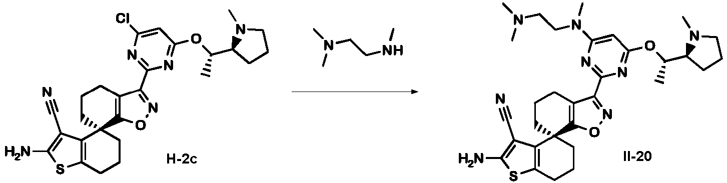

**H-2c** (100 mg, 0.19 mmol, 1.0 equiv.) is suspended in ethanol (0.70 mL). DIPEA (49.8 µL, 0.29 mmol, 1.5 equiv.) and N,N,N'-trimethylethylenediamine (29.5 µL, 0.229 mmol, 1.2 equiv.) are added and the reaction mixture is stirred overnight at 80 °C. After full conversion, the reaction mixture is filtered and purified with RP chromatography yielding **II-20.** (HPLC method: A, tᵣₑₜ = 1.49 min; [M+H]⁺ = 591).

### Experimental procedure for the conversion of H-1 and H-2 to I and II (method II)

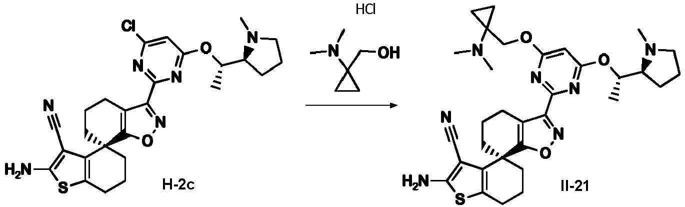

[1-(Dimethylamino)cyclopropyl]methanol hydrochloride (34.4 mg, 0.227 mmol, 1.30 equiv.) is dissolved in THF (2 mL) and cooled to 0-5 °C. Potassium tert-butoxide (1 N in THF, 366 µL, 0.366 mmol, 2.10 equiv.) is added dropwise and the reaction mixture is stirred 30 min at 0-5 °C. **H-2c** (100 mg, 0.174 mmol, 1.00 equiv.) is dissolved in THF (1.0 mL) and added dropwise at 0-5 °C. The reaction mixture is stirred 1 h at rt. HPLC shows approx. 50 % conversion, starting material and side products. The reaction mixture is quenched with water and concentrated under reduced pressure. The crude product is purified with RP chromatography yielding **II-21** (HPLC method: A, tret = 1.65 min; [M+H]+ = 604).

The following compounds **I** and **II** (Table 18) are available in an analogous manner. The crude products are purified by chromatography if necessary.

**Table 18**

| **#** | **structure** | **tᵣₑₜ [min]** | **[M+H]⁺** | **HPLC method** |
|---|---|---|---|---|
| **II-19** | 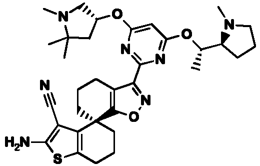 | 1.66 | 618 | A |
| **II-20** | 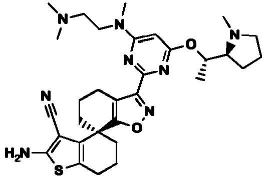 | 1.49 | 591 | A |
| **II-21** | 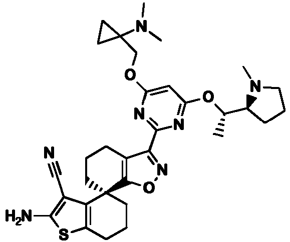 | 1.65 | 604 | A |
| **I-8** | 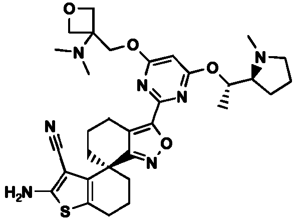 | 1.49 | 620 | A |
| **II-22** | 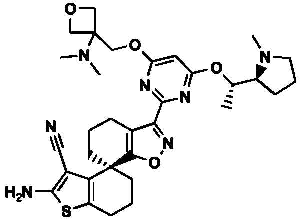 | 1.47 | 620 | A |
| **II-23** | 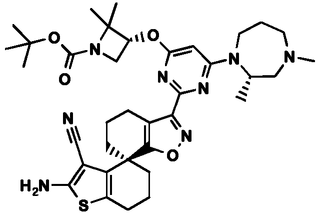 | 1.73 | 689 | A |
| **II-24** | 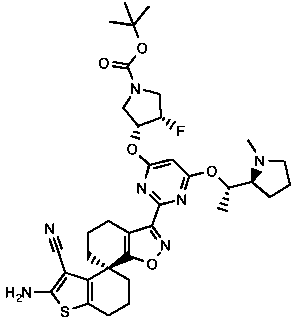 | 0.75 | 604 | E |
| **II-25** | 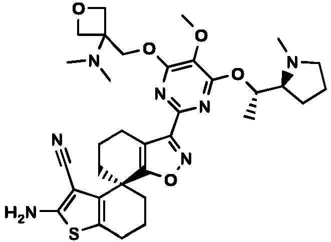 | 1.46 | 650 | A |

### Experimental procedure for the conversion of H-2 to II (method III)

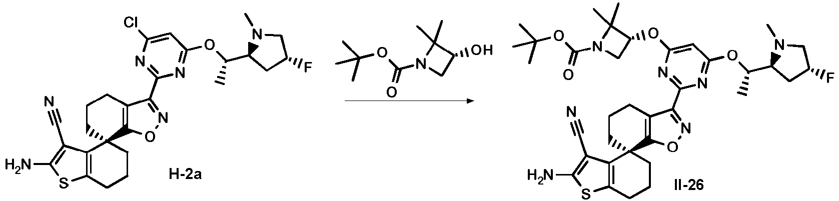

Tert-butyl-(3R)-3-hydroxy-2,2-dimethyl-azetidine-1-carboxylate (98.8 mg, 0.476 mmol, 1.10 equiv.) is dissolved in THF (3.0 mL). NaH (60 % in mineral oil, 36.3 mg, 0.909 mmol, 2.10 equiv.) is added and the mixture is stirred at rt for 10 min. **H-2a** (235 mg, 0.433 mmol; 1.0 equiv.) is dissolved in dry THF (5 mL) and added to the mixture. The mixture is stirred for 1 h at rt. The reaction mixture is quenched with aq. saturated NH₄Cl, DCM is added, and the organic layer is separated, dried, filtered, and evaporated. The crude product is purified with RP chromatography yielding **II-26.**

The following compounds **II** (Table 19) are available in an analogous manner. The crude products are purified by chromatography if necessary.

**Table 19**

| **#** | **structure** | **tᵣₑₜ [min]** | **[M+H]⁺** | **HPLC method** |
|---|---|---|---|---|
| **II-26** | 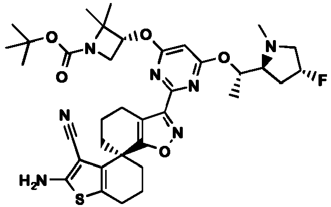 | 1.03 | 708 | E |
| **II-27** | 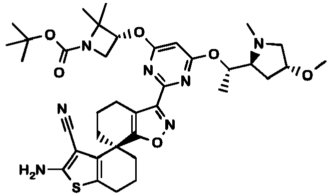 | 1.02 | 720 | E |

### Experimental procedure for the synthesis of II

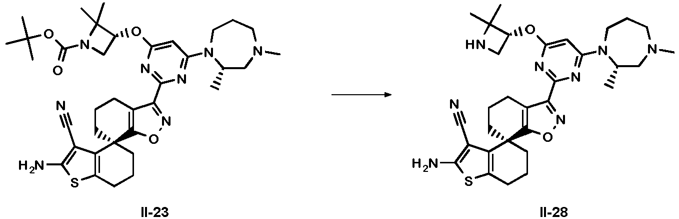

**II-23** (60.0 mg, 0.087 mmol, 1.0 equiv.) is dissolved in TFA (1 mL, 13.4 mmol, 154 equiv.) and the reaction is stirred at rt for 15 min. After complete conversion, the reaction mixture is concentrated under vacuum, the residue is diluted with DCM, washed with aq. saturated NaHCO₃, dried, filtered and concentrated. The crude product is purified with RP chromatography.

The following compounds **II** (Table 20) are available in an analogous manner. The crude products are purified by chromatography if necessary.

**Table 20**

| **#** | **structure** | **tᵣₑₜ [min]** | **[M+H]⁺** | **HPLC method** |
|---|---|---|---|---|
| **II-28** | 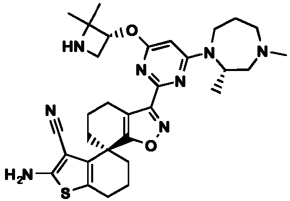 | 1.37 | 589 | A |
| **II-29** | 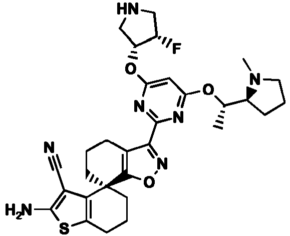 | 1.38 | 594 | A |
| **II-30** | 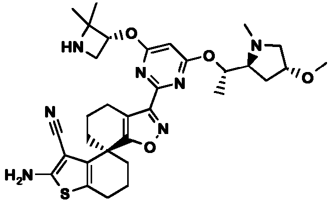 | 1.43 | 620 | A |
| **II-31** | 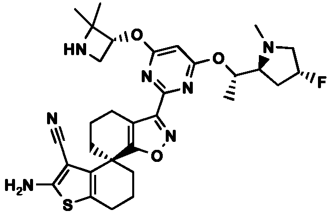 | 1.47 | 608 | A |

The following Examples describe the biological activity of the compounds according to the invention, without restricting the invention to these Examples.

### KRAS::SOS1 AlphaScreen Binding Assay

This assay can be used to examine the potency with which compounds according to the invention binding to (mutated) KRAS inhibit the protein-protein interaction between SOS1 and (mutated) KRAS e.g., KRAS WT, KRAS G12C, KRAS G12D, KRAS G12V, KRAS G13D. This inhibits the GEF functionality of SOS1 and locks the corresponding mutated KRAS protein in its inactive, GDP-bound state. Low IC₅₀ values in this assay setting are indicative of strong inhibition of protein-protein interaction between SOS1 and KRAS:

### Description of the Assay:

### These assays measure the inhibitory effect of compounds on KRAS mutant protein-protein interactions using the Alpha Screen technology by Perkin Elmer

The following (mutant) enzyme forms of KRAS and interacting proteins are used in these assays at the given concentrations:
KRAS (G12D) 1-169, N-terminal 6His-tag, C-terminal avi-tag (Xtal BioStructures, Inc.); final assay concentration 10 nM and SOS1 564-1049, N-terminal 229 GST-tag, TEV cleavage site (Viva Biotech Ltd); final assay concentration 5 nM;
KRAS (G12C) 1-169, N-terminal 6His-tag for purification, cleaved off, C-terminal avi-tag, biotinylated, mutations: C51S, C80L, C118S (in house); final assay concentration 7.5 nM and SOS1 564-1049, N-terminal 229 GST-tag, TEV cleavage site (Viva Biotech Ltd); final assay concentration 5 nM;
KRAS (G12V) 1-169, N-terminal 6His-tag for purification, cleaved off, C-terminal avi-tag, biotinylated, TEV cleavage site, mutation: C118S, GDP loaded (in house); final assay concentration 10nM and SOS1 564-1049, N-terminal 229 GST-tag, TEV cleavage site (Viva Biotech Ltd); final assay concentration 10 nM;
KRAS (G13D) 1-169, N-terminal 6His-tag for purification, cleaved off, C-terminal avi-tag, biotinylated, TEV cleavage site, mutation: C118S, GDP loaded (in house); final assay concentration 10 nM and SOS1 564-1049, N-terminal 229 GST-tag, TEV cleavage site (Viva Biotech Ltd); final assay concentration 10 nM;
KRAS (WT) 1-169, N-terminal 6His-tag for purification, cleaved off, C-terminal avi-tag, biotinylated, TEV cleavage site, mutation: C118S, GDP loaded (in house); final assay concentration 10nM and SOS1 564-1049, N-terminal 229 GST-tag, TEV cleavage site (Viva Biotech Ltd); final assay concentration 10 nM.

Test compounds dissolved in DMSO are dispensed onto assay plates (Proxiplate 384 PLUS, white, PerkinElmer; 6008289) using an Access Labcyte Workstation with the Labcyte Echo 55x. For the chosen highest assay concentration of 100 µM, 150 nL of compound solution are transferred from a 10 mM DMSO compound stock solution. A series of eleven fivefold dilutions per compound are transferred to the assay plate, compound dilutions are tested in duplicates. DMSO are added as backfill to a total volume of 150 nL.

The assays run on a fully automated robotic system in a darkened room below 100 Lux. To 150nl of compound dilution 10 µl of a mix including KRAS mutant protein, SOS1 (final assay concentrations see above) and GDP nucleotide (Sigma G7127; final assay concentration 10µM) in assay buffer (1 x PBS, 0.1% BSA, 0.05% Tween 20) are added into columns 1-24.

After 30 minutes incubation time 5 µl of Alpha Screen bead mix in assay buffer are added into columns 1-23. Bead mix consists of AlphaLISA Glutathione Acceptor Beads (PerkinElmer, Cat No AL109) and AlphaScreen Streptavidin Donor Beads (PerkinElmer Cat No 6760002) in assay buffer at a final assay concentration of 10 µg/ml each.

Plates are kept at room temperature in a darkened incubator. After an additional 60 minutes incubation time the signal is measured in a PerkinElmer Envision HTS Multilabel Reader using the AlphaScreen specs from PerkinElmer.

Each plate contains up to 16 wells of a negative control depending on the dilution procedure (platewise or serial) (DMSO instead of test compound; with KRAS mutant::SOS1 GDP mix and bead mix; column 23) and 16 wells of a positive control (DMSO instead of test compound; with KRAS mutant::SOS1 GDP mix w/o bead mix; column 24).

As internal control known inhibitors of KRAS mutant::SOS1 interaction can be measured on each compound plate.

IC₅₀ values are calculated and analyzed with Boehringer Ingelheim's MEGALAB IC50 application using a 4 parametric logistic model.

IC₅₀ values of example compounds disclosed herein determined using the above assay (see Table 21).

**Table 21**

| **Ex #** | **KRASG12D IC50 (nM)** | **KRASG12C IC50 (nM)** | **KRAS WT IC₅₀ (nM)** | **KRAS G13D IC₅₀ (nM)** | **KRAS G12V IC₅₀ (nM)** |
|---|---|---|---|---|---|
| I-1 | 2 | 1 | 4 | | 2 |
| I-2 | 1 | 1 | 4 | | 1 |
| I-3 | 1 | 1 | 2 | 10 | |
| I-4 | 4 | 3 | | | |
| I-5 | 12 | 5 | | | |
| I-6 | 4 | 3 | | | |
| I-7 | 6 | 5 | | | |
| I-8 | 2 | 2 | | 8 | 3 |
| II-1 | 1 | 1 | 3 | | |
| II-2 | 2 | 2 | | | |
| II-3 | 2 | 2 | | | |
| II-4 | 2 | 2 | | | |
| II-5 | 1 | 2 | | | |
| II-6 | 1 | 1 | | | |
| II-7 | 1 | 2 | | 10 | |
| II-8 | 1 | 2 | | | 10 |
| II-9 | 1 | 2 | 2 | 10 | 3 |
| II-10 | 2 | 2 | | | |
| II-11 | 1 | 2 | | | |
| II-12 | 6 | 4 | | | |
| II-13 | 2 | 2 | 7 | | 2 |
| II-14 | 3 | 3 | 10 | | 3 |
| II-15 | 3 | 3 | | | |
| II-16 | 3 | 2 | | | |
| II-17 | 2 | 2 | 8 | | 2 |
| II-18 | 2 | 2 | | | |
| II-19 | 1 | 4 | | | |
| II-20 | 2 | 10 | | | |
| II-21 | 1 | 3 | | | |
| II-22 | 1 | 2 | 2 | 6 | 2 |
| II-25 | 1 | 2 | 4 | 8 | 2 |
| II-28 | 1 | 6 | 10 | | |
| II-29 | 1 | 2 | 4 | | 2 |
| II-30 | 1 | 1 | 2 | | 2 |
| II-31 | 1 | 1 | 2 | | 2 |

### Ba/F3 cell model generation and proliferation assay

Ba/F3 cells are ordered from DSMZ (ACC300, Lot17) and grown in RPMI-1640 (ATCC 30-2001) + 10 % FCS + 10 ng/mL IL-3 at 37 °C in 5 % CO₂ atmosphere. Plasmids containing KRASG12 mutants(i.e. G12D, G12C, G12V) are obtained from GeneScript. To generate KRASG12-dependent Ba/F3 models, Ba/F3 cells are transduced with retroviruses containing vectors that harbor KRASG12 isoforms. Platinum-E cells (Cell Biolabs) are used for retrovirus packaging. Retrovirus is added to Ba/F3 cells. To ensure infection, 4 µg/mL polybrene is added and cells are spinfected. Infection efficiency is confirmed by measuring GFP-positive cells using a cell analyzer. Cells with an infection efficiency of 10 % to 20 % are further cultivated and puromycin selection with 1 µg/mL is initiated. As a control, parental Ba/F3 cells are used to show selection status. Selection is considered successful when parental Ba/F3 cells cultures died. To evaluate the transforming potential of KRASG12 mutations, the growth medium is no longer supplemented with IL-3. Ba/F3 cells harboring the empty vector are used as a control. Approximately ten days before conducting the experiments, puromycin is left out.

For proliferation assays, Ba/F3 cells are seeded into 384-well plates at 1.5 x 10³ cells / 60 µL in growth media (RPMI-1640 + 10 % FCS). Compounds are added using an Access Labcyte Workstation with a Labcyte Echo 550 or 555 accoustic dispenser. All treatments are performed in technical duplicates. Treated cells are incubated for 72 h at 37 °C with 5 % CO₂. AlamarBlue^{™}(ThermoFisher), a viability stain, is added and fluorescence measured in the PerkinElmer Envision HTS Multilabel Reader. The raw data are imported into and analyzed with the Boehringer Ingelheim proprietary software MegaLab (curve fitting based on the program PRISM, GraphPad Inc.).

IC₅₀ values of representative compounds according to the invention measured with this assay are presented in table 23.

**Table 23**

| **Ex #** | **KRAS G12D Ba/F3 IC₅₀ (nM)** | **KRAS G12C Ba/F3 IC₅₀ (nM)** | **KRAS G12V Ba/F3 IC₅₀ (nM)** |
|---|---|---|---|
| I-1 | 11 | 4 | 3 |
| I-2 | 6 | 4 | 2 |
| I-3 | 5 | 4 | 2 |
| I-4 | 41 | 83 | 46 |
| I-5 | 122 | 71 | 67 |
| I-6 | 47 | 58 | 14 |
| I-7 | 97 | 75 | 31 |
| I-8 | 11 | 51 | 85 |
| II-1 | 6 | 4 | 4 |
| II-2 | 20 | 5 | 4 |
| II-3 | 27 | 17 | 9 |
| II-4 | 31 | 12 | 6 |
| II-5 | 38 | 17 | 7 |
| II-6 | 16 | 11 | 7 |
| II-7 | 10 | 4 | 3 |
| II-8 | 388 | 592 | 869 |
| II-9 | 5 | 133 | 161 |
| II-10 | 81 | 61 | 43 |
| II-11 | 28 | 14 | 29 |
| II-12 | 65 | 44 | 11 |
| II-13 | 37 | 17 | 10 |
| II-14 | 59 | 15 | 7 |
| II-15 | 59 | 29 | 12 |
| II-16 | 43 | 24 | 11 |
| II-17 | 33 | 19 | 10 |
| II-18 | 46 | 37 | 11 |
| II-19 | 91 | 218 | 151 |
| II-20 | 304 | 376 | 303 |
| II-21 | 22 | 195 | 237 |
| II-22 | 6 | 22 | 35 |
| II-25 | 15 | 85 | 137 |
| II-28 | 130 | 2080 | 2040 |
| II-29 | 22 | 79 | 65 |
| II-30 | 5 | 219 | 297 |
| II-31 | 3 | 203 | 230 |

### Additional proliferation assays with mutant cancer cell lines

### • NCI-H358 CTG proliferation assay (120 h) (NSCLC, G12C)

NCI-H358 cells (ATCC No. CRL-5807) are dispensed into black 384-well plates, flat and clear bottom (Greiner, PNr. 781091) at a density of 200 cells per well in 60 µl RPMI-1640 ATCC-Formulation (Gibco # A10491) + 10 % FCS (fetal calf serum). Cells are incubated overnight at 37 °C in a humidified tissue culture incubator at 5 % CO₂. Compounds (10 mM stock in DMSO) are added at logarithmic dose series using the ECHO acoustic liquid handler system (Beckman Coulter), normalizing for added DMSO and including DMSO controls. For the T0 time point measurement, untreated cells are analyzed at the time of compound addition. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four parameter model.

### • NCI-H2122 CTG proliferation assay (120 h) (NSCLC, G12C)

The CTG assay is designed to measure quantitatively the proliferation of NCI-H2122 cells (ATCC CRL-5985), using the CellTiter Glow Assay Kit (Promega G7571). Cells are grown in RPMI medium (ATCC) supplemented with Fetal Calf Serum (Life Technologies, Gibco BRL, Cat. No. 10270-106). On "day 0" 200 NCI-H2122 cells are seeded in 60 µL RPMI ATCC+10 % FCS+ Penstrep in a black 384-well plate, flat and clear bottom (Greiner, PNr. 781091). Cells are then incubated in the plates at 37 °C in a CO₂ incubator overnight. On day 1, compounds (10 mM stock in DMSO)are added with the ECHO acoustic liquid handler system (Beckman Coulter), including DMSO controls. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four parameter model.

### • AsPC-1 CTG proliferation assay (120 h) (pancreatic cancer, G12D)

The CTG assay is designed to measure quantitatively the proliferation of AsPC-1 cells (ATCC CRL-5985), using the CellTiter Glow Assay Kit (Promega G7571). Cells are grown in RPMI medium (ATCC) supplemented with Fetal Calf Serum (Life Technologies, Gibco BRL, Cat. No. 10270-106). On "day 0" 2000 AsPC-1 cells are seeded in 60 µL RPMI ATCC+10 % FCS+ Penstrep in a 384-well plate, flat and clear bottom (Greiner, PNr. 781091). Cells are then incubated in the plates at 37 °C in a CO₂ incubator overnight. On day 1, compounds (10 mM stock in DMSO) are added with the ECHO acoustic liquid handler system (Beckman Coulter), including DMSO controls. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four-parameter model.

### • GP2D proliferation assay (120 h) (colorectal cancer, G12D)

GP2D cells (ATCC No. CRL-5807) are dispensed into white 384-well plates, flat and white bottom (Perkin Elmer, 6007680) at a density of 500 cells per well in 40 µl DMEM (Sigma, D6429) + 1x GlutaMAX (Gibco, 35050038) + 10 % FCS (fetal calf serum). Cells are incubated overnight at 37 °C in a humidified tissue culture incubator at 5 % CO₂. Compounds (10 mM stock in DMSO) are added at logarithmic dose series using the HP Digital Dispenser D300 (Tecan), including DMSO controls and normalizing for added DMSO. For the T0 time point measurement, untreated cells are analyzed at the time of compound addition. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four parameter model.

### • SAS CTG proliferation assay (120 h) (HNSCC, wt amplified)

SAS cells (JCRB0260) are dispensed into 384-well plates, flat and clear bottom (Greiner, PNr. 781091) at a density of 300 cells per well in 60 µL DMEM:F12 (Gibco 31330-038) + 10% Fetal Calf Serum (HyClone, PNr.: SH30084.03) and incubated at 37 °C in a CO₂ incubator overnight.

The next day, compounds (10 mM stock in DMSO) are added with the ECHO acoustic liquid handler system (Beckman Coulter), including DMSO controls. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four-parameter model.

### • MKN1 CTG proliferation assay (120 h) (gastric cancer, wt amplified)

MKN1 cells (JCRB0252) are dispensed into white 384-well plates, flat and white bottom (Perkin Elmer, 6007680) at a density of 500 cells per well in 40 µl RPMI (Gibco, PNr.: 21875034) + 10 % FCS (HyClone, PNr.: SH30084.03) (assay 1) or into black 384-well plates, flat and clear bottom (Greiner, PNr. 781091) at a density of 200 cells per well in 60 µl RPMI-1640 (Gibco # A10491) + 10% FCS (HyClone, PNr.: SH30084.03) + PenStrep (Gibco, PNr.15140-122) (assay 2). Cells are incubated overnight at 37 °C in a humidified tissue culture incubator at 5 % CO₂. Compounds (10 mM stock in DMSO) are added at logarithmic dose series using the HP Digital Dispenser D300 (Tecan) (assay 1) or the ECHO acoustic liquid handler system (Beckman Coulter) (assay 2), including DMSO controls and normalizing for added DMSO. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four-parameter model.

### • SK-CO-1 CTG proliferation assay (120 h) (CRC, G12V)

SK-CO-1 cells (ATCC HTB-39) are dispensed into 384-well plates, flat and clear bottom (Greiner, PNr. 781091) at a density of 500 cells per well in 60 µL EMEM (Sigma M5650) + 10% Fetal Calf Serum (HyClone, PNr.: SH30084.03) and incubated at 37 °C in a CO₂ incubator overnight. The next day, compounds (10 mM stock in DMSO) are added with the ECHO acoustic liquid handler system (Beckman Coulter), including DMSO controls. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four-parameter model.

### • LOVO CTG proliferation assay (120 h) (CRC, G13D)

LOVO cells (ATCC CCL-229) are dispensed into 384-well plates, flat and clear bottom (Greiner, PNr. 781091) at a density of 1000 cells per well in 60 µL DMEM (Sigma D6429) + 10% Fetal Calf Serum (HyClone, PNr.: SH30084.03) and incubated at 37 °C in a CO₂ incubator overnight. The next day, compounds (10 mM stock in DMSO) are added with the ECHO acoustic liquid handler system (Beckman Coulter), including DMSO controls. Plates are incubated for 120 h, and cell viability is measured using CellTiter-Glo luminescent cell viability reagent (Promega product code G7570). Viability (stated as percent of control) is defined as relative luminescence units RLU of each well divided by the RLU of cells in DMSO controls. IC₅₀ values are determined from viability measurements by non-linear regression using a four-parameter model.

IC₅₀ values of representative compounds according to the invention measured with these assays in the indicated cell lines are presented in table 22 and 23.

**Table 22**

| **Ex #** | **H358 IC₅₀ (nM)** | **H2122 IC₅₀ (nM)** | **ASPC-1 IC₅₀ (nM)** | **GP2D IC₅₀ (nM)** | **SAS IC₅₀ (nM)** |
|---|---|---|---|---|---|
| I-1 | | 1468 | 2503 | | 19 |
| I-2 | | 740 | 1311 | | 15 |
| I-3 | | 2278 | 682 | 12 | 14 |
| I-4 | 2276 | 2390 | 9466 | | 719 |
| I-6 | 1358 | 1121 | 3355 | | 81 |
| I-7 | 5624 | 3607 | 10263 | | 221 |
| I-8 | 76 | 1262 | 272 | 11 | 51 |
| II-1 | 178 | 715 | 699 | 10 | 20 |
| II-2 | 115 | 77 | 1836 | | 44 |
| II-3 | 275 | 275 | 1977 | | 67 |
| II-4 | 193 | 817 | 2754 | 178 | 44 |
| II-5 | 339 | 861 | 2550 | | 67 |
| II-6 | 165 | 1650 | 1762 | | 40 |
| II-7 | | 997 | 3005 | | 1388 |
| II-8 | 1177 | 1339 | 1725 | | 2598 |
| II-9 | 69 | 1247 | 49 | 0 | 179 |
| II-10 | | 2419 | 1937 | | 795 |
| II-11 | | 2130 | 2266 | | 21 |
| II-12 | 819 | 1741 | 2920 | | 594 |
| II-13 | 146 | 532 | 966 | | 46 |
| II-14 | 558 | 1419 | 3177 | | 285 |
| II-15 | 123 | 847 | 3551 | | 87 |
| II-16 | 440 | | | | |
| II-17 | 775 | 1648 | 2720 | 363 | 104 |
| II-18 | 430 | 822 | 3012 | | 171 |
| II-19 | 3270 | 1460 | 1416 | | 297 |
| II-20 | | 4696 | 3581 | | 327 |
| II-21 | | 2205 | 277 | 8 | 577 |
| II-22 | | 1709 | 298 | 5 | 84 |
| II-25 | | 389 | 571 | 17 | 55 |
| II-28 | 952 | 9741 | 443 | 20 | 4452 |
| II-29 | 36 | 731 | 430 | 9 | 1050 |
| II-30 | 85 | 1225 | 36 | 0 | 267 |
| II-31 | 119 | 1212 | 25 | 1 | 402 |

**Table 23**

| **Ex #** | **MKN1 (assay 1) IC₅₀ (nM)** | **MKN1 (assay 2) IC₅₀ (nM)** | **SK-CO-1 IC₅₀ (nM)** | **LOVO IC₅₀ (nM)** |
|---|---|---|---|---|
| I-1 | | | 24 | 54 |
| I-2 | | 109 | 23 | 32 |
| I-3 | | | 29 | 35 |
| I-4 | | | 762 | 575 |
| I-6 | | | 273 | 595 |
| I-7 | | | 796 | 3237 |
| I-8 | | 108 | 401 | 27 |
| II-1 | | 43 | 18 | 433 |
| II-2 | | | 26 | 53 |
| II-3 | | | 84 | 102 |
| II-4 | | | 28 | 93 |
| II-5 | | | 23 | 117 |
| II-6 | | 90 | 88 | 781 |
| II-7 | | | 21 | 15 |
| II-8 | | | 774 | 501 |
| II-9 | | 129 | 171 | 99 |
| II-10 | | | 1201 | 4083 |
| II-11 | | | 46 | 568 |
| II-12 | | | 308 | 1132 |
| II-13 | | | 69 | 500 |
| II-14 | | | 115 | 960 |
| II-15 | | | 91 | 708 |
| II-17 | | | 60 | 297 |
| II-18 | | | 133 | 1117 |
| II-19 | | | 933 | 344 |
| II-20 | | | 900 | 139 |
| II-21 | | | 977 | 186 |
| II-22 | 33 | | 354 | 39 |
| II-25 | | | 326 | 35 |
| II-28 | | | 2590 | 3383 |
| II-29 | | | 139 | 216 |
| II-30 | 223 | | 260 | 130 |
| .II-31 | 357 | | 394 | 202 |

### ERK Phosphorylation Assay

ERK phosphorylation assays are used to examine the potency with which compounds inhibit the KRAS G12C-mediated signal transduction in a KRAS G12C mutant human cancer cell line *in vitro.* This demonstrates the molecular mode of action of compounds according to the invention by interfering with the RAS G12C protein signal transduction cascade. Low IC₅₀ values in this assay setting are indicative of high potency of the compounds according to the invention. It is observed that compounds according to the invention demonstrate an inhibitory effect on ERK phosphorylation in a KRAS G12C mutant human cancer cell line, thus confirming the molecular mode of action of the compounds on RAS G12C protein signal transduction.

ERK phosphorylation assays are performed using the following human cell lines:
NCI-H358 (ATCC (ATCC CRL-5807): human lung cancer with a KRAS G12C mutation (→ assay 1) and NCI-H358_Cas9_SOS2, *i.e.* the same cell line, in which SOS2 is knocked (→ assay 2). Vectors containing the designed DNA sequences for the production of gRNA for SOS2 protein knock-out are obtained from Sigma-Aldrich. To generate the NCI-H358 SOS2 knock-out cell line, NCI-H358 cells expressing Cas9 endonuclease are transfected with XtremeGene9 reagent and the correspondent plasmids. Transfection efficiency is confirmed by measuring GFP-positive cells using a cell analyzer. GFP positive cells are collected and further expanded. These GFP-positive cell pools are single-cell diluted and SOS2 knock-out clones are identified via Western-blot and genomic DNA sequencing analysis.

### Materials used for the assay:

RPMI-1640 Medium (ATCC^{®} 30-2001^{™})
Fetal Bovine Serum (FBS) from HyClone (SH30071.03)
Non-essential amino acids from Thermo Fischer Scientific (11140035)
Pyruvate from Thermo Fischer Scientific (11360039)
Glutamax from Thermo Fischer Scientific (35050061)
384 plates from Greiner Bio-One (781182)
Proxiplate^{™} 384 from PerkinElmer Inc. (6008280)
AlphaLISA SureFire Ultra p-ERK1/2 (Thr202/Tyr204) Assay Kit (ALSU-PERK-A500)
EGF from Sigma (E4127)
Acceptor Mix: Protein A Acceptor Beads from PerkinElmer (6760137M)
Donor Mix: AlphaScreen Streptavidin-coated Donor Beads from PerkinElmer (6760002)
Trametinib
Staurosporine from Sigma Aldrich (S6942)

### Assay setup:

Cells are seeded at 40,000 cells per well in /60 µL of RPMI with 10 % FBS, non-essential amino acids, pyruvate and glutamax in Greiner TC 384 plates. The cells are incubated for 1 h at room temperature and then incubated overnight in an incubator at 37 °C and 5 % CO₂ in a humidified atmosphere. 60 nL compound solution (10 mM DMSO stock solution) is then added using a Labcyte Echo 550 device. After a 1 h incubation in the aforementioned incubator the medium is removed after centrifugation and the cells lysed by addition of 20 µL of 1.6-fold lysis buffer from the AlphaLISA SureFire Ultra pERK1/2 (Thr202/Tyr204) Assay Kit with added protease inhibitors, 100 nM trametinib + 100 nM staurosporine. After 20 min of incubation at room temperature with shaking, 6 µL of each lysate sample is transferred to a 384-well Proxiplate and analyzed for pERK (Thr202/Tyr204) with the AlphaLISA SureFire Ultra pERK1/2 (Thr202/Tyr204) Assay Kit. 3 µL Acceptor Mix and 3 µL Donor Mix are added under subdued light and incubated for 2 h at room temperature in the dark, before the signal is measured on a PerkinElmer Envision HTS Multilabel Reader. The raw data are imported into and analyzed with the Boehringer Ingelheim proprietary software MegaLab (curve fitting based on the program PRISM, GraphPad Inc.).

Analogously the described assay (pERK reduction; SureFire) can be performed on additional cell lines, carrying various KRAS mutations or KRAS wildtype, allowing the measurement and determination of the activity of compounds on various additional KRAS allels in a cellular background.

### Metabolic (microsomal) stability assay

The metabolic degradation of the test compound is assayed at 37 °C with pooled liver microsomes (mouse (MLM), rat (RLM) or human (HLM)). The final incubation volume of 48 µL per time point contains TRIS buffer (pH 7.5; 0.1 M), magnesium chloride (6.5 mM), microsomal protein (0.5 mg/mL for mouse/rat, 1 mg/mL for human specimens) and the test compound at a final concentration of 1 µM. Following a short preincubation period at 37 °C, the reactions are initiated by addition of 12 µL beta-nicotinamide adenine dinucleotide phosphate, reduced form (NADPH, 10 mM) and terminated by transfering an aliquot into solvent after different time points (0, 5, 15, 30, 60 min). Additionally, the NADPH-independent degradation is monitored in incubations without NADPH, terminated at the last time point by addition of acetonitrile. The quenched incubations are pelleted by centrifugation (4,000 rpm, 15 min). An aliquot of the supernatant is assayed by LC-MS/MS to quantify the concentration of parent compound in the individual samples.

*In vitro* intrinsic clearance (CL_{int, *in vitro*}) is calculated from the time course of the disappearance of the test drug during the microsomal incubation. Each plot is fitted to the first-order elimination rate constant as *C*(*t*) = *C*₀*exp(-*ke***t*), where *C*(*t*) and *C*₀ are the concentration of unchanged test drug at incubation time *t* and that at preincubation and *ke* is the disappearance rate constant of the unchanged drug. Subsequently, CL_{int, *in vitro*} (µL min⁻¹ · amount protein) values are converted to predicted CL_{int,}*_{in vivo}* (mL min⁻¹ ·kg⁻¹) from incubation parameters according to the equation CL_{int,} *_{in vivo} =* CL_{int,} *_{in vitro}* x (incubation volume (ml) / amount protein (mg)) x (amount protein (mg) / g liver tissue) x (liver weight / body wt.). For better across species comparison the predicted clearance is expressed as percent of the liver blood flow [% QH] (mL min⁻¹ ·kg⁻¹) in the individual species. In general, high stability (corresponding to low % QH) of the compounds across species is desired.

Table 24 shows metabolic stability data obtained with the disclosed assay in HLM for a selection of compounds **(I)** according to the invention.

**Table 24**

| **Ex #** | **HLM QH [%]** |
|---|---|
| I-1 | 44 |
| I-2 | 70 |
| I-3 | 69 |
| I-4 | 26 |
| I-5 | <24 |
| I-6 | <24 |
| I-7 | 38 |
| I-8 | 30 |
| II-1 | <24 |
| II-2 | 46 |
| II-3 | 70 |
| II-4 | 59 |
| II-5 | 31 |
| II-6 | 40 |
| II-7 | 72 |
| II-8 | <24 |
| II-9 | <24 |
| II-10 | <24 |

| **Ex #** | **HLM QH [%]** |
|---|---|
| II-11 | 36 |
| II-12 | <24 |
| II-13 | <24 |
| II-14 | <24 |
| II-15 | <24 |
| II-16 | 42 |
| II-17 | <24 |
| II-18 | 41 |
| II-19 | <24 |
| II-20 | <24 |
| II-21 | 48 |
| II-22 | 53 |
| II-25 | 46 |
| II-28 | 26 |
| II-29 | 28 |
| II-30 | 37 |
| II-31 | 37 |

### Plasma protein binding assay (PPB)

Binding of test compounds to plasma was determined using equilibrium dialysis (ED) and quantitative mass spectrometry interfaced with liquid chromatography (LC-MS). In brief, ED was performed with dialysis devices consisting of two chambers separated by a semipermeable membrane with a molecular weight cut-off of 5-10 kg/mol. One chamber was filled with 10 % FCS in PBS containing 1-10 µmol/L test compound and the other chamber was filled with phosphate-buffer saline (PBS) with or without dextran. The dialysis chamber was incubated for 3-5 hours at 37°C. After incubation, protein was precipitated from aliquots of each chamber and the concentration of test compound in the supernatant of the plasmacontaining compartment (cₛₑᵣᵤₘ) and of the buffer-containing compartment (c_{buffer}) was determined by LC-MS. The fraction of unbound test compound (not bound to plasma) (fᵤ) was calculated according to the following equation: ${f}_{u} \left[%\right] = \frac{{c}_{buffer}}{{c}_{plasma}} \times 100$

Table 25 shows metabolic stability data obtained with the disclosed assay for a selection of compounds **(I)** according to the invention.

**Table 25**

| **Ex #** | **PPB 10% FCS (%fu)** |
|---|---|
| I-1 | 25 |
| I-2 | 31 |
| I-3 | 57 |
| I-4 | 60 |
| I-5 | 45 |
| I-6 | 31 |
| I-7 | 61 |
| I-8 | 45 |
| II-1 | 19 |
| II-2 | 7 |
| II-3 | 12 |
| II-4 | 54 |
| II-5 | 50 |
| II-6 | 72 |
| II-7 | 28 |
| II-8 | 59 |
| II-9 | 52 |
| II-10 | 8 |
| II-11 | 62 |
| II-12 | 7 |
| II-13 | 20 |
| II-14 | 10 |
| II-15 | 50 |
| II-16 | 37 |
| II-17 | 28 |
| II-18 | 61 |
| II-19 | 12 |
| II-20 | 80 |
| II-21 | 72 |
| II-22 | 46 |
| II-25 | 48 |
| II-28 | 59 |
| II-29 | 52 |
| II-30 | 30 |
| II-31 | 31 |

### Mechanism based inhibition of CYP3A4 assay (MBI 3A4):

The time dependent inhibition towards CYP3A4 is assayed in human liver microsomes (0.02 mg/mL) with midazolam (15 µM) as a substrate. The test compounds and water control (wells w/o test compound) are preincubated in presence of NADPH (1mM) with human liver microsomes (0.2 mg/mL) at a concentration of 25 uM for 0 min and 30 min. After preincubation, the incubate is diluted 1:10 and the substrate midazolam is added for the main incubation (15 min). The main incubation is quenched with acetonitrile and the formation of hydroxy-midazolam is quantified via LC/MS-MS. The formation of hydroxy-midazolam from the 30 min preincubation relative to the formation from the 0 min preincubation is used as a readout. Values of less than 100 % mean that the substrate midazolam is metabolized to a lower extent upon 30 min preincubation compared to 0 min preincubation. In general low effects upon 30 min preincubation are desired (corresponding to values close to 100 %/ not different to the values determined with water control).

Table 26 shows data obtained with the disclosed assay for a selection of compounds **(I)** according to the invention.

**Table 26**

| **Ex #** | **MBI 3A4 [%]** |
|---|---|
| I-5 | 61 |
| I-8 | 77 |
| II-2 | 40 |
| II-4 | 58 |
| II-5 | 69 |
| II-6 | 61 |
| II-9 | 72 |
| II-13 | 52 |
| II-14 | 67 |
| II-15 | 64 |
| II-16 | 48 |
| II-17 | 75 |
| II-18 | 64 |
| II-21 | 76 |
| II-22 | 78 |
| II-25 | 69 |
| II-28 | 65 |

### Solubility measurement (DMSO solution precipitation method)

A 10 mM DMSO stock solution of a test compound is used to determine its aqueous solubility. The DMSO solution is diluted with an aqueous medium (Mcllvaine buffer with pH=4.5 or 6.8) to a final concentration of 250 µM. After 24 h of shaking at ambient temperature a potentially formed precipitate is removed by filtration. The concentration of the test compound in the filtrate is determined by LC-UV methods by calibrating the signal to the signal of a reference solution with complete dissolution of the test compound in acetonitrile/water (1:1) with known concentration.

Table 27 shows data obtained with the disclosed assay for a selection of compounds (I) according to the invention.

**Table 27**

| **Ex #** | **Solubility [µg/ml] pH 4,5** | **Solubility [µg/ml] pH 6,8** |
|---|---|---|
| I-1 | 95 | <1 |
| I-2 | >125 | 89 |
| I-3 | >128 | >121 |
| I-4 | >135 | 107 |
| I-5 | 111 | 109 |
| I-6 | 23 | 2 |
| I-7 | >146 | 70 |
| I-8 | >143 | >125 |
| II-1 | >137 | >124 |
| II-2 | >109 | <1 |
| II-3 | >138 | 42 |
| II-4 | 80 | <1 |
| II-5 | >140 | 108 |
| II-6 | >121 | >119 |
| II-7 | >118 | 90 |
| II-8 | >145 | >146 |
| II-10 | >107 | 95 |
| II-12 | 2 | <1 |
| II-13 | >157 | 44 |
| II-14 | 67 | 11 |
| II-16 | 41 | <1 |
| II-17 | 67 | <1 |
| II-19 | >161 | >164 |
| II-20 | >144 | >142 |
| II-21 | >143 | >126 |
| II-22 | >168 | >157 |
| II-25 | >163 | >152 |
| II-28 | >151 | >146 |
| II-30 | >171 | >165 |
| II-31 | >157 | >143 |

### Caco-2 assay

The assay provides information on the potential of a compound to pass the cell membrane, on the extent of oral absorption as well as on whether the compound is actively transported by uptake and/or efflux transporters. Permeability measurements across polarized, confluent Caco-2 cell monolayers grown on permeable filter supports (Corning, catalog #3391) are used. 10 µM test compound solution in assay buffer (128.13 mM NaCl, 5.36 mM KCI, 1 mM MgSO₄, 1.8 mM CaCl₂, 4.17 mM NaHCO₃, 1.19 mM Na₂HPO₄, 0.41 mM NaH₂PO₄, 15 mM 2-[4-(2-hydroxyethyl)piperazin-1-yl]ethanesulfonic acid (HEPES), 20 mM glucose, pH 7.4) was added to the donor compartment of the cell chamber containing a monolayer of Caco-2 cells in between the donor and the receiver compartment. The receiver and donor compartments contain 0.25 % bovine serum albumine (BSA) in assay buffer. Passive diffusion and/or active transport of compounds across the monolayer is measured in both apical to basolateral (a-b) and basolateral to apical (b-a) direction. a-b permeability (PappAB) represents drug absorption from the intestine into the blood and b-a permeability (PappBA) drug secretion from the blood back into the intestine via both passive permeability as well as active transport mechanisms mediated by efflux and uptake transporters that are expressed on the Caco-2 cells. After a pre-incubation of 25-30 min at 37 °C, at predefined time points (0, 30, 60 and 90 min), samples were taken from the receiver and donor compartment, respectively. Concentrations of test compounds in samples were measured by HPLC/MS/MS, samples from the donor compartment were diluted 1:50 (v:v) with assay buffer, samples from receiver compartment were measured without dilution.

Apparent permeabilities in a-b (PappAB) and b-a (PappBA) directions are calculated according to the formula: $Papp \left[cm/s\right] = \frac{1}{A ⋅ {C}_{don}} \cdot \frac{Vrec ⋅ ΔCrec}{Δt}$
Vrec [mL] : buffer volume in receiver compartment
Cdon [µmol/mL] : concentration of test compound in donor compartment at t = 0
ΔCrec: difference between concentrations of test compound in receiver compartment at start and end of incubation time
Δt: Incubation time
Vrec · ΔCrec / Δt [µmol/min]: Amount of compound transferred to receiver compartment per time
A [cm²]: filter surface

Caco-2 efflux ratios (ER) are calculated as the ratio of PappBA / PappAB.

Table 28 shows data obtained with the disclosed assay for a selection of compounds **(I)** according to the invention.

**Table 28**

| **Ex #** | **Caco ER** | **Caco PappAB (x 10⁻⁶ cm/sec)** |
|---|---|---|
| I-3 | 1.3 | 18.0 |
| I-5 | 7.7 | 4.3 |
| I-6 | 2.5 | |
| II-1 | 1.5 | 17.0 |
| II-2 | 0.5 | |
| II-4 | 3.7 | 8.6 |
| II-5 | 1.4 | 14.0 |
| II-6 | 20.8 | 1.2 |
| II-9 | 132.5 | 0.2 |
| II-10 | 3.0 | 7.6 |
| II-13 | 3.1 | 5.2 |
| II-15 | 14.5 | 2.0 |
| II-16 | 3.1 | |
| II-17 | 5.0 | 3.9 |
| II-18 | 17.9 | 1.4 |
| II-20 | 38.0 | 0.8 |
| II-21 | 1.4 | |
| II-22 | 15.5 | 2.6 |
| II-25 | 13.6 | 2.5 |
| II-29 | 25.5 | 0.9 |
| II-30 | 125.0 | 0.2 |
| II-31 | 50.7 | 0.8 |

The formulation examples which follow illustrate the present invention without restricting its scope:

### Examples of pharmaceutical formulations

A)

| Tablets | per tablet |
|---|---|
| active substance according to formula **(I)** | 100 mg |
| lactose | 140 mg |
| corn starch | 240 mg |
| polyvinylpyrrolidone | 15 mg |
| magnesium stearate | 5 mg |
| | 500 mg |

The finely ground active substance, lactose and some of the corn starch are mixed together. The mixture is screened, then moistened with a solution of polyvinylpyrrolidone in water, kneaded, wet-granulated and dried. The granules, the remaining corn starch and the magnesium stearate are screened and mixed together. The mixture is compressed to produce tablets of suitable shape and size.
B)

| Tablets | per tablet |
|---|---|
| active substance according to formula **(I)** | 80 mg |
| lactose | 55 mg |
| corn starch | 190 mg |
| microcrystalline cellulose | 35 mg |
| polyvinylpyrrolidone | 15 mg |
| sodiumcarboxymethyl starch | 23 mg |
| magnesium stearate | 2 mg |
| | 400 mg |

The finely ground active substance, some of the corn starch, lactose, microcrystalline cellulose and polyvinylpyrrolidone are mixed together, the mixture is screened and worked with the remaining corn starch and water to form a granulate which is dried and screened. The sodiumcarboxymethyl starch and the magnesium stearate are added and mixed in and the mixture is compressed to form tablets of a suitable size.
C)

| Tablets | per tablet |
|---|---|
| active substance according to formula **(I)** | 25 mg |
| lactose | 50 mg |
| microcrystalline cellulose | 24 mg |
| magnesium stearate | 1 mg |
| | 100 mg |

The active substance, lactose and cellulose are mixed together. The mixture is screened, then either moistened with water, kneaded, wet-granulated and dried or dry-granulated or directly final blend with the magnesium stearate and compressed to tablets of suitable shape and size. When wet-granulated, additional lactose or cellulose and magnesium stearate is added and the mixture is compressed to produce tablets of suitable shape and size.
D) Ampoule solution

| | |
|---|---|
| active substance according to formulae **(I)** | 50 mg |
| sodium chloride | 50 mg |
| water for inj. | 5 mL |

The active substance is dissolved in water at its own pH or optionally at pH 5.5 to 6.5 and sodium chloride is added to make it isotonic. The solution obtained is filtered free from pyrogens and the filtrate is transferred under aseptic conditions into ampoules which are then sterilised and sealed by fusion. The ampoules contain 5 mg, 25 mg and 50 mg of active substance.

## Claims

1. A compound of the formula **(la)** or a salt thereof 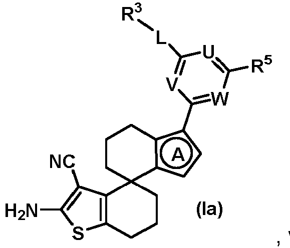 wherein
**L** is selected from -O-, -S- and -N(**R**⁷)-,
**R⁷** is hydrogen or C₁₋₆alkyl;
**R³** is selected from the group consisting of C₁₋₆alkyl, C₁₋₆alkoxy, 5-10 membered heteroaryl and 3-11 membered heterocyclyl, wherein the C₁₋₆alkyl, 5-10 membered heteroaryl, C₁₋₆alkoxy and 3-11 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with -N(C₁₋₄alkyl)₂;
**W** is nitrogen (-N=);
**V** is nitrogen (-N=);
**U** is -C(**R**¹¹)=;
**R¹¹** is selected from hydrogen, halogen and C₁₋₄alkoxy;
ring **A** is a ring selected from the group consisting 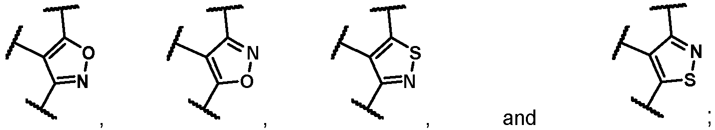
**R⁵** is selected from the group consisting of

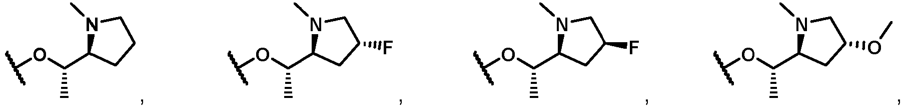

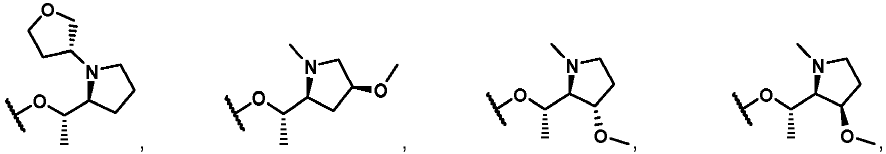

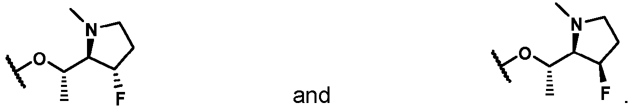

2. A compound of the formula **(Ib)** or a salt thereof 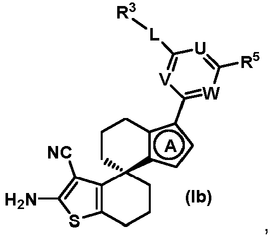 wherein
**A, V, U, W, L, R³** and **R⁵** are defined as in claim 1.

3. The compound or salt according to any one of claim 1 and 2, wherein
**R³** is selected from the group consisting of C₁₋₆alkyl, C₁₋₆alkoxy, 5-6 membered heteroaryl and 4-5 membered heterocyclyl, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

4. The compound or salt according to any one of claim 1 to 3, wherein
**R³** is selected from the group consisting of C₁₋₆alkyl, C₁₋₆alkoxy, 5-6 membered heteroaryl and 4-5 membered heterocyclyl, each of which independently contains one or two nitrogen or one oxygen heteroatom, wherein the C₁₋₆alkyl, 5-6 membered heteroaryl, C₁₋₆alkoxy and 4-5 membered heterocyclyl are all optionally and independently substituted with one or more, identical or different halogen, C₁₋₆alkyl, C₁₋₆alkoxy, -OH, -NH₂, -NH(C₁₋₄alkyl), -N(C₁₋₄alkyl)₂, -C(O)O-C₁₋₆alkyl, C₃₋₅cycloalkyl or 3-11 membered heterocyclyl optionally substituted with - N(C₁₋₄alkyl)₂.

5. The compound or salt according to any one of claim 1 to 4, wherein
**R³** is -C₁₋₄alkyl substituted with a 4-7 membered heterocyclyl or a C₃₋₅cycloalkyl, wherein the 4-7 membered heterocyclyl is optionally further substituted with -N(C₁₋₄alkyl)₂.

6. The compound or salt according to any one of claim 1 to 5, wherein
**R³** is selected from the group consisting of C₁₋₆alkyl, -CH(CH₃)CH₂-O-CH₃, -(CH₂)₂-O-CH₃, -(CH₂)₂-OH and -(CH₂)₂-N-(CH₃)₂,
or
**R³** is a ring selected from the group consisting of 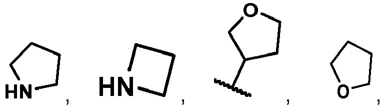 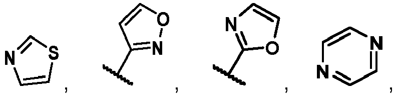 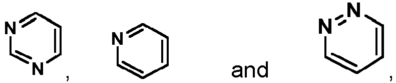 wherein each of these rings is optionally and independently substituted with one or more, identical or different halogen, C₁₋₆alkyl, -OH, -NH₂, -NH(C₁₋₄alkyl), -N(C₁₋₄alkyl)₂, C₃₋₅cycloalkyl or 3-11 membered heterocyclyl.

7. The compound or salt according to any one of claim 1 to 6, wherein
**R³** is selected from the group consisting of C₁₋₆alkyl, -CH(CH₃)CH₂-O-CH₃, -(CH₂)₂-O-CH₃, -(CH₂)₂-OH, -(CH₂)₂-N-(CH₃)₂,

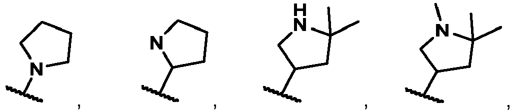

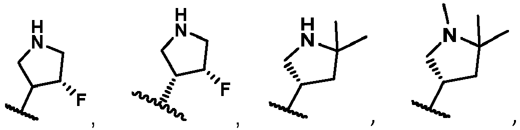

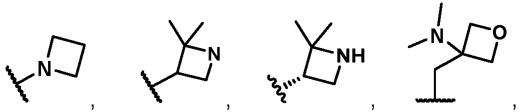

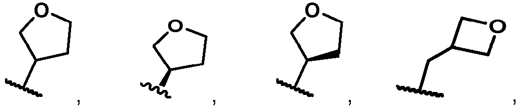

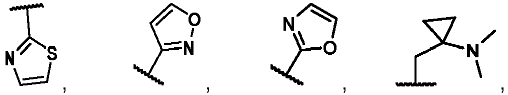

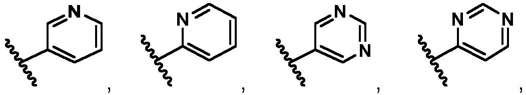

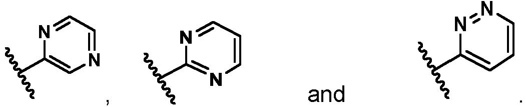

8. The compound or its pharmaceutically acceptable salt according to any one of claim 1 to 7 being selected from the group consisting of: 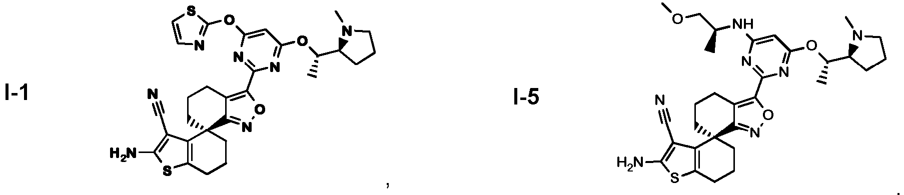 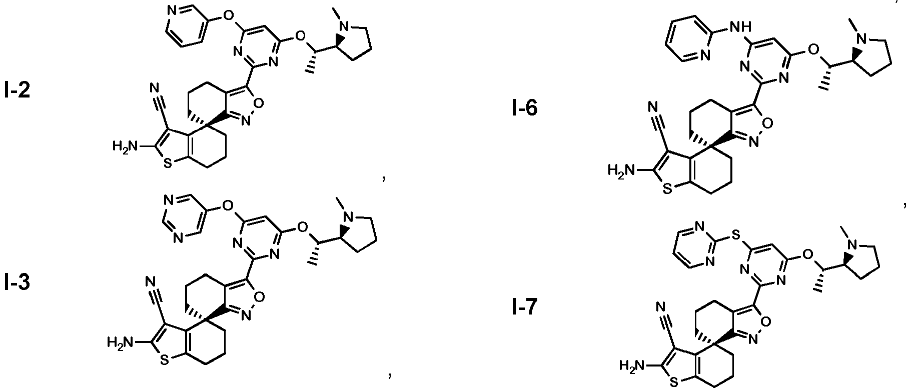 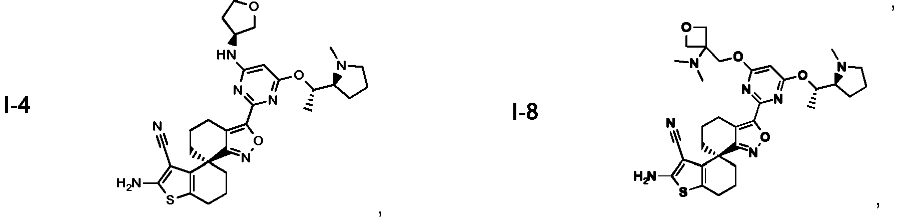 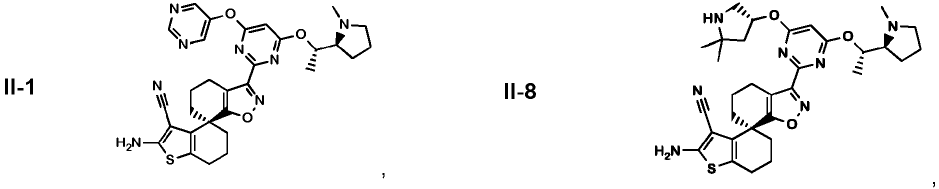 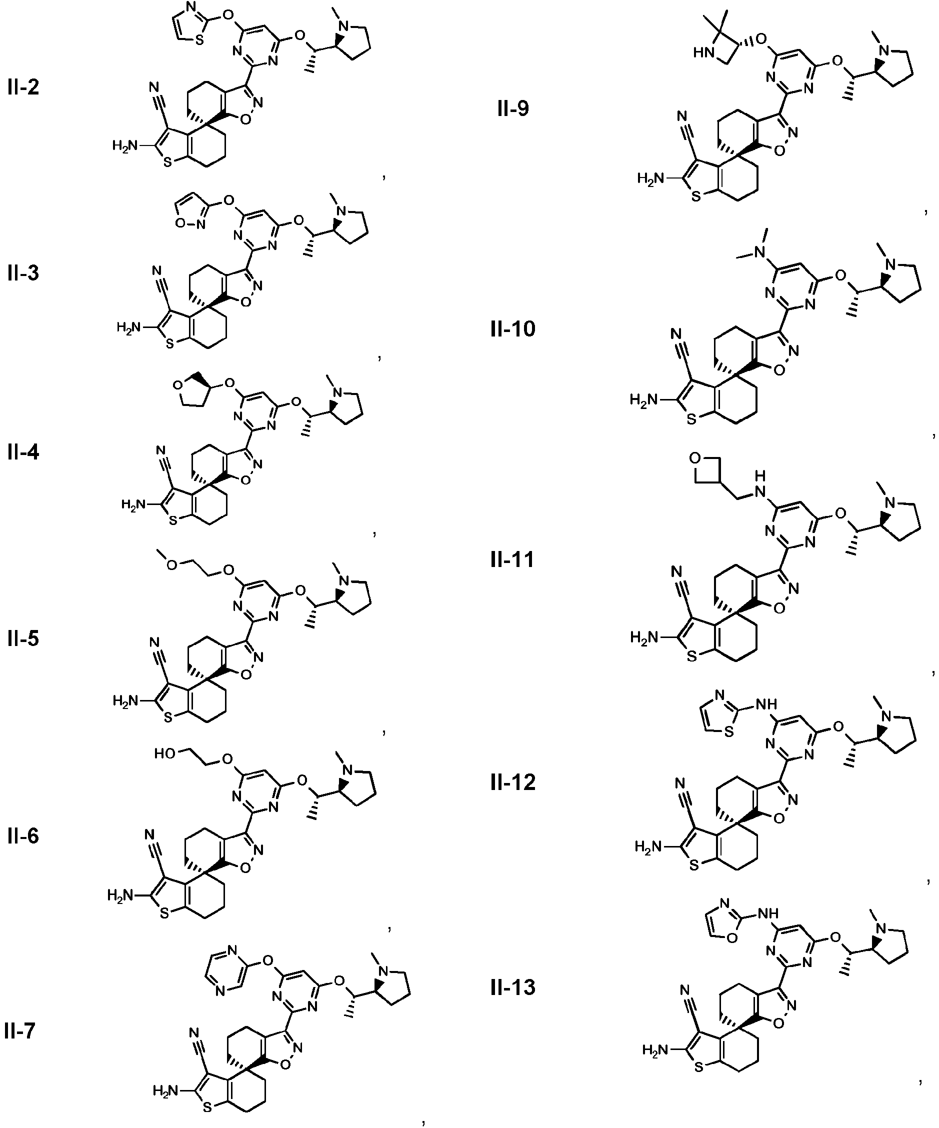 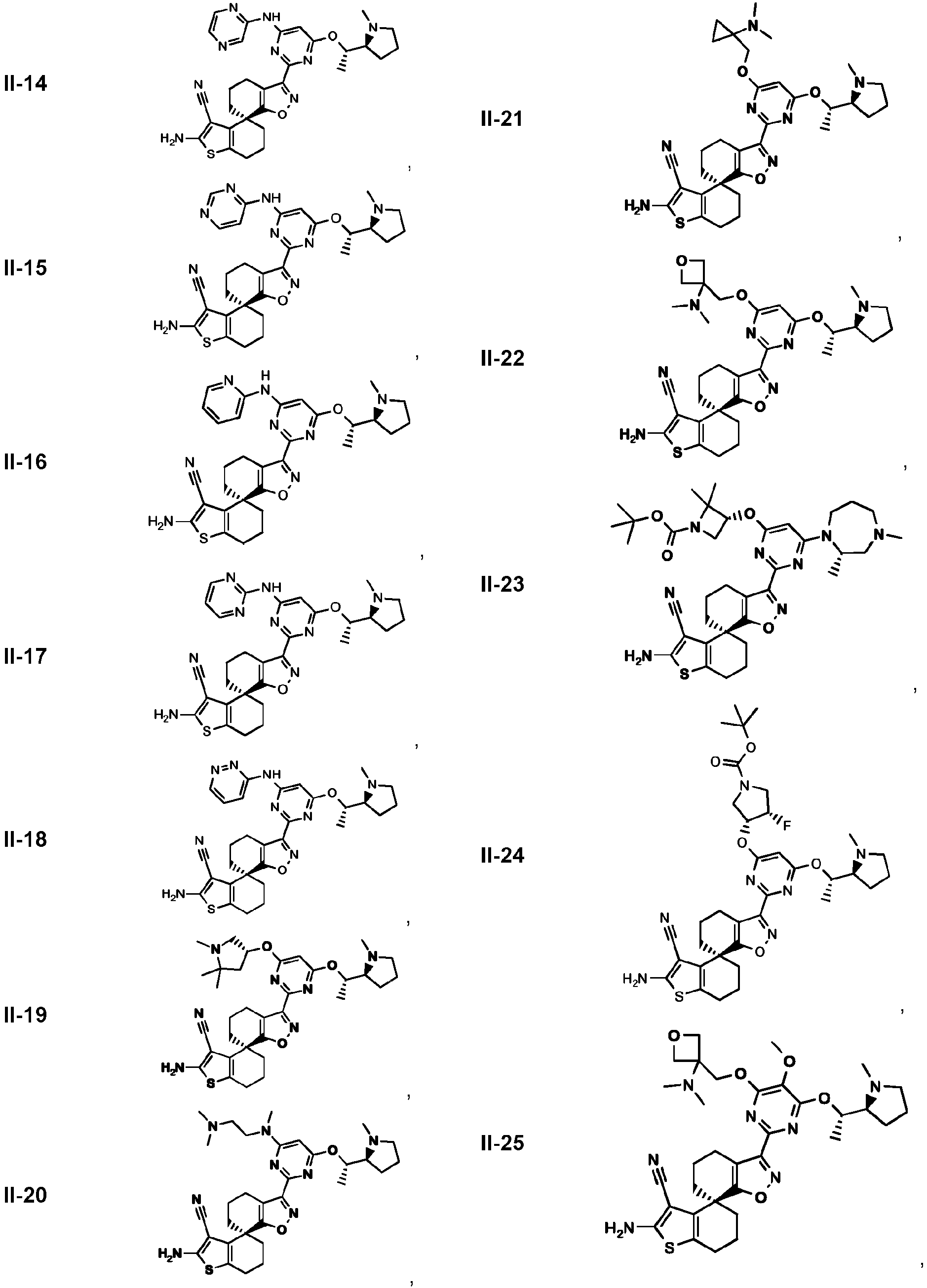 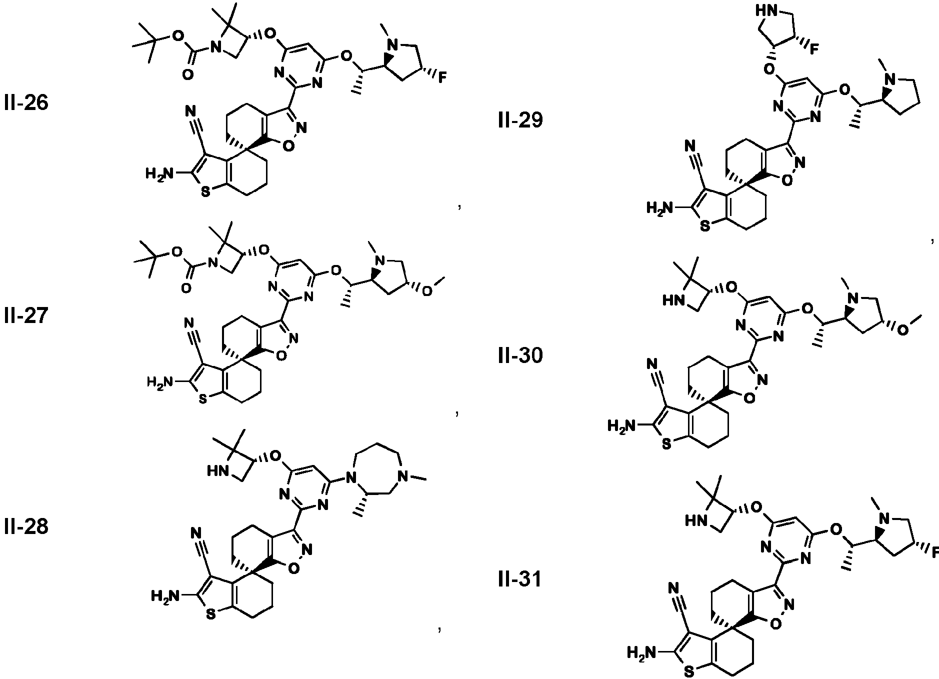 and

9. A compound according to any one of claim 1 to 8 - or a pharmaceutically acceptable salt thereof - for use in the treatment and/or prevention of cancer.

10. The compound - or a pharmaceutically acceptable salt thereof - for use according to claim 9, wherein said compound or salt is administered in combination with one or more other pharmacologically active substance(s).

11. A pharmaceutical composition comprising a compound according to any one of claim 1 to 8 - or a pharmaceutically acceptable salt thereof - and one or more pharmaceutically acceptable excipient(s).

## Patentansprüche

1. Verbindung der Formel **(Ia)** oder deren Salz 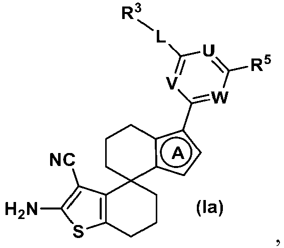 wobei
**L** ausgewählt ist aus -O-, -S- und -N(**R**⁷)-,
**R⁷** Wasserstoff oder C₁₋₆-Alkyl darstellt;
**R³** ausgewählt ist aus der Gruppe, bestehend aus C₁₋₆-Alkyl, C₁₋₆-Alkoxy, 5-10-gliedrigem Heteroaryl und 3-11-gliedrigem Heterocyclyl, wobei das C₁₋₆-Alkyl, 5-10-gliedrige Heteroaryl, C₁₋₆-Alkoxy und 3-11-gliedrige Heterocyclyl alle gegebenenfalls und unabhängig substituiert sind mit ein oder mehreren, gleichen oder verschiedenen Halogenen, C₁₋₆-Alkyl, C₁₋₆-Alkoxy, -OH, -NH₂, -NH(C₁₋₄-Alkyl), -N(C₁₋₄-Alkyl)₂, -C(O)O-C₁₋₆-Alkyl, C₃₋₅-Cycloalkyl oder 3-11-gliedrigem Heterocyclyl, gegebenenfalls substituiert mit -N(C₁₋₄-Alkyl)₂;
**W** ist Stickstoff (-N=);
**V** ist Stickstoff (-N=);
**U** ist -C(**R**¹¹)=;
**R¹¹** ist ausgewählt aus Wasserstoff, Halogen und C₁₋₄-Alkoxy;
Ring **A** ist ein Ring, ausgewählt aus der Gruppe, bestehend aus 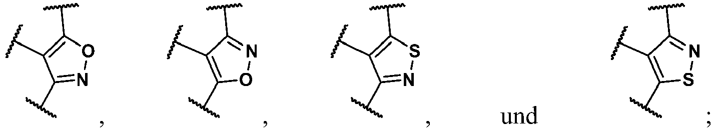
**R⁵** ist ausgewählt aus der Gruppe, bestehend aus

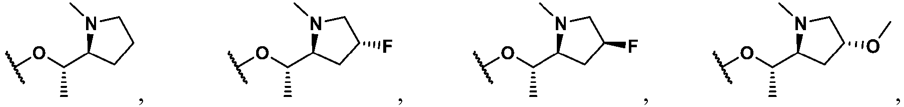

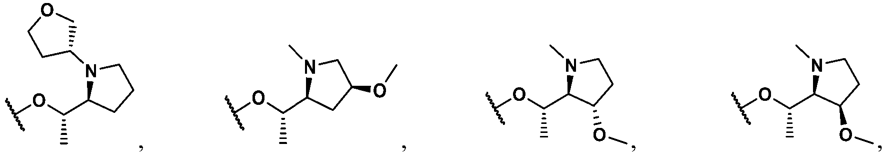

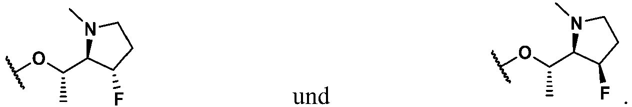

2. Verbindung der Formel **(Ib)** oder deren Salz 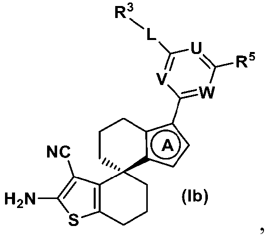 wobei
**A, V, U, W, L, R³** und R⁵ wie in Anspruch 1 definiert sind.

3. Verbindung oder Salz nach irgendeinem der Ansprüche 1 und 2, wobei
**R³** ausgewählt ist aus der Gruppe, bestehend aus C₁₋₆-Alkyl, C₁₋₆-Alkoxy, 5-6-gliedrigem Heteroaryl und 4-5-gliedrigem Heterocyclyl, wobei das C₁₋₆-Alkyl, 5-6-gliedrige Heteroaryl, C₁₋₆-Alkoxy und 4-5-gliedrige Heterocyclyl alle gegebenenfalls und unabhängig substituiert sind mit ein oder mehreren, gleichen oder verschiedenen Halogenen, C₁₋₆-Alkyl, C₁₋₆-Alkoxy, -OH, -NH₂, -NH(C₁₋₄-Alkyl), -N(C₁₋₄-Alkyl)₂, -C(O)O-C₁₋₆-Alkyl, C₃₋₅-Cycloalkyl oder 3-11-gliedrigem Heterocyclyl, gegebenenfalls substituiert mit -N(C₁₋₄-Alkyl)₂.

4. Verbindung oder Salz nach irgendeinem der Ansprüche 1 bis 3, wobei
**R³** ausgewählt ist aus der Gruppe, bestehend aus C₁₋₆-Alkyl, C₁₋₆-Alkoxy, 5-6-gliedrigem Heteroaryl und 4-5-gliedrigem Heterocyclyl, wobei jedes hiervon unabhängig ein oder zwei Stickstoff- oder ein Sauerstoff-Heteroatom(e) enthält, wobei das C₁₋₆-Alkyl, 5-6-gliedrige Heteroaryl, C₁₋₆-Alkoxy und 4-5-gliedrige Heterocyclyl alle gegebenenfalls und unabhängig substituiert sind mit ein oder mehreren, gleichen oder verschiedenen Halogenen, C₁₋₆-Alkyl, C₁₋₆-Alkoxy, -OH, -NH₂, -NH(C₁₋₄-Alkyl), -N(C₁₋₄-Alkyl)₂, -C(O)O-C₁₋₆-Alkyl, C₃₋₅-Cycloalkyl oder 3-11-gliedrigem Heterocyclyl, gegebenenfalls substituiert mit -N(C₁₋₄-Alkyl)₂.

5. Verbindung oder Salz nach irgendeinem der Ansprüche 1 bis 4, wobei
**R³** -C₁₋₄-Alkyl darstellt, substituiert mit einem 4-7-gliedrigen Heterocyclyl oder einem C₃₋₅-Cycloalkyl, wobei das 4-7-gliedrige Heterocyclyl gegebenenfalls weiterhin mit -N(C₁₋₄-Alkyl)₂ substituiert ist.

6. Verbindung oder Salz nach irgendeinem der Ansprüche 1 bis 5, wobei
**R³** ausgewählt ist aus der Gruppe, bestehend aus C₁₋₆-Alkyl, -CH(CH₃)CH₂-O-CH₃, -(CH₂)₂-O-CH₃, -(CH₂)₂-OH und -(CH₂)₂-N-(CH₃)₂,
oder
**R³** ist ein Ring, ausgewählt aus der Gruppe, bestehend aus 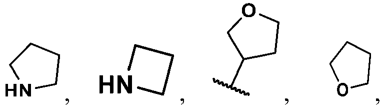 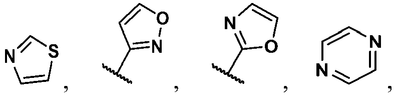 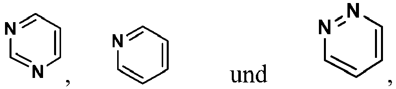 wobei jeder dieser Ringe gegebenenfalls und unabhängig substituiert ist mit ein oder mehreren, gleichen oder verschiedenen Halogenen, C₁₋₆-Alkyl, -OH, -NH₂, -NH(C₁₋₄-Alkyl), -N(C₁₋₄-Alkyl)₂, C₃₋₅-Cycloalkyl oder 3-11-gliedrigem Heterocyclyl.

7. Verbindung oder Salz nach irgendeinem der Ansprüche 1 bis 6, wobei
**R³** ausgewählt ist aus der Gruppe, bestehend aus C₁₋₆-Alkyl, -CH(CH₃)CH₂-O-CH₃, -(CH₂)₂-O-CH₃, -(CH₂)₂-OH, -(CH₂)₂-N-(CH₃)₂,

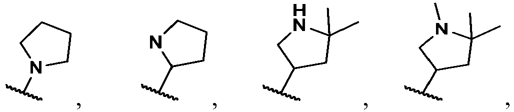

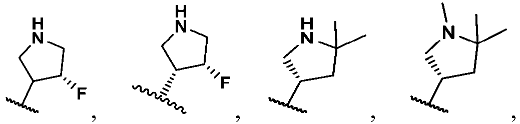

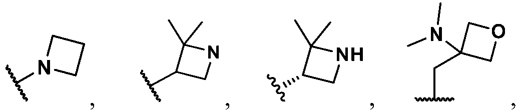

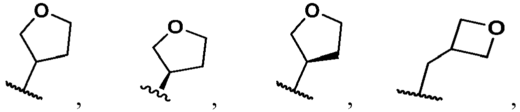

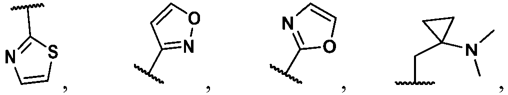

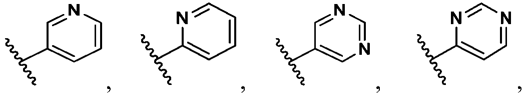

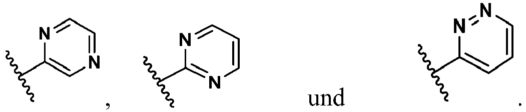

8. Verbindung oder dessen pharmazeutisch akzeptables Salz nach irgendeinem der Ansprüche 1 bis 7, ausgewählt aus der Gruppe, bestehend aus: 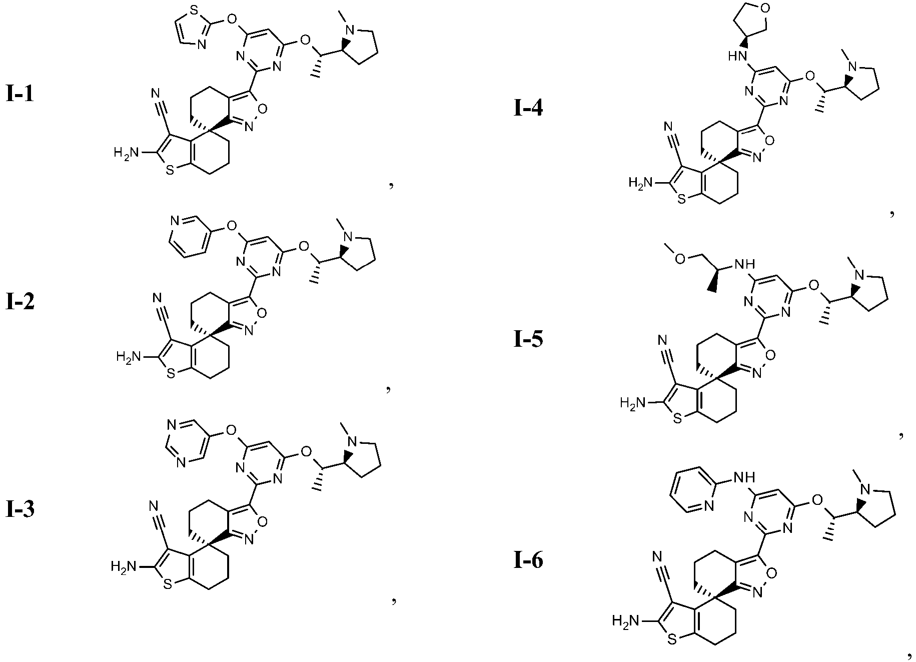 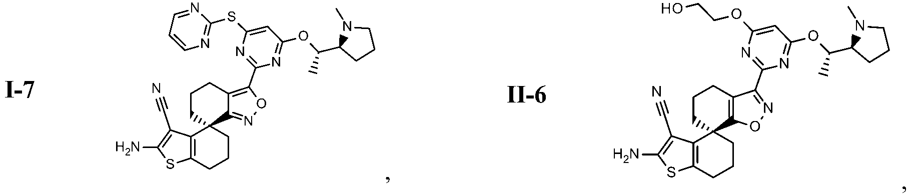 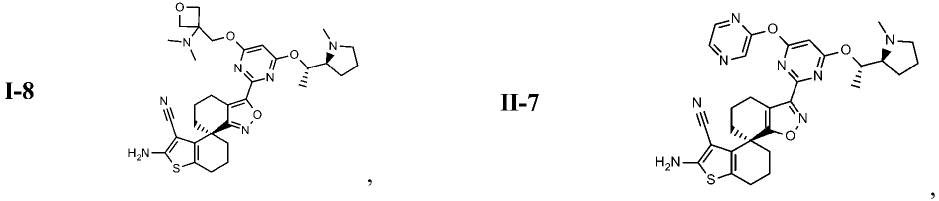 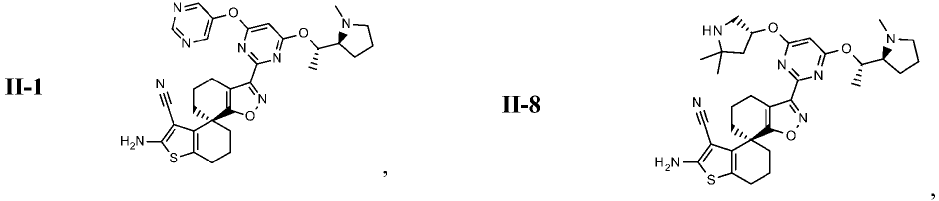 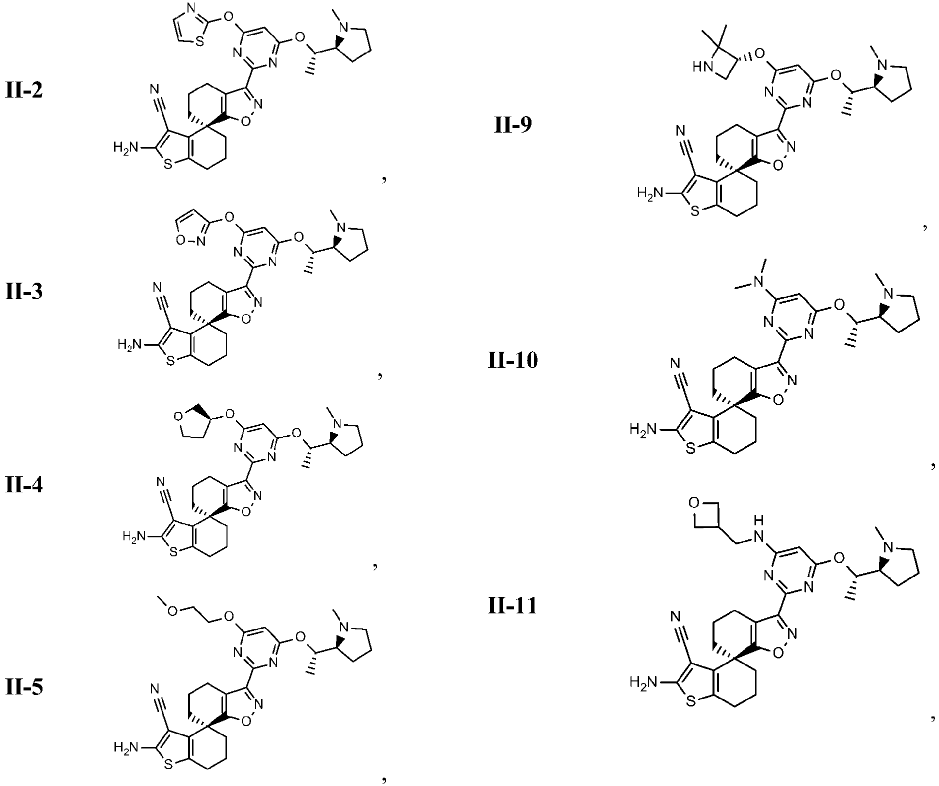 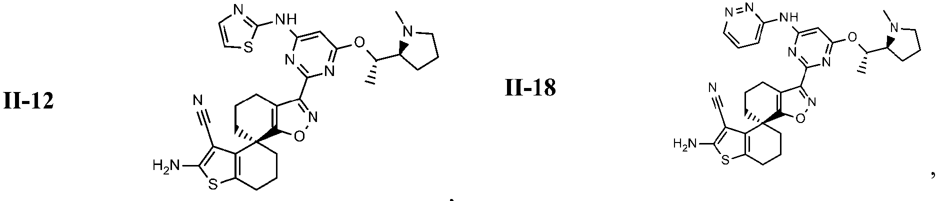 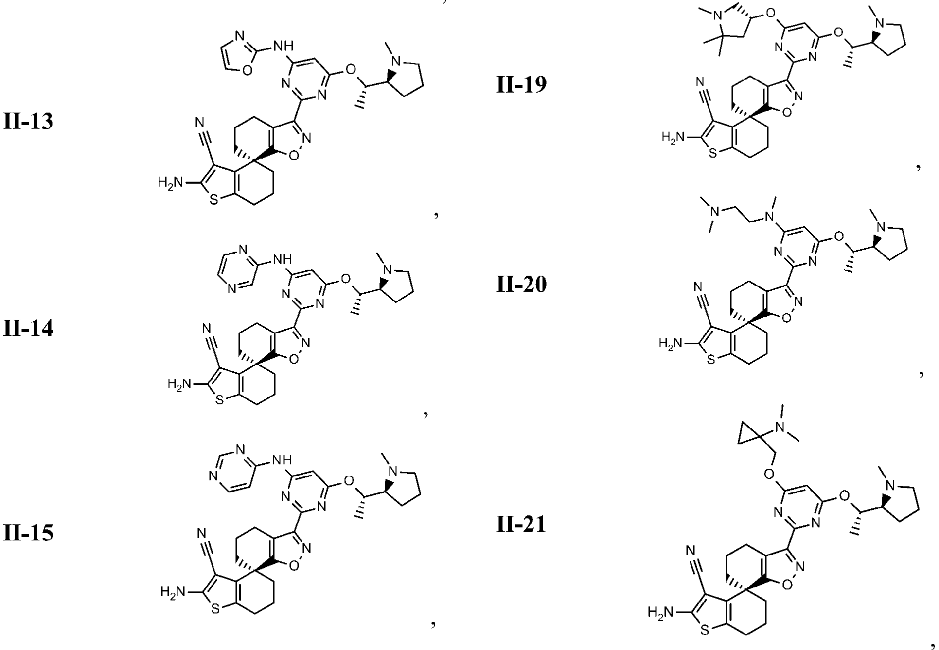 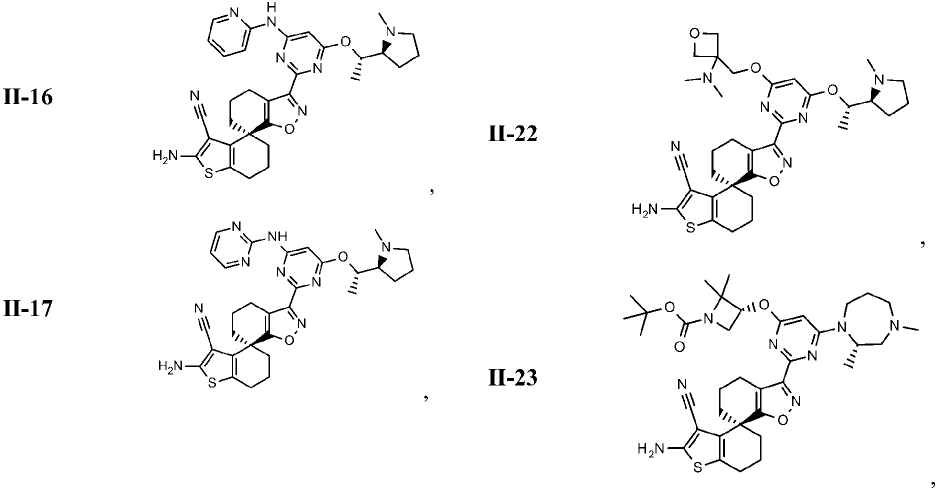 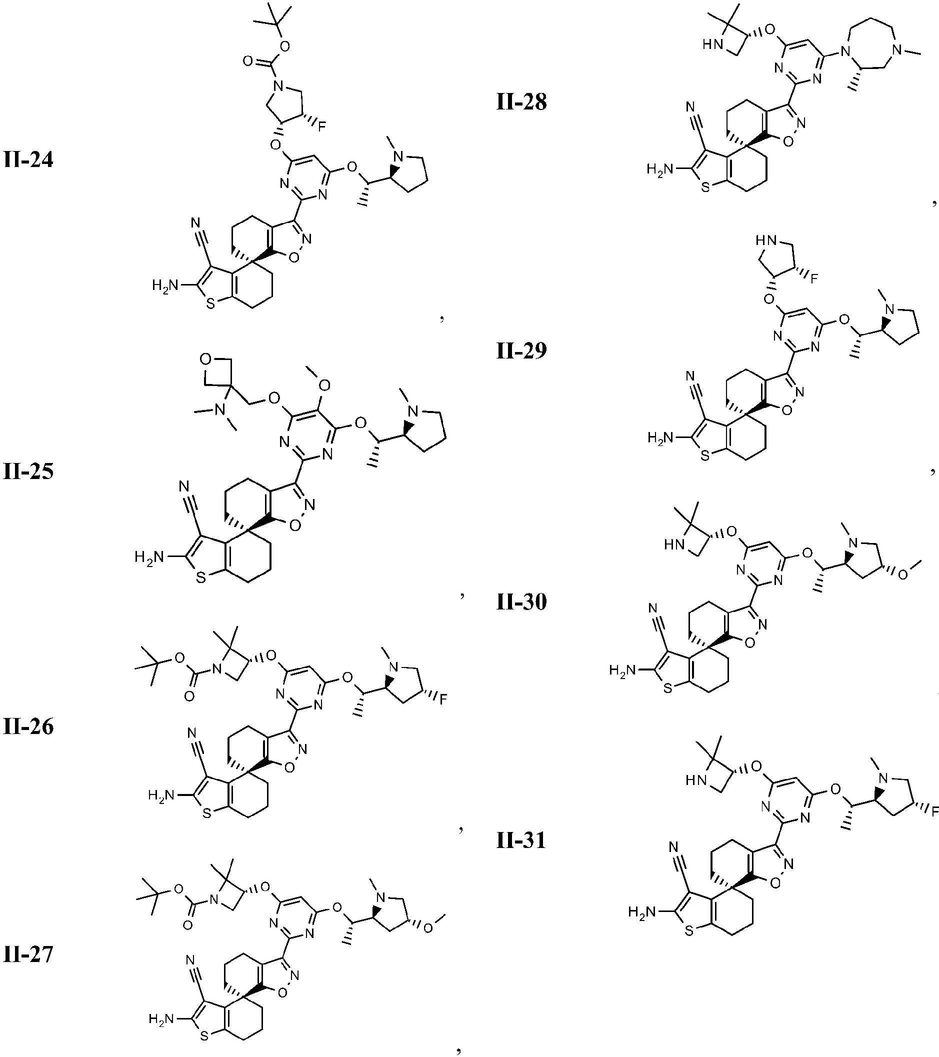 und

9. Verbindung nach irgendeinem der Ansprüche 1 bis 8 - oder ein pharmazeutisch akzeptables Salz hiervon - zur Verwendung in der Behandlung und/oder Vorbeugung von Krebs.

10. Verbindung - oder deren pharmazeutisch akzeptables Salz - zur Verwendung nach Anspruch 9, wobei die Verbindung oder das Salz in Kombination mit einer oder mehreren anderen pharmakologisch aktiven Substanz(en) verabreicht wird.

11. Pharmazeutische Zusammensetzung, umfassend eine Verbindung nach irgendeinem der Ansprüche 1 bis 8 - oder deren pharmazeutisch akzeptables Salz - und einen oder mehrere pharmazeutisch akzeptable Hilfsstoffe.

## Revendications

1. Composé de la formule **(Ia)** ou sel de celui-ci 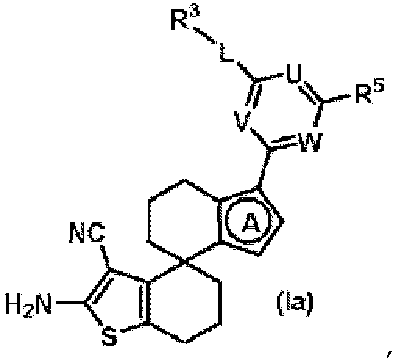 dans lequel
**L** est sélectionné parmi -O-, -S- et -N(**R⁷**)-,
**R⁷** est hydrogène ou C₁₋₆ alkyle ;
**R³** est sélectionné dans le groupe consistant en C_{1- 6} alkyle, C₁₋₆ alcoxy, hétéroaryle à 5-10 chaînons et hétérocyclyle à 3-11 chaînons, dans lequel C₁₋₆ alkyle, hétéroaryle à 5-10 chaînons, C₁₋₆ alcoxy et hétérocyclyle à 3-11 chaînons sont tous éventuellement et indépendamment substitués par un ou plusieurs éléments, identiques ou différents, parmi halogène, C₁₋₆ alkyle, C_{1- 6} alcoxy, -OH, -NH₂, -NH(C₁₋₄ alkyle), -N(C₁₋₄ alkyle)₂, - C(O)O-C₁₋₆ alkyle, C₃₋₅ cycloalkyle ou hétérocyclyle à 3-11 chaînons éventuellement substitué par -N(C₁₋₄ alkyle)₂ ;
**W est** azote (-N=) ;
**V** est azote (-N=) ;
**U** est -C(**R¹¹**)= ;
**R¹¹** est sélectionné parmi hydrogène, halogène et C_{1- 4} alcoxy ;
le cycle **A** est sélectionné dans le groupe consistant en 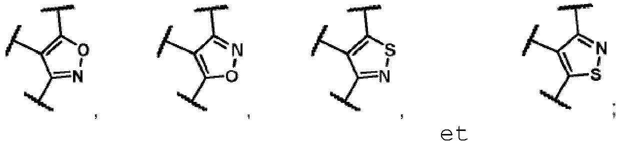
**R⁵** est sélectionné dans le groupe consistant en

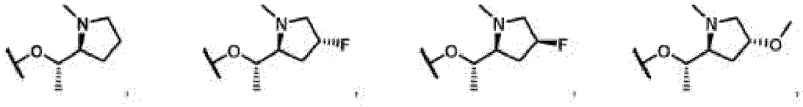

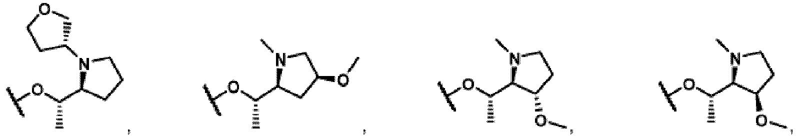

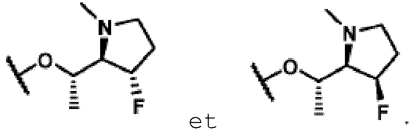

2. Composé de la formule **(Ib)** ou sel de celui-ci 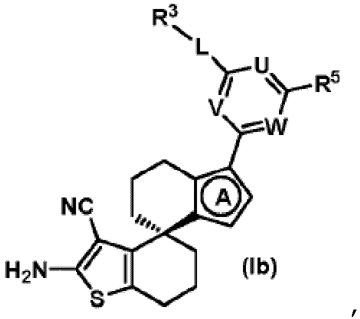 dans lequel
**A, V, U, W, L, R³** et **R⁵** sont tels que définis en revendication 1.

3. Composé ou sel selon l'une quelconque des revendications 1 et 2, dans lequel
**R³** est sélectionné dans le groupe consistant en C₁₋₆ alkyle, C₁₋₆ alcoxy, hétéroaryle à 5-6 chaînons et hétérocyclyle à 4-5 chaînons, dans lequel C₁₋₆ alkyle, hétéroaryle à 5-6 chaînons, C₁₋₆ alcoxy et hétérocyclyle à 4-5 chaînons sont tous éventuellement et indépendamment substitués par un ou plusieurs éléments, identiques ou différents, parmi halogène, C₁₋₆ alkyle, C₁₋₆ alcoxy, -OH, -NH₂, -NH(C₁₋₄ alkyle), -N(C₁₋₄ alkyle)₂, - C(O)O-C₁₋₆ alkyle, C₃₋₅ cycloalkyle ou hétérocyclyle à 3-11 chaînons éventuellement substitué par -N(C₁₋₄ alkyle)₂.

4. Composé ou sel selon l'une quelconque des revendications 1 à 3, dans lequel
**R³** est sélectionné dans le groupe consistant en C₁₋₆ alkyle, C₁₋₆ alcoxy, hétéroaryle à 5-6 chaînons et hétérocyclyle à 4-5 chaînons, chacun contenant indépendamment un ou deux hétéroatomes d'azote ou un hétéroatome d'oxygène, dans lequel C₁₋₆ alkyle, hétéroaryle à 5-6 chaînons, C₁₋₆ alcoxy et hétérocyclyle à 4-5 chaînons sont tous éventuellement et indépendamment substitués par un ou plusieurs éléments, identiques ou différents, parmi halogène, C₁₋₆ alkyle, C₁₋₆ alcoxy, -OH, -NH₂, -NH(C₁₋₄ alkyle), -N(C₁₋₄ alkyle)₂, - C(O)O-C₁₋₆ alkyle, C₃₋₅ cycloalkyle ou hétérocyclyle à 3-11 chaînons éventuellement substitué par - N(C₁₋₄ alkyle)₂.

5. Composé ou sel selon l'une quelconque des revendications 1 à 4, dans lequel
**R³** est -C₁₋₄ alkyle substitué par hétérocyclyle à 4-7 chaînons ou C₃₋₅ cycloalkyle, dans lequel hétérocyclyle à 4-7 chaînons est en outre éventuellement substitué par -N(C1-4 alkyle)₂.

6. Composé ou sel selon l'une quelconque des revendications 1 à 5, dans lequel
**R³** est sélectionné dans le groupe consistant en C₁₋₆ alkyle, -CH(CH₃)CH₂-O-CH₃, -(CH₂)₂-O-CH₃, -(CH₂)₂-OH et - (CH₂)₂-N-(CH₃)₂,
ou
**R³** est un cycle sélectionné dans le groupe consistant en 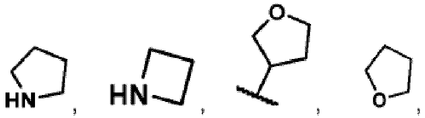 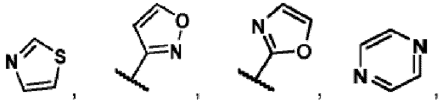 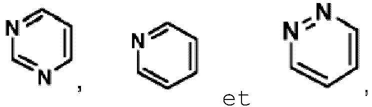
dans lequel chacun de ces cycles est éventuellement et indépendamment substitué par un ou plusieurs éléments, identiques ou différents, parmi halogène, C₁₋₆ alkyle, - OH, -NH₂, -NH(C₁₋₄ alkyle), -N(C₁₋₄ alkyle)₂, C₃₋₅ cycloalkyle ou hétérocyclyle à 3-11 chaînons.

7. Composé ou sel selon l'une quelconque des revendications 1 à 6, dans lequel
**R³** est sélectionné dans le groupe consistant en C₁₋₆ alkyle, -CH(CH₃)CH₂-O-CH₃, -(CH₂)₂-O-CH₃, -(CH₂)₂-OH, - (CH₂)₂-N-(CH₃)₂,

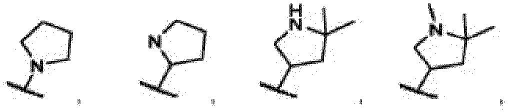

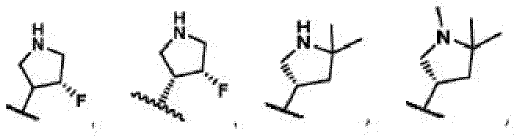

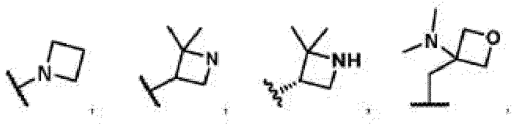

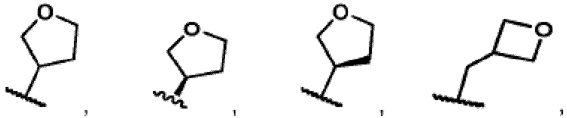

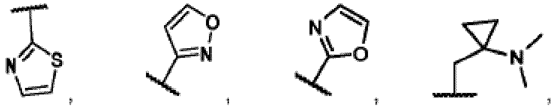

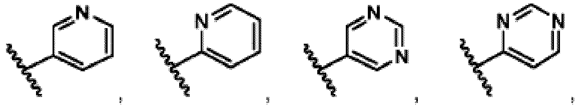

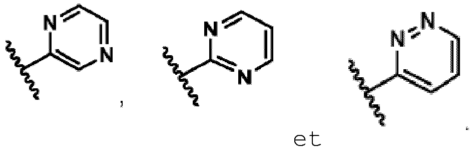

8. Composé ou son sel pharmaceutiquement acceptable selon l'une quelconque des revendications 1 à 7, étant sélectionné dans le groupe consistant en : 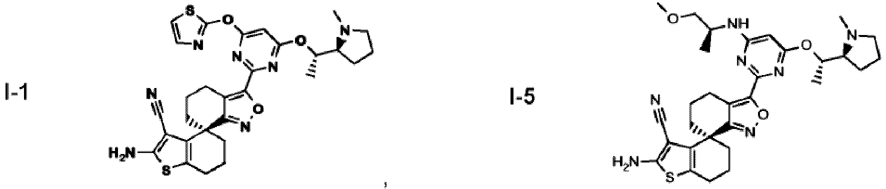 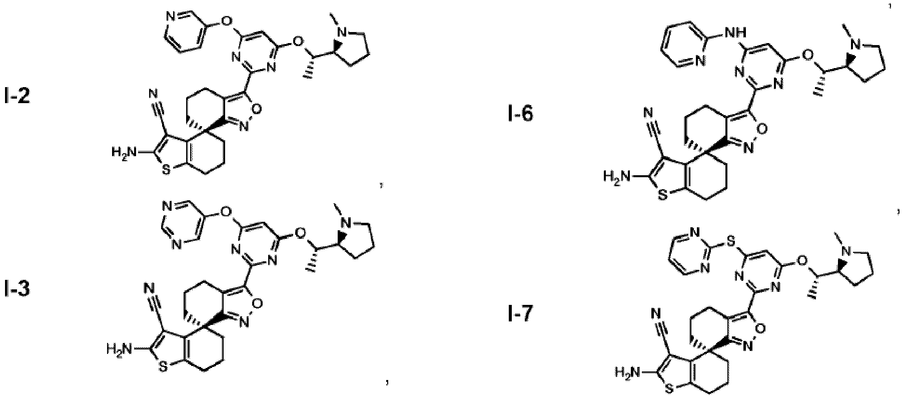 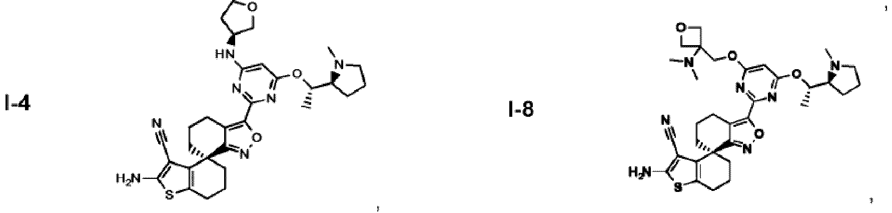 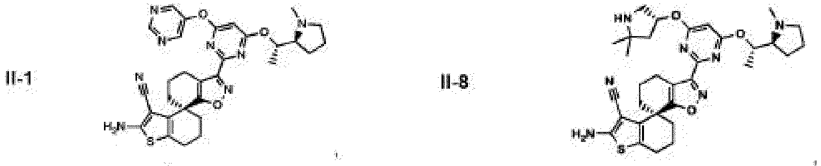 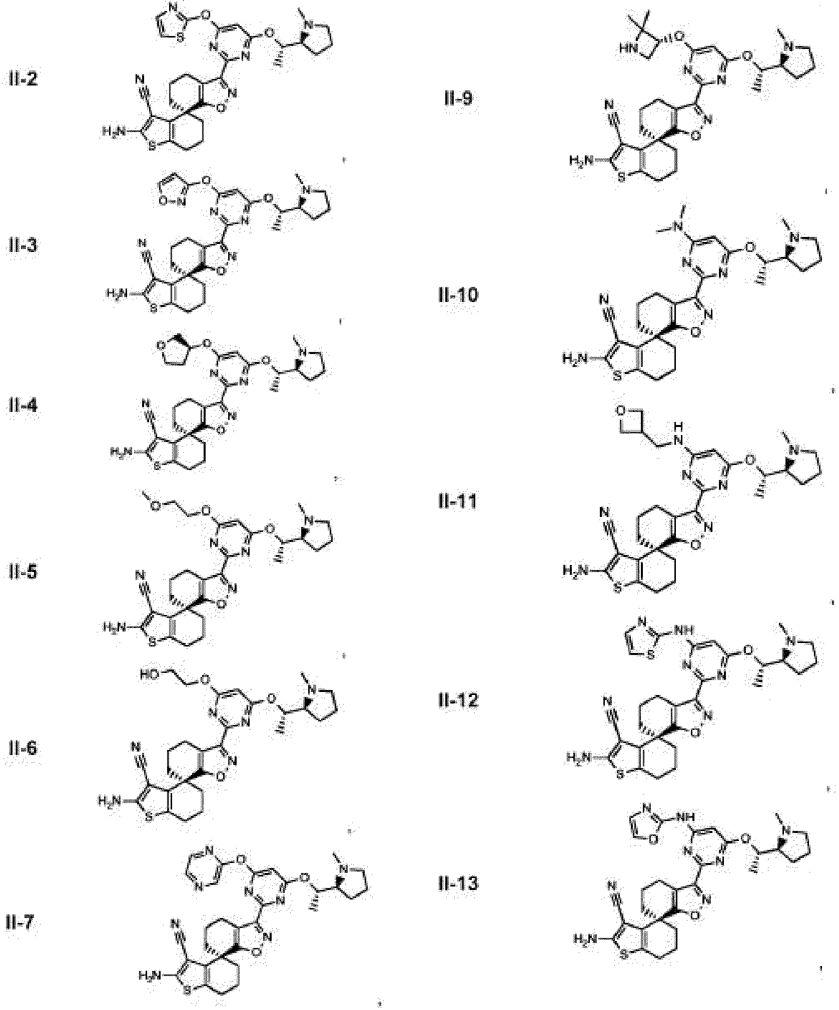 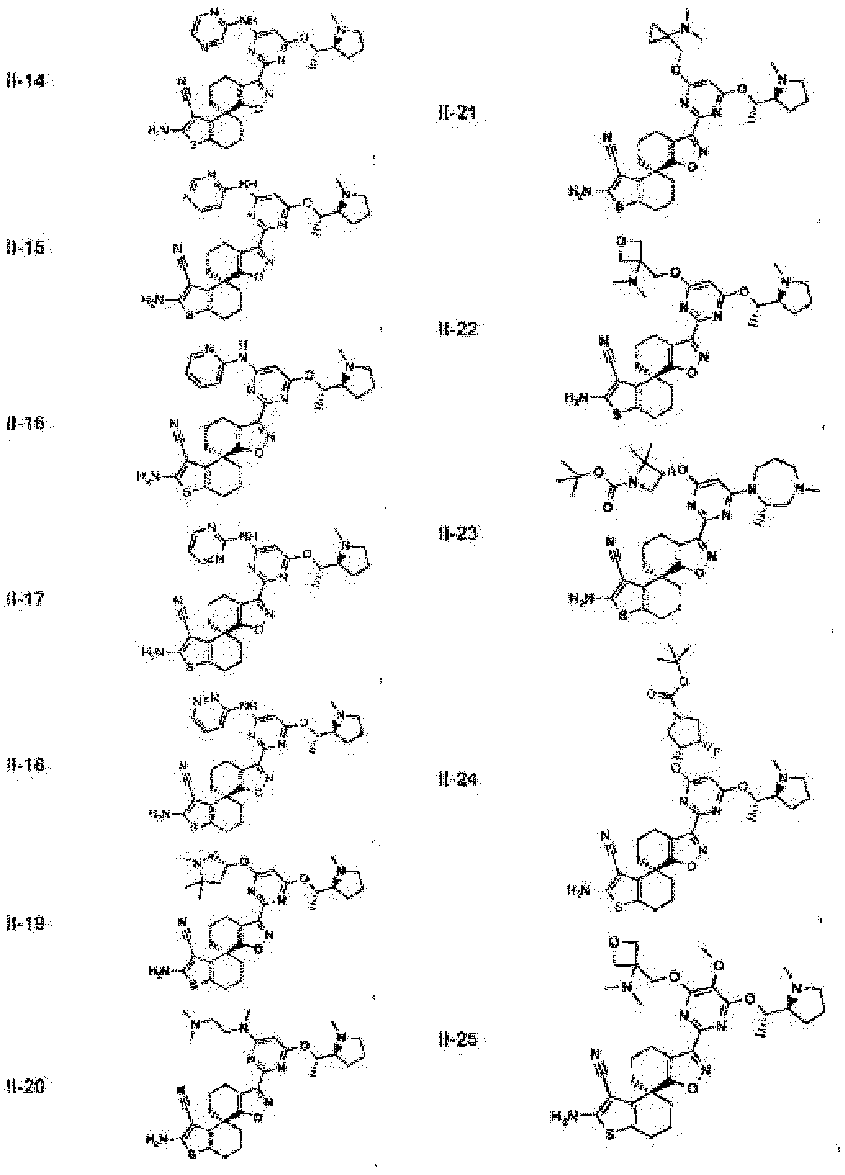 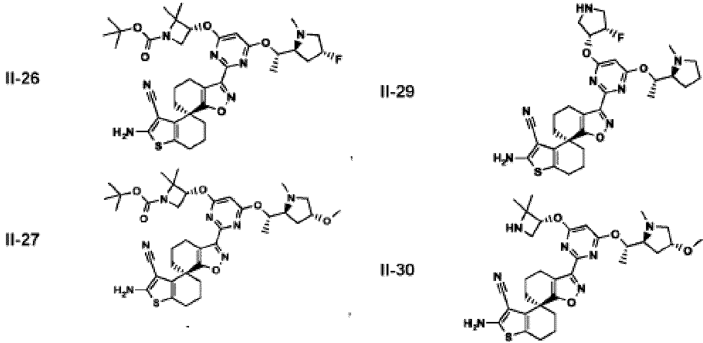 et

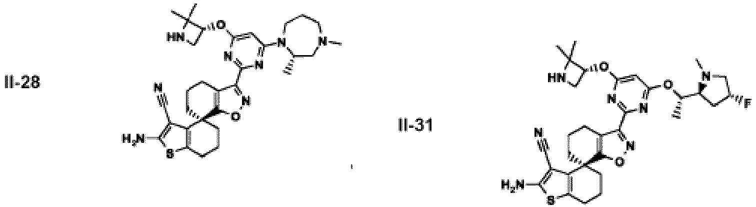

9. Composé selon l'une quelconque des revendications 1 à 8, ou sel pharmaceutiquement acceptable de celui-ci, pour son utilisation dans le traitement et/ou la prévention d'un cancer.

10. Composé, ou sel pharmaceutiquement acceptable de celui-ci, pour son utilisation selon la revendication 9, dans lequel ledit composé ou sel est administré en combinaison avec un ou plusieurs autres principes pharmacologiquement actifs.

11. Composition pharmaceutique comprenant un composé selon l'une quelconque des revendications 1 à 8, ou un sel pharmaceutiquement acceptable de celui-ci, et un ou plusieurs excipients pharmaceutiquement acceptables.
